# EUROPEAN PATENT APPLICATION

(11) **EP 4 797 268 A1**
(43) Date of publication of application: **26.08.2026**
(21) Application number: 26160384.9
(22) Date of filing: 24.02.2026
(51) Int. Cl.: G16B 20/20

(54) **SYSTEMS AND METHODS FOR CHARACTERIZING MUTATIONS**

(30) Priority: 25.02.2025 US 202563763154 P
(71) Applicant: Tempus AI, Inc., Chicago, IL 60654 (US)
(72) Inventor: Parsons, Jerod R., Chicago, IL 60654 (US); Sonnenschein, Anne Franklin, Chicago, IL 60654 (US)
(74) Representative: Grund, Martin

(57) **Abstract**

Systems and methods for characterizing a mutation include obtaining reference sequence reads mapping to a genomic location of the mutation from a first sequencing reaction using a reference sample of a subject, and obtaining tumor sequence reads mapping to the genomic location of the mutation from a second sequencing reaction using a solid tumor sample of the subject. A reference base fraction of the mutation is determined using the reference sequence reads. A germline expectation of the mutation is determined using a tumor purity of the solid tumor sample, the major and the minor copy number at the genomic location of the mutation derived from tumor sequence reads. The reference base fraction and the germline expectation of the mutation are inputted into a model thereby obtaining, as output from the model, a determination of whether the mutation is a clonal hematopoiesis of indeterminate potential mutation or a germline mutation.

## Description

### TECHNICAL FIELD

The present disclosure relates generally to the use of DNA sequencing data to characterize a candidate mutation.

### BACKGROUND

Tumor-normal matched platforms, in which sequences of a solid tumor biopsy are paired with a matched buffy coat specimen, has enabled the accumulation of large amounts of clonal hematopoiesis (CH) data. Although buffy-coat matched sequencing distinguishes tumor from non-tumor variants, accurately identifying CH variants and distinguishing them from germline or artifactual variants presents unique challenges. Typically, the buffy coat is sequenced at lower depth than the tumor, potentially impacting the accuracy of variant calling at low variant allele fractions (VAFs). For CH variants with high VAFs, which are often clinically relevant, distinguishing germline variants is challenging. Due to immune infiltration, CH may be found in both the normal and tumor samples, and copy number variants and loss of heterozygosity in tumor samples can substantially bias VAFs.

Given the above background what is needed in the art are improved systems and methods for identifying CH, from germline, with high accuracy accounting for these challenges.

The information disclosed in this Background section is only for enhancement of understanding of the general background of the invention and should not be taken as an acknowledgement or any form of suggestion that this information forms the prior art already known to a person skilled in the art.

### SUMMARY

Given the above background, there is a need in the art for improved methods and systems for determining whether a candidate mutation is a clonal hematopoiesis of indeterminate potential (CHIP) mutation or a germline mutation.

One aspect of the present disclosure provides a method of characterizing a candidate mutation.

A plurality of reference sequence reads mapping to a genomic location of the candidate mutation is obtained from a first sequencing reaction using a reference sample of a test subject.

In some embodiments, the candidate mutation is a single nucleotide variant (SNV), an indel, a copy number variant or a translocation.

In some embodiments, the reference sample is saliva. In some embodiments, the reference sample is blood, whole blood, peripheral blood, plasma, serum, or lymph. In some embodiments, the reference sample consists of or comprises blood, whole blood, peripheral blood, plasma, serum, lymph, or saliva. In some embodiments, the reference sample consists or comprises buffy coat.

In some embodiments, the first sequencing reaction is a panel-based sequencing reaction of a plurality of loci. In some embodiments, the plurality of loci is sequenced at an average sequence depth of at less than 200x or 250x by the first sequencing reaction.

In some embodiments, the test subject is afflicted with a cancer condition. In some embodiments, the cancer condition is lung cancer, breast cancer, ovarian cancer, cervical cancer, a uveal melanoma, colorectal cancer, chromophobe renal cell carcinoma, liver cancer, an endocrine tumor, oropharyngeal cancer, retinoblastoma, biliary cancer, adrenal cancer, neural cancer, neuroblastoma, basal cell carcinoma, brain cancer, a non-clear cell renal cell carcinoma, a glioblastoma, a glioma, kidney cancer, gastrointestinal stromal tumor, a medulloblastoma, bladder cancer, gastric cancer, bone cancer, thymoma, prostate cancer, a clear cell renal cell carcinoma, skin cancer, thyroid cancer, a sarcoma, testicular cancer, head and neck cancer, a meningioma, peritoneal cancer, endometrial cancer, pancreatic cancer, mesothelioma, esophageal cancer, small cell lung cancer, HER2 negative breast cancer, ovarian serous carcinoma, HR+ breast cancer, uterine serous carcinoma, a uterine corpus endometrial carcinoma, a gastroesophageal junction adenocarcinoma, gallbladder cancer, chordoma, or a papillary renal cell carcinoma.

In some embodiments, the cancer condition is a particular stage of a particular type of cancer.

A plurality of tumor sequence reads mapping to the genomic location of the candidate mutation is obtained from a second sequencing reaction using a solid tumor sample of the test subject. In some embodiments, the second sequencing reaction is a panel-based sequencing reaction of the plurality of loci. In some embodiments, the plurality of loci is sequenced at an average sequence depth of greater than 250x or 400x by the second sequencing reaction. In some embodiments, the plurality of loci comprises at least 5 genes, 25 genes or 50 genes in Table 1. In some embodiments, the plurality of loci comprises at least 5 genes, 25 genes, or 50 genes in Table 2.

In some embodiments, the first sequencing reaction or the second sequencing reaction is a whole genome sequencing.

In some embodiments, a tumor base fraction of the candidate mutation is determined using the plurality of tumor sequence reads.

A reference base fraction of the candidate mutation is determined using the plurality of reference sequence reads.

A germline expectation of the candidate mutation is determined using at least: (i) a tumor purity of the solid tumor sample, (ii) a major copy number at the genomic location of the candidate mutation derived from the plurality of tumor sequence reads, and (iii) a minor copy number at the genomic location of the candidate mutation derived from the plurality of tumor sequence reads.

First information is inputted into a first model comprising a first plurality of parameters thereby obtaining, as output from the first model, through application of the first plurality of parameters to the first information, an indication of whether the candidate mutation is (a) a clonal hematopoiesis of indeterminate potential (CHIP) mutation or (b) a germline mutation. The first information comprises (i) the reference base fraction for the candidate mutation and (ii) the germline expectation of the candidate mutation.

In some embodiments, the first information further comprises an absolute value of a difference between the reference base fraction for the candidate mutation and the germline expectation of the candidate mutation.

In some embodiments, the first information further comprises the tumor base fraction of the candidate mutation.

In some embodiments, the first information further comprises the tumor purity of the solid tumor sample.

In some embodiments, a determination is made as to whether the reference base fraction for the candidate mutation satisfies a first threshold. When the reference base fraction for the candidate mutation satisfies the first threshold, the candidate mutation is deemed to be (a) the CHIP mutation or (b) the germline mutation in accordance with the first model. When the reference base fraction for the candidate mutation fails to satisfy the first threshold, the candidate mutation is deemed to be (a) the CHIP mutation or (b) an artifact in accordance with a second model.

In some embodiments, the reference base fraction for the candidate mutation satisfies the first threshold when the reference base fraction exceeds twenty percent.

In some embodiments, second information is inputted into the second model, where the second model comprises a second plurality of parameters, thereby obtaining as output from the second model, through application of the second plurality of parameters to the second information, an indication of whether the candidate mutation is (a) the CHIP mutation or (b) the artifact. The second information comprises at least two of (i) a total number of sequence reads in the plurality of reference sequence reads, (ii) a sequencing depth of the first sequencing reaction at the genomic location of the candidate mutation, and (iii) an indication of whether the candidate mutation satisfied a minimum variant fraction or a minimum insertion or deletion test.

In some embodiments, the second information further comprises the reference base fraction of the candidate mutation.

In some embodiments, the first or second information further comprises an indication as to whether or not the candidate mutation is in DNMT3A, STAG2, TET2, ASXL1, JAK2, SF3B1, PPM1d, EGFR, or KMT2C.

In some embodiments, the first or second information further comprises a length of the candidate mutation.

In some embodiments, the first or second information further comprises a length of a wildtype allele corresponding to the candidate mutation.

In some embodiments, the first or second information further comprises a binary indication of whether or not the candidate mutation is in a curated list of known CHIP mutations.

In some embodiments, the first or second information further comprises a binary indication as to whether or not the candidate mutation is in a CHIP driver gene.

In some embodiments, the first or second information further comprises a COSMIC solid tumor frequency of the candidate mutation.

In some embodiments, the first or second information further comprises a score for the candidate mutation using any one of single-base substitution signatures SBS1 - SBS60.

In some embodiments, the first or second information further comprises a score for the candidate mutation using any one of single-base substitution signatures in the group consisting of SBS55, SBS2, SBS35, SBS7a, SBS54, SBS8, SBS6, SBS44, SBS10a, SBS59, SBS25, SBS21, SBS19, SBS30, SBS10b, SBS7b, SBS11, SBS32, SBS17b, SBS26, SBS4, and SBS33.

In some embodiments, the first or second information further comprises an ExAC frequency of the candidate mutation.

In some embodiments, the first model or the second model is selected from the group consisting of a regression model, a neural network, a support vector machine, a Naive Bayes model, a nearest neighbor model, a boosted trees model, a random forest model, a decision tree, a gradient boosted tree, an elastic net, a logistic regression model, and a clustering model.

In some embodiments, the first model or the second model is a random forest model.

In some embodiments, the first model or the second model is an XGboost model.

In some embodiments, when the first model indicates the candidate variant is a CHIP variant, a report for the test subject includes the identity of the candidate variant. In some embodiments, the report further comprises a therapeutic recommendation for the test subject based on the identity of the candidate variant. In some embodiments, the report further comprises an identification of the candidate variant as being associated with a secondary hematologic malignancy. In some embodiments, the secondary hematologic malignancy is a therapy-related neoplasm, a secondary acute lymphoblastic leukemia, a secondary myelodysplastic syndrome, a secondary myeloproliferative neoplasm, a secondary non-Hodgkin lymphoma, a secondary plasma cell dyscrasia, or Richter's Transformation.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

Figures 1A, 1B, 1C, and 1D collectively illustrate a block diagram of an example computing device for characterizing a candidate mutation, in accordance with some embodiments of the present disclosure.
Figure 2A illustrates an example workflow for generating a clinical report based on characterizing a candidate mutation, in accordance with some embodiments of the present disclosure.
Figure 2B illustrates an example of a distributed diagnostic environment for collecting and evaluating patient data for the purpose of precision oncology, in accordance with some embodiments of the present disclosure.
Figure 3 provides an example flow chart of processes and features for biopsy sample collection and analysis for use in precision oncology, in accordance with some embodiments of the present disclosure.

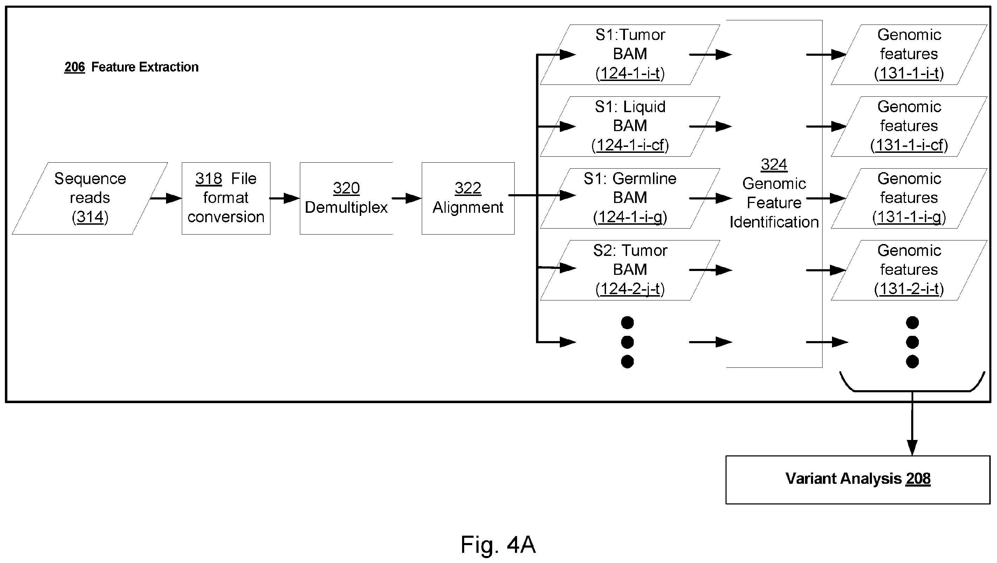

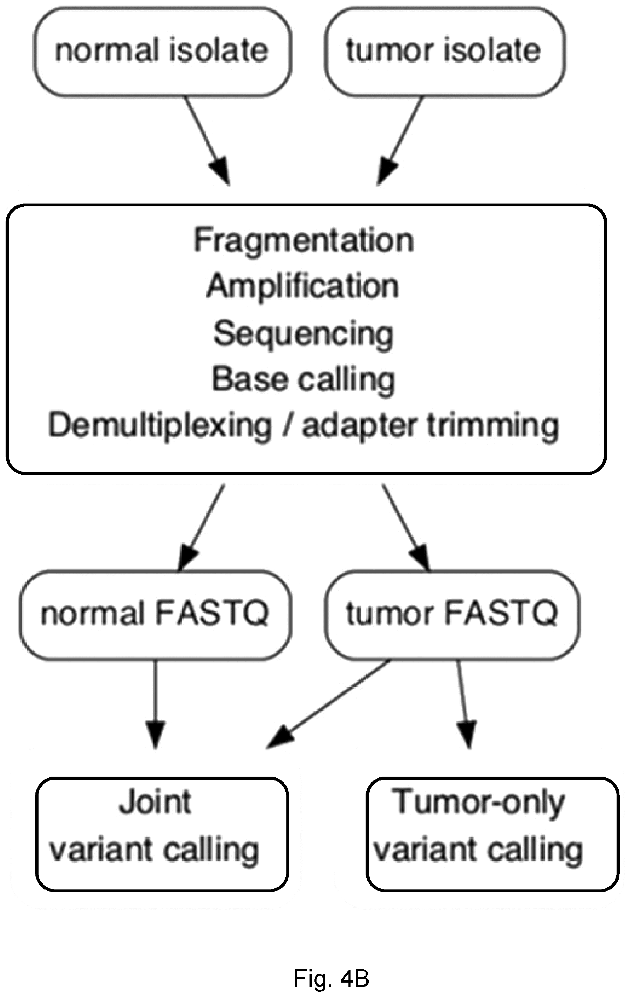

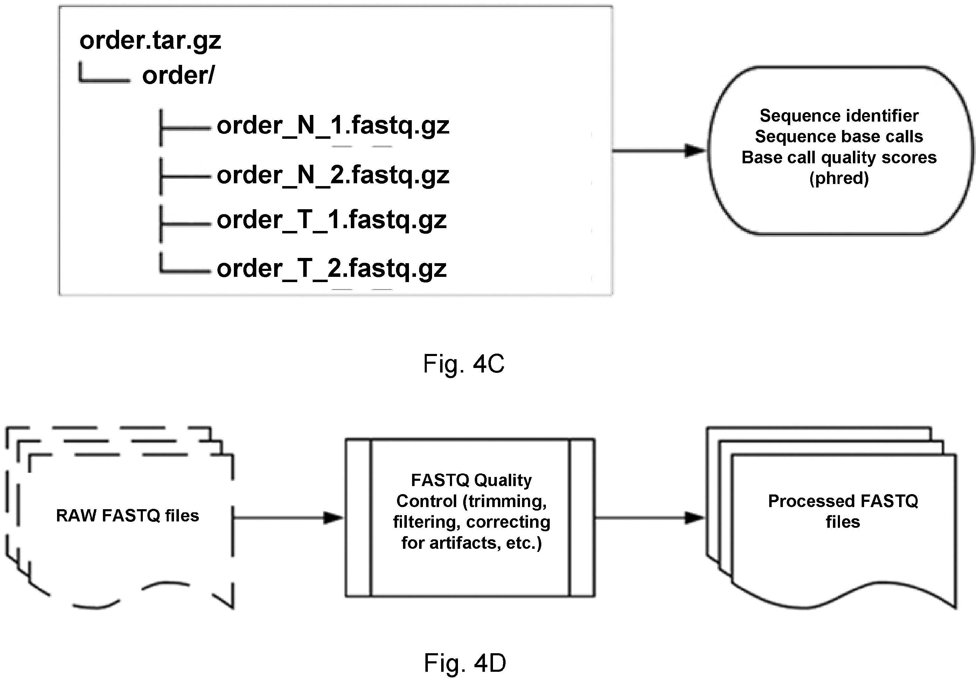

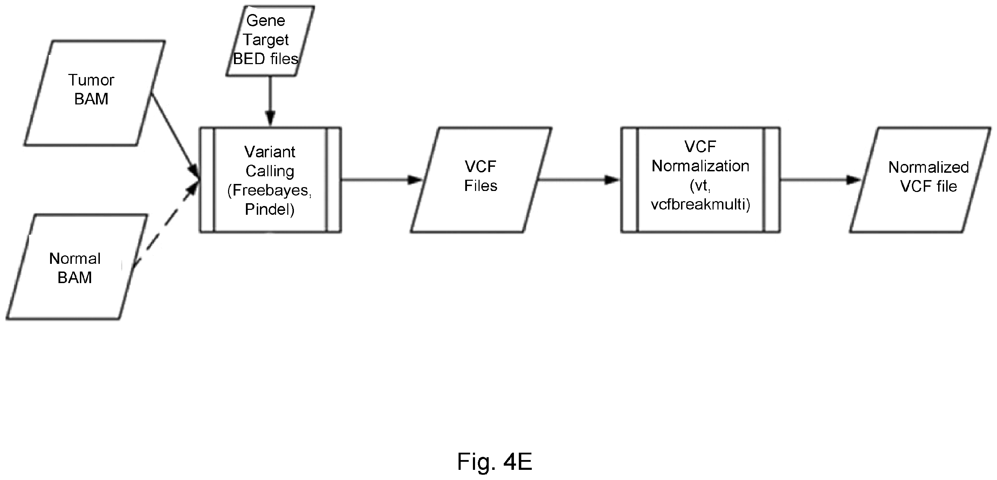

Figures 4A, 4B, 4C, 4D, and 4E collectively illustrate an example bioinformatics pipeline for precision oncology. Figure 4A provides an overview flow chart of processes and features in a bioinformatics pipeline, in accordance with some embodiments of the present disclosure. Figure 4B provides an overview of a bioinformatics pipeline executed with either a liquid biopsy sample alone or a liquid biopsy sample and a matched normal sample. Figure 4C illustrates that paired end reads from tumor and normal isolates are zipped and stored separately under the same order identifier, in accordance with some embodiments of the present disclosure. Figure 4D illustrates quality correction for FASTQ files, in accordance with some embodiments of the present disclosure. Figure 4E illustrates processes for obtaining tumor and normal BAM alignment files, in accordance with some embodiments of the present disclosure.
Figures 5A, 5B, 5C, 5D, 5E, 5F, and 5G collectively provide a flowchart of processes and features for characterizing a candidate mutation from a test subject, in which dashed boxes represent optional portions of the method, in accordance with some embodiments of the present disclosure.
Figure 6 illustrates how comparison of CHIP versus germline mutations in test samples based on VAF in normal (normal base fraction) ~ VAF in tumor (tumor base fraction) does have visual separation, but with significant overlap due to loss of heterozygosity, in accordance with some embodiments of the present disclosure.
Figure 7 illustrates how comparison of CHIP versus germline mutations in test samples based on germline expectation in tumor ~ VAF in tumor (tumor base fraction) has clear separation under all conditions, in accordance with some embodiments of the present disclosure.
Figure 8 illustrates the relative feature importance of each of the features used in a model to discriminate between CHIP and germline mutants, in accordance with some embodiments of the present disclosure.
Figure 9 illustrates the overall confusion matrix for the model of Figure 8 across all folds of training data, in accordance with some embodiments of the present disclosure.
Figure 10 illustrates the Receiver Operating Characteristic - Area Under the Curve (ROC-AUC) for the model of Figure 8 across all folds of training data, in accordance with some embodiments of the present disclosure.
Figure 11 illustrates the relative feature importance of each of the features used in a model to discriminate between CHIP mutants and artifacts, in accordance with some embodiments of the present disclosure.
Figure 12 illustrates the Receiver Operating Characteristic - Area Under the Curve (ROC-AUC) for the model of Figure 11 across all folds of training data, in accordance with some embodiments of the present disclosure.

Like reference numerals refer to corresponding parts throughout the several views of the drawings.

### DETAILED DESCRIPTION

### Introduction.

As described above, conventional liquid biopsy assays do accurately distinguish between somatic and hematopoietic lineages for certain nucleotide variants without data from a second sequencing reaction of DNA isolated from a matched tumor or buffy coat preparation. In certain circumstances, obtaining a matched tumor sample is impracticable, such as for brain cancers. Moreover, performing additional sequencing reactions doubles the preparation time and cost. Advantageously, methods and systems are provided herein for detecting clonal hematopoiesis variants and/or solid tumor variants.

The accurate identification of nucleotide variant lineage is important because precision oncology therapies are tailored for each individual cancer based on, among other factors, the nucleotide variants in the genome of the cancer. Misidentification of nucleotide variants of hematopoietic lineage as somatic variants may result in the selection of an ineffective treatment of the cancer because the cancer being treated does not actually harbor that variant. Where the targeted therapy derives its beneficial effects from the presence of a particular nucleotide variant, treatment of cancers without the particular nucleotide variant may lack effectiveness.

The identification of actionable genomic alterations in a patient's cancer genome is a difficult and computationally demanding problem. For instance, the determination of various prognostic metrics useful for precision oncology, such as variant allelic ratio, copy number variation, tumor mutational burden, microsatellite instability status, etc., requires analysis of hundreds of millions to billions, of sequenced nucleic acid bases. An example of a typical bioinformatics pipeline established for this purpose includes at least five stages of analysis: assessment of the quality of raw next generation sequencing data, generation of collapsed nucleic acid fragment sequences and alignment of such sequences to a reference genome, detection of structural variants in the aligned sequence data, annotation of identified variants, and visualization of the data. See, Wadapurkar and Vyas, Informatics in Medicine Unlocked, 11:75-82 (2018), the content of which is hereby incorporated by reference, in its entirety, for all purposes. Each one of these procedures is computationally taxing in its own right.

For instance, the overall temporal and spatial computation complexity of simple global and local pairwise sequence alignment algorithms are quadratic in nature (e.g., second order problems), that increase rapidly as a function of the size of the nucleic acid sequences (n and m) being compared. Specifically, the temporal and spatial complexities of these sequence alignment algorithms can be estimated as O(mn), where O is the upper bound on the asymptotic growth rate of the algorithm, n is the number of bases in the first nucleic acid sequence, and m is the number of bases in the second nucleic acid sequence. See, Baichoo and Ouzounis, BioSystems, 156-157:72-85 (2017), the content of which is hereby incorporated by reference, in its entirety, for all purposes. Given that the human genome contains more than 3 billion bases, these alignment algorithms are extremely computationally taxing, especially when used to analyze next generation sequencing (NGS) data, which can generate more than 3 billion sequence reads per reaction.

Thus, in addition to the computationally taxing processes required to align sequence reads to a human genome, there is a computation problem of determining whether a particular abnormal signal, e.g., one or more sequence reads corresponding to a genomic alteration, (i) is not an artifact, and (ii) originated from a cancerous source in the subject. This is increasingly difficult during the early stages of cancer-when treatment is presumably most effective.

Advantageously, the present disclosure provides various systems and methods that improve the computational elucidation of actionable genomic alterations from a tumor or cancer cell sample of a cancer patient. Specifically, the present disclosure improves upon the accuracy of distinguishing between (a) a somatic variant derived from cancer cell DNA (b) other than a somatic variant derived from cancer cell DNA. As described above, the disclosed methods and systems are necessarily computer-implemented due to their complexity and heavy computational requirements, and thus solve a problem in the computing arts.

Advantageously, the methods and systems described herein provide an improvement to the abovementioned technical problem (e.g., performing complex computer-implemented methods for distinguishing between (a) a somatic variant derived from cancer cell DNA (b) other than a somatic variant derived from cancer cell. The methods described herein therefore solve a problem in the computing arts by improving upon conventional methods for identifying such variants in a liquid biopsy assay.

The methods and systems described herein also improve precision oncology methods for assigning and/or administering treatment because of the improved accuracy of variant classification provided. Nucleotide variants can be reported as biomarkers and/or used in downstream analysis for identification of therapeutically actionable variants to be included in a clinical report for patient and/or clinician review. Additionally, therapeutically actionable somatic variants identified can be matched with appropriate therapies and/or clinical trials, allowing for more accurate assignment of treatments. The improved accuracy of biomarker detection increases the chance of efficacy and reduces the risk of patients undergoing unnecessary or potentially harmful regimens due to misdiagnoses.

### Definitions.

As used herein, the term "subject" refers to any living or non-living organism including, but not limited to, a human (*e.g.,* a male human, female human, fetus, pregnant female, child, or the like), a non-human mammal, or a non-human animal. Any human or non-human animal can serve as a subject, including but not limited to mammal, reptile, avian, amphibian, fish, ungulate, ruminant, bovine (*e.g.,* cattle), equine (*e.g.,* horse), caprine and ovine (*e.g.,* sheep, goat), swine (*e.g.,* pig), camelid (*e.g.,* camel, llama, alpaca), monkey, ape (*e.g.,* gorilla, chimpanzee), ursid (*e.g.,* bear), poultry, dog, cat, mouse, rat, fish, dolphin, whale and shark. In some embodiments, a subject is a male or female of any age (*e.g.,* a man, a woman, or a child).

As used herein, the terms "control," "control sample," "reference," "reference sample," "normal," and "normal sample" describe a sample from a non-diseased tissue. In some embodiments, such a sample is from a subject that does not have a particular condition (*e.g.,* cancer). In other embodiments, such a sample is an internal control from a subject, *e.g.,* who may or may not have the particular disease (*e.g.,* cancer), but is from a healthy tissue of the subject. For example, where a liquid or solid tumor sample is obtained from a subject with cancer, an internal control sample may be obtained from a healthy tissue of the subject, *e.g.,* a white blood cell sample from a subject without a blood cancer or a solid germline tissue sample from the subject. Accordingly, a reference sample can be obtained from the subject or from a database, *e.g.,* from a second subject who does not have the particular disease (*e.g.,* cancer).

As used herein the term "cancer," "cancerous tissue," or "tumor" refers to an abnormal mass of tissue in which the growth of the mass surpasses, and is not coordinated with, the growth of normal tissue, including both solid masses (*e.g.,* as in a solid tumor) or fluid masses (*e.g.,* as in a hematological cancer). A cancer or tumor can be defined as "benign" or "malignant" depending on the following characteristics: degree of cellular differentiation including morphology and functionality, rate of growth, local invasion and metastasis. A "benign" tumor can be well differentiated, have characteristically slower growth than a malignant tumor and remain localized to the site of origin. In addition, in some cases a benign tumor does not have the capacity to infiltrate, invade or metastasize to distant sites. A "malignant" tumor can be a poorly differentiated (anaplasia), have characteristically rapid growth accompanied by progressive infiltration, invasion, and destruction of the surrounding tissue. Furthermore, a malignant tumor can have the capacity to metastasize to distant sites. Accordingly, a cancer cell is a cell found within the abnormal mass of tissue whose growth is not coordinated with the growth of normal tissue. Accordingly, a "tumor sample" refers to a biological sample obtained or derived from a tumor of a subject, as described herein.

Non-limiting examples of cancer types include ovarian cancer, cervical cancer, uveal melanoma, colorectal cancer, chromophobe renal cell carcinoma, liver cancer, endocrine tumor, oropharyngeal cancer, retinoblastoma, biliary cancer, adrenal cancer, neural cancer, neuroblastoma, basal cell carcinoma, brain cancer, breast cancer, non-clear cell renal cell carcinoma, glioblastoma, glioma, kidney cancer, gastrointestinal stromal tumor, medulloblastoma, bladder cancer, gastric cancer, bone cancer, non-small cell lung cancer, thymoma, prostate cancer, clear cell renal cell carcinoma, skin cancer, thyroid cancer, sarcoma, testicular cancer, head and neck cancer (*e.g.,* head and neck squamous cell carcinoma), meningioma, peritoneal cancer, endometrial cancer, pancreatic cancer, mesothelioma, esophageal cancer, small cell lung cancer, Her2 negative breast cancer, ovarian serous carcinoma, HR+ breast cancer, uterine serous carcinoma, uterine corpus endometrial carcinoma, gastroesophageal junction adenocarcinoma, gallbladder cancer, chordoma, and papillary renal cell carcinoma.

As used herein, the terms "cancer state" or "cancer condition" refer to a characteristic of a cancer patient's condition, *e.g.,* a diagnostic status, a type of cancer, a location of cancer, a primary origin of a cancer, a cancer stage, a cancer prognosis, and/or one or more additional characteristics of a cancer (*e.g.,* tumor characteristics such as morphology, heterogeneity, size, *etc*.). In some embodiments, one or more additional personal characteristics of the subject are used further describe the cancer state or cancer condition of the subject, *e.g.,* age, gender, weight, race, personal habits (*e.g.,* smoking, drinking, diet), other pertinent medical conditions (*e.g.,* high blood pressure, dry skin, other diseases), current medications, allergies, pertinent medical history, current side effects of cancer treatments and other medications, *etc.*

As used herein, the term "liquid biopsy" sample refers to a liquid sample obtained from a subject that includes cell-free DNA. Examples of liquid biopsy samples include, but are not limited to, blood, whole blood, plasma, serum, urine, cerebrospinal fluid, fecal material, saliva, sweat, tears, pleural fluid, pericardial fluid, or peritoneal fluid of the subject. In some embodiments, a liquid biopsy sample is a cell-free sample, *e.g.,* a cell free blood sample. In some embodiments, a liquid biopsy sample is obtained from a subject with cancer. In some embodiments, a liquid biopsy sample is collected from a subject with an unknown cancer status, *e.g.,* for use in determining a cancer status of the subject. Likewise, in some embodiments, a liquid biopsy is collected from a subject with a non-cancerous disorder, *e.g.,* a cardiovascular disease. In some embodiments, a liquid biopsy is collected from a subject with an unknown status for a non-cancerous disorder, *e.g.,* for use in determining a non-cancerous disorder status of the subject.

As used herein, the term "cell-free DNA" and "cfDNA" interchangeably refer to DNA fragments that circulate in a subject's body (*e.g.,* bloodstream) and originate from one or more healthy cells and/or from one or more cancer cells. These DNA molecules are found outside cells, in bodily fluids such as blood, whole blood, plasma, serum, urine, cerebrospinal fluid, fecal material, saliva, sweat, sweat, tears, pleural fluid, pericardial fluid, or peritoneal fluid of a subject, and are believed to be fragments of genomic DNA expelled from healthy and/or cancerous cells, *e.g.,* upon apoptosis and lysis of the cellular envelope.

As used herein, the term "locus" refers to a position (*e.g.,* a site) within a genome, *e.g.,* on a particular chromosome. In some embodiments, a locus refers to a single nucleotide position, on a particular chromosome, within a genome. In some embodiments, a locus refers to a group of nucleotide positions within a genome. In some instances, a locus is defined by a mutation (*e.g.,* substitution, insertion, deletion, inversion, or translocation) of consecutive nucleotide within a cancer genome. In some instances, a locus is defined by a gene, a sub-genic structure (*e.g.,* a regulatory element, exon, intron, or combination thereof), or a predefined span of a chromosome. Because normal mammalian cells have diploid genomes, a normal mammalian genome (*e.g.,* a human genome) will generally have two copies of every locus in the genome, or at least two copies of every locus located on the autosomal chromosomes, *e.g.,* one copy on the maternal autosomal chromosome and one copy on the paternal autosomal chromosome.

As used herein, the term "allele" refers to a particular sequence of one or more nucleotides at a chromosomal locus. In a haploid organism, the subject has one allele at every chromosomal locus. In a diploid organism, the subject has two alleles at every chromosomal locus.

As used herein, the term "base pair" or "bp" refers to a unit consisting of two nucleobases bound to each other by hydrogen bonds. Generally, the size of an organism's genome is measured in base pairs because DNA is typically double stranded. However, some viruses have single-stranded DNA or RNA genomes.

As used herein, the terms "genomic alteration," "mutation," and "variant" refer to a detectable change in the genetic material of one or more cells. A genomic alteration, mutation, or variant can refer to various type of changes in the genetic material of a cell, including changes in the primary genome sequence at single or multiple nucleotide positions, *e.g.,* a single nucleotide variant (SNV), a multi-nucleotide variant (MNV), an indel (*e.g.,* an insertion or deletion of nucleotides), a DNA rearrangement (*e.g.,* an inversion or translocation of a portion of a chromosome or chromosomes), a variation in the copy number of a locus (*e.g.,* an exon, gene, or a large span of a chromosome) (CNV), a partial or complete change in the ploidy of the cell, as well as in changes in the epigenetic information of a genome, such as altered DNA methylation patterns. In some embodiments, a mutation is a change in the genetic information of the cell relative to a particular reference genome, or one or more 'normal' alleles found in the population of the species of the subject. For instance, mutations can be found in both germline cells (*e.g.,* non-cancerous, 'normal' cells) of a subject and in abnormal cells (*e.g.,* pre-cancerous or cancerous cells) of the subject. As such, a mutation in a germline of the subject (*e.g.,* which is found in substantially all 'normal cells' in the subject) is identified relative to a reference genome for the species of the subject. However, many loci of a reference genome of a species are associated with several variant alleles that are significantly represented in the population of the subject and are not associated with a diseased state, *e.g.,* such that they would not be considered 'mutations.' By contrast, in some embodiments, a mutation in a cancerous cell of a subject can be identified relative to either a reference genome of the subject or to the subject's own germline genome. In certain instances, identification of both types of variants can be informative. For instance, in some instances, a mutation that is present in both the cancer genome of the subject and the germline of the subject is informative for precision oncology when the mutation is a so-called 'driver mutation,' which contributes to the initiation and/or development of a cancer. However, in other instances, a mutation that is present in both the cancer genome of the subject and the germline of the subject is not informative for precision oncology, *e.g.,* when the mutation is a so-called 'passenger mutation,' which does not contribute to the initiation and/or development of the cancer. Likewise, in some instances, a mutation that is present in the cancer genome of the subject but not the germline of the subject is informative for precision oncology, *e.g.,* where the mutation is a driver mutation and/or the mutation facilitates a therapeutic approach, *e.g.,* by differentiating cancer cells from normal cells in a therapeutically actionable way. However, in some instances, a mutation that is present in the cancer genome but not the germline of a subject is not informative for precision oncology, *e.g.,* where the mutation is a passenger mutation and/or where the mutation fails to differentiate the cancer cell from a germline cell in a therapeutically actionable way.

As used herein, the term "reference allele" refers to the sequence of one or more nucleotides at a chromosomal locus that is either the predominant allele represented at that chromosomal locus within the population of the species (*e.g.,* the "wild-type" sequence), or an allele that is predefined within a reference genome for the species.

As used herein, the term "variant allele" refers to a sequence of one or more nucleotides at a chromosomal locus that is either not the predominant allele represented at that chromosomal locus within the population of the species (*e.g.,* not the "wild-type" sequence), or not an allele that is predefined within a reference sequence construct (*e.g.,* a reference genome or set of reference genomes) for the species. In some instances, sequence isoforms found within the population of a species that do not affect a change in a protein encoded by the genome, or that result in an amino acid substitution that does not substantially affect the function of an encoded protein, are not variant alleles.

As used herein, the term "variant allele fraction," "VAF," "allelic fraction," or "AF" refers to the number of times a variant or mutant allele was observed (*e.g.,* a number of reads supporting a candidate variant allele) divided by the total number of times the position was sequenced (*e.g.,* a total number of reads covering a candidate locus).

As used herein, the term "germline variants" refers to genetic variants inherited from maternal and paternal DNA. Germline variants may be determined through a matched tumor-normal calling pipeline.

As used herein, the term "somatic variants" refers to variants arising as a result of dysregulated cellular processes associated with neoplastic cells, *e.g.,* a mutation. Somatic variants may be detected via subtraction from a matched normal sample.

As used herein, the term "single nucleotide variant" or "SNV" refers to a substitution of one nucleotide to a different nucleotide at a position (*e.g.,* site) of a nucleotide sequence, *e.g.,* a sequence read from an individual. A substitution from a first nucleobase X to a second nucleobase Y may be denoted as "X>Y." For example, a cytosine to thymine SNV may be denoted as "C>T."

As used herein, the term "insertions and deletions" or "indels" refers to a variant resulting from the gain or loss of DNA base pairs within an analyzed region.

As used herein, the term "copy number variation" or "CNV" refers to the process by which large structural changes in a genome associated with tumor aneuploidy and other dysregulated repair systems are detected. These processes are used to detect large scale insertions or deletions of entire genomic regions. CNV is defined as structural insertions or deletions greater than a certain base pair ("bp") in size, such as 500 bp.

As used herein, the term "gene fusion" refers to the product of large-scale chromosomal aberrations resulting in the creation of a chimeric protein. These expressed products can be non-functional, or they can be highly over or underactive. This can cause deleterious effects in cancer such as hyper-proliferative or anti-apoptotic phenotypes.

As used herein, the term "loss of heterozygosity" refers to the loss of one copy of a segment (*e.g.,* including part or all of one or more genes) of the genome of a diploid subject (*e.g.,* a human) or loss of one copy of a sequence encoding a functional gene product in the genome of the diploid subject, in a tissue, *e.g.,* a cancerous tissue, of the subject. As used herein, when referring to a metric representing loss of heterozygosity across the entire genome of the subject, loss of heterozygosity is caused by the loss of one copy of various segments in the genome of the subject. Loss of heterozygosity across the entire genome may be estimated without sequencing the entire genome of a subject, and such methods for such estimations based on gene panel targeting-based sequencing methodologies are described in the art. Accordingly, in some embodiments, a metric representing loss of heterozygosity across the entire genome of a tissue of a subject is represented as a single value, *e.g.,* a percentage or fraction of the genome. In some cases, a tumor is composed of various sub-clonal populations, each of which may have a different degree of loss of heterozygosity across their respective genomes. Accordingly, in some embodiments, loss of heterozygosity across the entire genome of a cancerous tissue refers to an average loss of heterozygosity across a heterogeneous tumor population. As used herein, when referring to a metric for loss of heterozygosity in a particular gene, *e.g.,* a DNA repair protein such as a protein involved in the homologous DNA recombination pathway (*e.g.,* BRCA1 or BRCA2), loss of heterozygosity refers to complete or partial loss of one copy of the gene encoding the protein in the genome of the tissue and/or a mutation in one copy of the gene that prevents translation of a full-length gene product, *e.g.,* a frameshift or truncating (creating a premature stop codon in the gene) mutation in the gene of interest. In some cases, a tumor is composed of various sub-clonal populations, each of which may have a different mutational status in a gene of interest. Accordingly, in some embodiments, loss of heterozygosity for a particular gene of interest is represented by an average value for loss of heterozygosity for the gene across all sequenced sub-clonal populations of the cancerous tissue. In other embodiments, loss of heterozygosity for a particular gene of interest is represented by a count of the number of unique incidences of loss of heterozygosity in the gene of interest across all sequenced sub-clonal populations of the cancerous tissue (*e.g.,* the number of unique frameshift and/or truncating mutations in the gene identified in the sequencing data).

As used herein, the term "gene product" refers to an RNA (*e.g.,* mRNA or miRNA) or protein molecule transcribed or translated from a particular genomic locus, *e.g.,* a particular gene. The genomic locus can be identified using a gene name, a chromosomal location, or any other genetic mapping metric.

As used herein, the terms "expression level," "abundance level," or simply "abundance" refers to an amount of a gene product, (an RNA species, *e.g.,* mRNA or miRNA, or protein molecule) transcribed or translated by a cell, or an average amount of a gene product transcribed or translated across multiple cells. When referring to mRNA or protein expression, the term generally refers to the amount of any RNA or protein species corresponding to a particular genomic locus, *e.g.,* a particular gene. However, in some embodiments, an expression level can refer to the amount of a particular isoform of an mRNA or protein corresponding to a particular gene that gives rise to multiple mRNA or protein isoforms. The genomic locus can be identified using a gene name, a chromosomal location, or any other genetic mapping metric.

As used herein, the term "ratio" refers to any comparison of a first metric X, or a first mathematical transformation thereof X' (*e.g.,* measurement of a number of units of a genomic sequence in a first one or more biological samples or a first mathematical transformation thereof) to another metric Y or a second mathematical transformation thereof Y' (*e.g.,* the number of units of a respective genomic sequence in a second one or more biological samples or a second mathematical transformation thereof) expressed as X/Y, Y/X, log_{N}(X/Y), log_{N}(Y/X), X'/Y, Y/X', log_{N}(X'/Y), or log_{N}(Y/X'), X/Y', Y'/X, log_{N}(X/Y'), logₙ(Y'/X) , X'/Y', Y'/X', log_{N}(X'/Y'), or log_{N}(Y'/X'), where N is any real number greater than 1 and where example mathematical transformations of X and Y include, but are not limited to. raising X or Y to a power Z, multiplying X or Y by a constant Q, where Z and Q are any real numbers, and/or taking an M based logarithm of X and/or Y, where M is a real number greater than 1. In one non-limiting example, X is transformed to X' prior to ratio calculation by raising X by the power of two (X²) and Y is transformed to Y' prior to ratio calculation by raising Y by the power of 3.2 (Y^{3.2}) and the ratio of X and Y is computed as log₂(X'/Y').

As used herein, the term "relative abundance" refers to a ratio of a first amount of a compound measured in a sample, *e.g.,* a gene product (an RNA species, *e.g.,* mRNA or miRNA, or protein molecule) or nucleic acid fragments having a particular characteristic (*e.g.,* aligning to a particular locus or encompassing a particular allele), to a second amount of a compound measured in a second sample. In some embodiments, relative abundance refers to a ratio of an amount of species of a compound to a total amount of the compound in the same sample. For instance, a ratio of the amount of mRNA transcripts encoding a particular gene in a sample (*e.g.,* aligning to a particular region of the exome) to the total amount of mRNA transcripts in the sample. In other embodiments, relative abundance refers to a ratio of an amount of a compound or species of a compound in a first sample to an amount of the compound of the species of the compound in a second sample. For instance, a ratio of a normalized amount of mRNA transcripts encoding a particular gene in a first sample to a normalized amount of mRNA transcripts encoding the particular gene in a second and/or reference sample.

As used herein, the terms "sequencing," "sequence determination," and the like refer to any biochemical processes that may be used to determine the order of biological macromolecules such as nucleic acids or proteins. For example, sequencing data can include all or a portion of the nucleotide bases in a nucleic acid molecule such as an mRNA transcript or a genomic locus.

As used herein, the term "genetic sequence" refers to a recordation of a series of nucleotides present in a subject's RNA or DNA as determined by sequencing of nucleic acids from the subject.

As used herein, the term "sequence reads" or "reads" refers to nucleotide sequences produced by any nucleic acid sequencing process described herein or known in the art. Reads can be generated from one end of nucleic acid fragments ("single-end reads") or from both ends of nucleic acid fragments (e.g., paired-end reads, double-end reads). The length of the sequence read is often associated with the particular sequencing technology. High-throughput methods, for example, provide sequence reads that can vary in size from tens to hundreds of base pairs (bp). In some embodiments, the sequence reads are of a mean, median or average length of about 15 bp to 900 bp long (*e.g.,* about 20 bp, about 25 bp, about 30 bp, about 35 bp, about 40 bp, about 45 bp, about 50 bp, about 55 bp, about 60 bp, about 65 bp, about 70 bp, about 75 bp, about 80 bp, about 85 bp, about 90 bp, about 95 bp, about 100 bp, about 110 bp, about 120 bp, about 130, about 140 bp, about 150 bp, about 200 bp, about 250 bp, about 300 bp, about 350 bp, about 400 bp, about 450 bp, or about 500 bp. In some embodiments, the sequence reads are of a mean, median or average length of about 1000 bp, 2000 bp, 5000 bp, 10,000 bp, or 50,000 bp or more. Such sequencing, for example, can provide sequence reads that can vary in size from tens to hundreds to thousands of base pairs. In other example, such sequencing can provide sequence reads that do not vary as much, for example, most of the sequence reads can be smaller than 200 bp. A sequence read (or sequencing read) can refer to sequence information corresponding to a nucleic acid molecule (*e.g.,* a string of nucleotides). For example, a sequence read can correspond to a string of nucleotides (*e.g.,* about 20 to about 150) from part of a nucleic acid fragment, can correspond to a string of nucleotides at one or both ends of a nucleic acid fragment, or can correspond to nucleotides of the entire nucleic acid fragment. A sequence read can be obtained in a variety of ways, *e.g.,* using sequencing techniques or using probes, *e.g.,* in hybridization arrays or capture probes, or amplification techniques, such as the polymerase chain reaction (PCR) or linear amplification using a single primer or isothermal amplification.

As used herein, the term "read segment" refers to any form of nucleotide sequence read including the raw sequence reads obtained directly from a nucleic acid sequencing technique or from a sequence derived therefrom, *e.g.,* an aligned sequence read, a collapsed sequence read, or a stitched sequence read.

As used herein, the term "read count" refers to the total number of nucleic acid reads generated, which may or may not be equivalent to the number of nucleic acid molecules generated, during a nucleic acid sequencing reaction.

As used herein, the term "read-depth," "sequencing depth," or "depth" can refer to a total number of unique nucleic acid fragments encompassing a particular locus or region of the genome of a subject that are sequenced in a particular sequencing reaction. Sequencing depth can be expressed as "Yx", *e.g.,* 50x, 100x, *etc.,* where "Y" refers to the number of unique nucleic acid fragments encompassing a particular locus that are sequenced in a sequencing reaction. In such a case, Y is necessarily an integer, because it represents the actual sequencing depth for a particular locus. Alternatively, read-depth, sequencing depth, or depth can refer to a measure of central tendency (*e.g.,* a mean or mode) of the number of unique nucleic acid fragments that encompass one of a plurality of loci or regions of the genome of a subject that are sequenced in a particular sequencing reaction. For example, in some embodiments, sequencing depth refers to the average depth of every locus across an arm of a chromosome, a targeted sequencing panel, an exome, or an entire genome. In such case, Y may be expressed as a fraction or a decimal, because it refers to an average coverage across a plurality of loci. When a mean depth is recited, the actual depth for any particular locus may be different than the overall recited depth. Metrics can be determined that provide a range of sequencing depths in which a defined percentage of the total number of loci fall. For instance, a range of sequencing depths within which 90% or 95%, or 99% of the loci fall. As understood by the skilled artisan, different sequencing technologies provide different sequencing depths. For instance, low-pass whole genome sequencing can refer to technologies that provide a sequencing depth of less than 5x, less than 4x, less than 3x, or less than 2x, *e.g.,* from about 0.5x to about 3x.

As used herein, the term "sequencing breadth" refers to what fraction of a particular reference exome (*e.g.,* human reference exome), a particular reference genome (*e.g.,* human reference genome), or part of the exome or genome has been analyzed. Sequencing breadth can be expressed as a fraction, a decimal, or a percentage, and is generally calculated as (the number of loci analyzed / the total number of loci in a reference exome or reference genome). The denominator of the fraction can be a repeat-masked genome, and thus 100% can correspond to all of the reference genome minus the masked parts. A repeat-masked exome or genome can refer to an exome or genome in which sequence repeats are masked (*e.g.,* sequence reads align to unmasked portions of the exome or genome). In some embodiments, any part of an exome or genome can be masked and, thus, sequencing breadth can be evaluated for any desired portion of a reference exome or genome. In some embodiments, "broad sequencing" refers to sequencing/analysis of at least 0.1% of an exome or genome.

As used herein, the term "sequencing probe" refers to a molecule that binds to a nucleic acid with affinity that is based on the expected nucleotide sequence of the RNA or DNA present at that locus.

As used herein, the term "targeted panel" or "targeted gene panel" refers to a combination of probes for sequencing (*e.g.,* by next-generation sequencing) nucleic acids present in a biological sample from a subject (*e.g.,* a tumor sample, liquid biopsy sample, germline tissue sample, white blood cell sample, or tumor or tissue organoid sample), selected to map to one or more loci of interest on one or more chromosomes. An example set of loci/genes useful for precision oncology, *e.g.,* via solid or liquid biopsy assay, that can be analyzed using a targeted panel is described in Table 1. Another example set of loci/genes useful for precision oncology, *e.g.,* via solid or liquid biopsy assay, that can be analyzed using a targeted panel is described in Table 2. In some embodiments, in addition to loci that are informative for precision oncology, a targeted panel includes one or more probes for sequencing one or more of a loci associated with a different medical condition, a loci used for internal control purposes, or a loci from a pathogenic organism (*e.g.,* an oncogenic pathogen).

As used herein, the term, "reference exome" refers to any sequenced or otherwise characterized exome, whether partial or complete, of any tissue from any organism or pathogen that may be used to reference identified sequences from a subject. Typically, a reference exome will be derived from a subject of the same species as the subject whose sequences are being evaluated. Example reference exomes used for human subjects as well as many other organisms are provided in the on-line genome browser hosted by the National Center for Biotechnology Information ("NCBI"). An "exome" refers to the complete transcriptional profile of an organism or pathogen, expressed in nucleic acid sequences. As used herein, a reference sequence or reference exome often is an assembled or partially assembled exomic sequence from an individual or multiple individuals. In some embodiments, a reference exome is an assembled or partially assembled exomic sequence from one or more human individuals. The reference exome can be viewed as a representative example of a species' set of expressed genes. In some embodiments, a reference exome comprises sequences assigned to chromosomes.

As used herein, the term "reference genome" refers to any sequenced or otherwise characterized genome, whether partial or complete, of any organism or pathogen that may be used to reference identified sequences from a subject. Typically, a reference genome will be derived from a subject of the same species as the subject whose sequences are being evaluated. Exemplary reference genomes used for human subjects as well as many other organisms are provided in the on-line genome browser hosted by the National Center for Biotechnology Information ("NCBI") or the University of California, Santa Cruz (UCSC). A "genome" refers to the complete genetic information of an organism or pathogen, expressed in nucleic acid sequences. As used herein, a reference sequence or reference genome often is an assembled or partially assembled genomic sequence from an individual or multiple individuals. In some embodiments, a reference genome is an assembled or partially assembled genomic sequence from one or more human individuals. The reference genome can be viewed as a representative example of a species' set of genes. In some embodiments, a reference genome comprises sequences assigned to chromosomes. Exemplary human reference genomes include but are not limited to NCBI build 34 (UCSC equivalent: hg16), NCBI build 35 (UCSC equivalent: hg17), NCBI build 36.1 (UCSC equivalent: hg18), GRCh37 (UCSC equivalent: hg19), and GRCh38 (UCSC equivalent: hg38). For a haploid genome, there can be only one nucleotide at each locus. For a diploid genome, heterozygous loci can be identified; each heterozygous locus can have two alleles, where either allele can allow a match for alignment to the locus.

As used herein, the term "bioinformatics pipeline" refers to a series of processing stages used to determine characteristics of a subject's genome or exome based on sequencing data of the subject's genome or exome. A bioinformatics pipeline may be used to determine characteristics of a germline genome or exome of a subject and/or a cancer genome or exome of a subject. In some embodiments, the pipeline extracts information related to genomic alterations in the cancer genome of a subject, which is useful for guiding clinical decisions for precision oncology, from sequencing results of a biological sample, *e.g.,* a tumor sample, liquid biopsy sample, reference normal sample, *etc.,* from the subject. Certain processing stages in a bioinformatics may be 'connected,' meaning that the results of a first respective processing stage are informative and/or essential for execution of a second, downstream processing stage. For instance, in some embodiments, a bioinformatics pipeline includes a first respective processing stage for identifying genomic alterations that are unique to the cancer genome of a subject and a second respective processing stage that uses the quantity and/or identity of the identified genomic alterations to determine a metric that is informative for precision oncology, *e.g.,* a tumor mutational burden. In some embodiments, the bioinformatics pipeline includes a reporting stage that generates a report of relevant and/or actionable information identified by upstream stages of the pipeline, which may or may not further include recommendations for aiding clinical therapy decisions.

As used herein, the term "limit of detection" or "LOD" refers to the minimal quantity of a feature that can be identified with a particular level of confidence. Accordingly, level of detection can be used to describe an amount of a substance that must be present in order for a particular assay to reliably detect the substance. A level of detection can also be used to describe a level of support needed for an algorithm to reliably identify a genomic alteration based on sequencing data. For example, a minimal number of unique sequence reads to support identification of a sequence variant such as a SNV.

As used herein, the term "BAM File" or "Binary file containing Alignment Maps" refers to a file storing sequencing data aligned to a reference sequence (*e.g.,* a reference genome or exome). In some embodiments, a BAM file is a compressed binary version of a SAM (Sequence Alignment Map) file that includes, for each of a plurality of unique sequence reads, an identifier for the sequence read, information about the nucleotide sequence, information about the alignment of the sequence to a reference sequence, and optionally metrics relating to the quality of the sequence read and/or the quality of the sequence alignment. While BAM files generally relate to files having a particular format, for simplicity they are used herein to simply refer to a file, of any format, containing information about a sequence alignment, unless specifically stated otherwise.

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used herein, the term "Positive Predictive Value" or "PPV" means the likelihood that a variant is properly called given that a variant has been called by an assay. PPV can be expressed as (number of true positives)/ (number of false positives + number of true positives).

As used herein, the term "assay" refers to a technique for determining a property of a substance, *e.g.,* a nucleic acid, a protein, a cell, a tissue, or an organ. An assay (*e.g.,* a first assay or a second assay) can comprise a technique for determining the copy number variation of nucleic acids in a sample, the methylation status of nucleic acids in a sample, the fragment size distribution of nucleic acids in a sample, the mutational status of nucleic acids in a sample, or the fragmentation pattern of nucleic acids in a sample. Any assay known to a person having ordinary skill in the art can be used to detect any of the properties of nucleic acids mentioned herein. Properties of a nucleic acids can include a sequence, genomic identity, copy number, methylation state at one or more nucleotide positions, size of the nucleic acid, presence or absence of a mutation in the nucleic acid at one or more nucleotide positions, and pattern of fragmentation of a nucleic acid (*e.g.,* the nucleotide position(s) at which a nucleic acid fragments). An assay or method can have a particular sensitivity and/or specificity, and their relative usefulness as a diagnostic tool can be measured using ROC-AUC statistics.

As used herein, the term "classification" can refer to any number(s) or other characters(s) that are associated with a particular property of a sample. For example, in some embodiments, the term "classification" can refer to a type of cancer in a subject, a stage of cancer in a subject, a prognosis for a cancer in a subject, a tumor load, a presence of tumor metastasis in a subject, and the like. The classification can be binary (*e.g.,* positive or negative) or have more levels of classification (*e.g.,* a scale from 1 to 10 or 0 to 1). The terms "cutoff" and "threshold" can refer to predetermined numbers used in an operation. For example, a cutoff size can refer to a size above which fragments are excluded. A threshold value can be a value above or below which a particular classification applies. Either of these terms can be used in either of these contexts.

As used herein, the term "sensitivity" or "true positive rate" (TPR) refers to the number of true positives divided by the sum of the number of true positives and false negatives. Sensitivity can characterize the ability of an assay or method to correctly identify a proportion of the population that truly has a condition. For example, sensitivity can characterize the ability of a method to correctly identify the number of subjects within a population having cancer. In another example, sensitivity can characterize the ability of a method to correctly identify the one or more markers indicative of cancer.

As used herein, the term "specificity" or "true negative rate" (TNR) refers to the number of true negatives divided by the sum of the number of true negatives and false positives. Specificity can characterize the ability of an assay or method to correctly identify a proportion of the population that truly does not have a condition. For example, specificity can characterize the ability of a method to correctly identify the number of subjects within a population not having cancer. In another example, specificity characterizes the ability of a method to correctly identify one or more markers indicative of cancer.

As used herein, an "actionable genomic alteration" or "actionable variant" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to be associated with a therapeutic course of action that is more likely to produce a positive effect in a cancer patient that has the actionable variant than in a similarly situated cancer patient that does not have the actionable variant. For instance, administration of EGFR inhibitors (*e.g.,* afatinib, erlotinib, gefitinib) is more effective for treating non-small cell lung cancer in patients with an EGFR mutation in exons 19/21 than for treating non-small cell lung cancer in patients that do not have an EGFR mutations in exons 19/21. Accordingly, an EGFR mutation in exon 19/21 is an actionable variant. In some instances, an actionable variant is only associated with an improved treatment outcome in one or a group of specific cancer types. In other instances, an actionable variant is associated with an improved treatment outcome in substantially all cancer types.

As used herein, a "variant of uncertain significance" or "VUS" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), whose impact on disease development/progression is unknown.

As used herein, a "benign variant" or "likely benign variant" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to not contribute to disease development/progression.

As used herein, a "pathogenic variant" or "likely pathogenic variant" refers to a genomic alteration (*e.g.,* a SNV, MNV, indel, rearrangement, copy number variation, or ploidy variation), or value of another cancer metric derived from nucleic acid sequencing data (*e.g.,* a tumor mutational burden, MSI status, or tumor fraction), that is known or believed to contribute to disease development/progression.

As used herein, an "effective amount" or "therapeutically effective amount" is an amount sufficient to affect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject, the condition being treated, the severity of the condition and the form and effective concentration of the therapeutic agent being administered.

The terminology used in the present disclosure is for the purpose of describing particular embodiments only and is not intended to be limiting of the invention. As used in the description of the invention and the appended items, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "including," "includes," "having," "has," "with," or variants thereof are used in either the detailed description and/or the disclosed items, such terms are intended to be inclusive in a manner similar to the term "comprising."

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

It will also be understood that, although the terms first, second, *etc.* may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject," "user," and "patient" are used interchangeably herein.

Reference will now be made in detail to embodiments, examples of which are illustrated in the accompanying drawings. In the following detailed description, numerous specific details are set forth in order to provide a thorough understanding of the present disclosure, including example systems, methods, techniques, instruction sequences, and computing machine program products that embody illustrative implementations. However, the illustrative discussions below are not intended to be exhaustive or to limit the implementations to the precise forms disclosed. Many modifications and variations are possible in view of the above teachings. The features described herein are not limited by the illustrated ordering of acts or events, as some acts can occur in different orders and/or concurrently with other acts or events.

The implementations provided herein are chosen and described in order to best explain the principles and their practical applications, to thereby enable others skilled in the art to best utilize the various embodiments with various modifications as are suited to the particular use contemplated. In some instances, well-known methods, procedures, components, circuits, and networks have not been described in detail so as not to unnecessarily obscure aspects of the embodiments. In other instances, it will be apparent to one of ordinary skill in the art that the present disclosure may be practiced without one or more of the specific details.

It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that though such a design effort might be complex and time-consuming, it will nevertheless be a routine undertaking of engineering for those of ordering skill in the art having the benefit of the present disclosure.

### Example System Embodiments

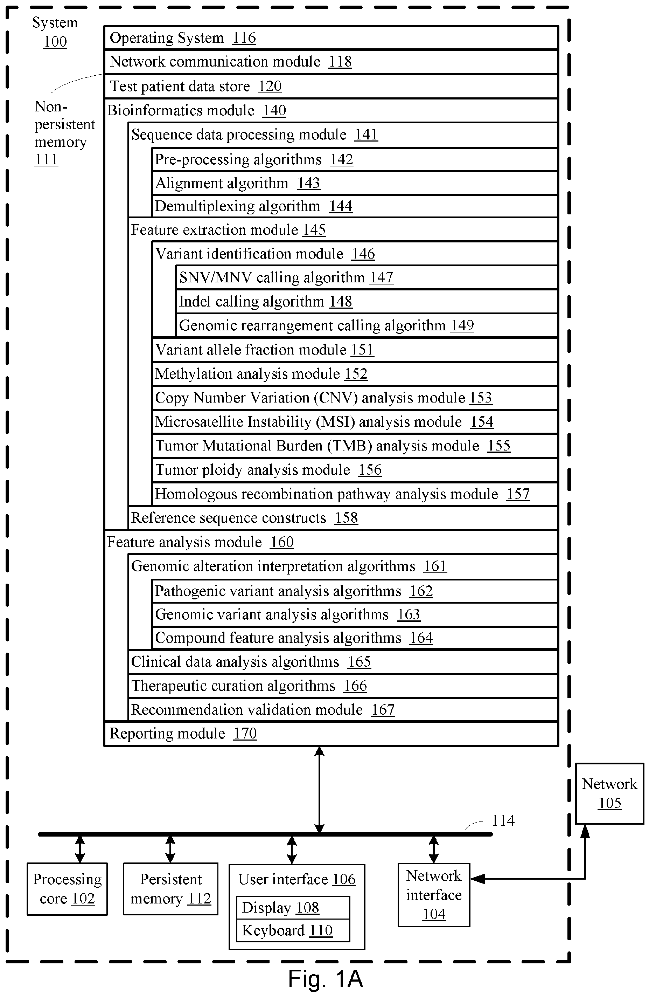

Now that an overview of some aspects of the present disclosure and some definitions used in the present disclosure have been provided, details of an exemplary system for detecting whether a variant in a biopsy from a subject is (a) a somatic variant derived from cell free DNA or (b) other than a somatic variant derived from cell free DNA are now described in conjunction with Figures 1A-1D. Figures 1A-1D collectively illustrate the topology of an example system for detecting whether a variant in a biopsy from a subject is (a) a somatic variant derived from cell free DNA or (b) other than a somatic variant derived from cell free DNA, in accordance with some embodiments of the present disclosure. Advantageously, the example system illustrated in Figures 1A-1D improves upon conventional methods for providing clinical support for personalized cancer therapy by detecting whether a variant in a biopsy from a subject is (a) a somatic variant derived from cell free DNA or (b) other than a somatic variant derived from cell free DNA.

Figure 1A is a block diagram illustrating a system in accordance with some implementations. The device 100 in some implementations includes one or more processing units CPU(s) 102 (also referred to as processors), one or more network interfaces 104, a user interface 106, *e.g.,* including a display 108 and/or an input 110 (*e.g.,* a mouse, touchpad, keyboard, *etc.),* a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112:
- an operating system 116, which includes procedures for handling various basic system services and for performing hardware dependent tasks;
- a network communication module (or instructions) 118 for connecting the system 100 with other devices and/or a communication network 105;
- a test patient data store 120 for storing one or more collections of features from patients (*e.g.,* subjects);
- a bioinformatics module 140 for processing sequencing data and extracting features from sequencing data, *e.g.,* from liquid biopsy sequencing assays;
- a feature analysis module 160 for evaluating patient features, *e.g.,* genomic alterations, compound genomic features, and clinical features; and
- a reporting module 180 for generating and transmitting reports that provide clinical support for personalized cancer therapy.

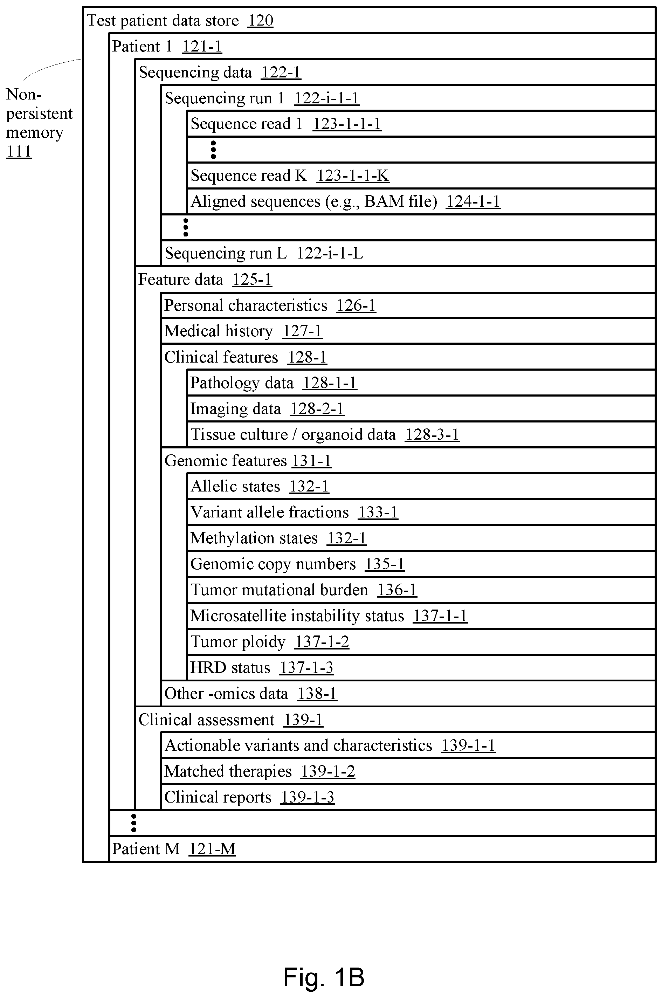

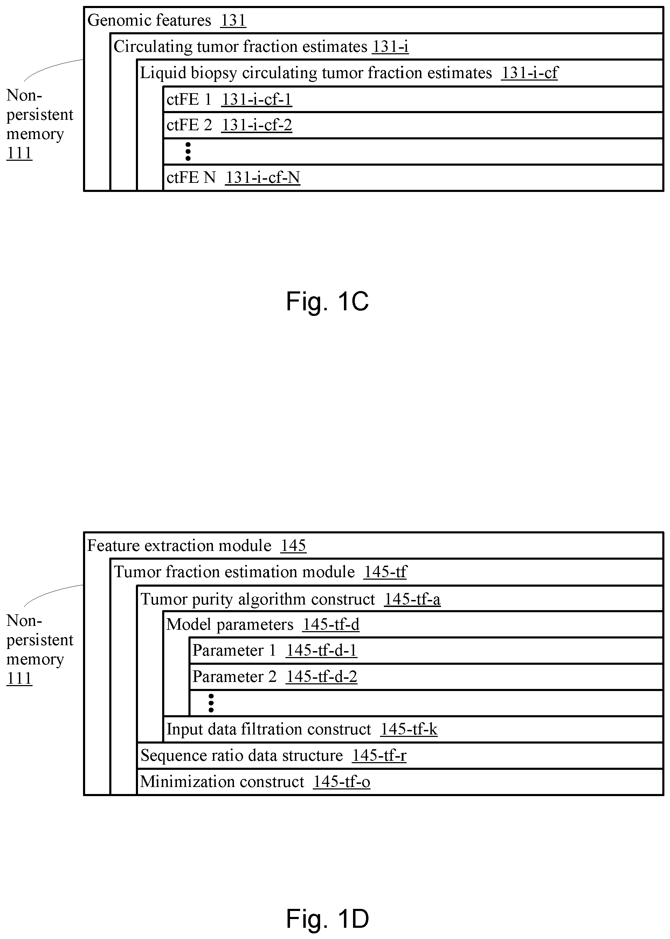

Although Figures 1A-1D depict a "system 100," the figures are intended more as a functional description of the various features that may be present in computer systems than as a structural schematic of the implementations described herein. In practice, and as recognized by those of ordinary skill in the art, items shown separately could be combined and some items could be separated. Moreover, although Figure 1 depicts certain data and modules in non-persistent memory 111, some or all of these data and modules may be in persistent memory 112. For example, in various implementations, one or more of the above identified elements are stored in one or more of the previously mentioned memory devices and correspond to a set of instructions for performing a function described above. The above identified modules, data, or programs (*e.g.,* sets of instructions) need not be implemented as separate software programs, procedures, datasets, or modules, and thus various subsets of these modules and data may be combined or otherwise re-arranged in various implementations.

In some implementations, the non-persistent memory 111 optionally stores a subset of the modules and data structures identified above. Furthermore, in some embodiments, the memory stores additional modules and data structures not described above. In some embodiments, one or more of the above-identified elements is stored in a computer system, other than that of system 100, that is addressable by system 100 so that system 100 may retrieve all or a portion of such data when needed.

For purposes of illustration in Figure 1A, system 100 is represented as a single computer that includes all of the functionality for providing clinical support for personalized cancer therapy. However, while a single machine is illustrated, the term "system" shall also be taken to include any collection of machines that individually or jointly execute a set (or multiple sets) of instructions to perform any one or more of the methodologies discussed herein.

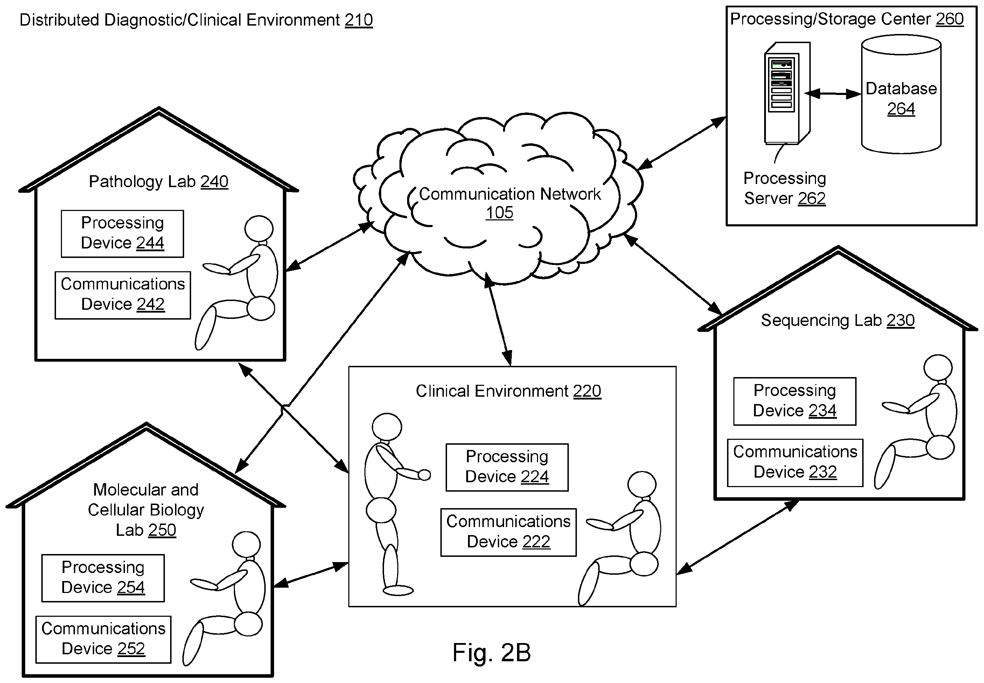

For example, in some embodiments, system 100 includes one or more computers. In some embodiments, the functionality for providing clinical support for personalized cancer therapy is spread across any number of networked computers and/or resides on each of several networked computers and/or is hosted on one or more virtual machines at a remote location accessible across the communications network 105. For example, different portions of the various modules and data stores illustrated in Figures 1A-1D can be stored and/or executed on the various instances of a processing device and/or processing server/database in the distributed diagnostic environment 210 illustrated in Figure 2B (*e.g.,* processing devices 224, 234, 244, and 254, processing server 262, and database 264).

The system may operate in the capacity of a server or a client machine in client-server network environment, as a peer machine in a peer-to-peer (or distributed) network environment, or as a server or a client machine in a cloud computing infrastructure or environment. The system may be a personal computer (PC), a tablet PC, a set-top box (STB), a Personal Digital Assistant (PDA), a cellular telephone, a web appliance, a server, a network router, a switch or bridge, or any machine capable of executing a set of instructions (sequential or otherwise) that specify actions to be taken by that machine.

In another implementation, the system comprises a virtual machine that includes a module for executing instructions for performing any one or more of the methodologies disclosed herein. In computing, a virtual machine (VM) is an emulation of a computer system that is based on computer architectures and provides functionality of a physical computer. Some such implementations may involve specialized hardware, software, or a combination of hardware and software.

One of skill in the art will appreciate that any of a wide array of different computer topologies are used for the application and all such topologies are within the scope of the present disclosure.

### Test Patient Data Store (120)

Referring to Figure 1B, in some embodiments, the system (*e.g.,* system 100) includes a patient data store 120 that stores data for patients 121-1 to 121-M (*e.g.,* cancer patients or patients being tested for cancer) including one or more sequencing data 122, feature data 125, and clinical assessments 139. These data are used and/or generated by the various processes stored in the bioinformatics module 140 and feature analysis module 160 of system 100, to ultimately generate a report providing clinical support for personalized cancer therapy of a patient. While the feature scope of patient data 121 across all patients may be informationally dense, an individual patient's feature set may be sparsely populated across the entirety of the collective feature scope of all features across all patients. That is to say, the data stored for one patient may include a different set of features that the data stored for another patient. Further, while illustrated as a single data construct in Figure 1B, different sets of patient data may be stored in different databases or modules spread across one or more system memories.

In some embodiments, sequencing data 122 from one or more sequencing reactions 122-i, including a plurality of sequence reads 123-i-1 to 123-i-K, is stored in the test patient data store 120. The data store may include different sets of sequencing data from a single subject, corresponding to different samples from the patient, *e.g.,* a tumor sample, liquid biopsy sample, tumor organoid derived from a patient tumor, and/or a normal sample, and/or to samples acquired at different times, *e.g.,* while monitoring the progression, regression, remission, and/or recurrence of a cancer in a subject. The sequence reads may be in any suitable file format, *e.g.,* BCL, FASTA, FASTQ, *etc.* In some embodiments, sequencing data 122 is accessed by a sequencing data processing module 141, which performs various pre-processing, genome alignment, and demultiplexing operations, as described in detail below with reference to bioinformatics module 140. In some embodiments, sequence data that has been aligned to a reference construct, *e.g.,* BAM file 124, is stored in test patient data store 120.

In some embodiments, the test patient data store 120 includes feature data 125, *e.g.,* that is useful for identifying clinical support for personalized cancer therapy. In some embodiments, the feature data 125 includes personal characteristics 126 of the patient, such as patient name, date of birth, gender, ethnicity, physical address, smoking status, alcohol consumption characteristic, anthropomorphic data, *etc.*

In some embodiments, the feature data 125 includes medical history data 127 for the patient, such as cancer diagnosis information (*e.g.,* date of initial diagnosis, date of metastatic diagnosis, cancer staging, tumor characterization, tissue of origin, previous treatments and outcomes, adverse effects of therapy, therapy group history, clinical trial history, previous and current medications, surgical history, *etc*.), previous or current symptoms, previous or current therapies, previous treatment outcomes, previous disease diagnoses, diabetes status, diagnoses of depression, diagnoses of other physical or mental maladies, and family medical history. In some embodiments, the feature data 125 includes clinical features 128, such as pathology data 128-1, medical imaging data 128-2, and tissue culture and/or tissue organoid culture data 128-3.

In some embodiments, yet other clinical features, such as previous laboratory testing results, are stored in the test patient data store 120. Medical history data 127 and clinical features may be collected from various sources, including at intake directly from the patient, from an electronic medical record (EMR) or electronic health record (EHR) for the patient, or curated from other sources, such as fields from various testing records (*e.g.,* genetic sequencing reports).

In some embodiments, the feature data 125 includes genomic features 131 for the patient. Non-limiting examples of genomic features include allelic states 132 (*e.g.,* the identity of alleles at one or more loci, support for wild type or variant alleles at one or more loci, support for SNVs/MNVs at one or more loci, support for indels at one or more loci, and/or support for gene rearrangements at one or more loci), allelic fractions 133 (*e.g.,* ratios of variant to reference alleles (or vice versa), methylation states 132 (*e.g.,* a distribution of methylation patterns at one or more loci and/or support for aberrant methylation patterns at one or more loci), genomic copy numbers 135 (*e.g.,* a copy number value at one or more loci and/or support for an aberrant (increased or decreased) copy number at one or more loci), tumor mutational burden 136 (*e.g.,* a measure of the number of mutations in the cancer genome of the subject), and microsatellite instability status 137 (*e.g.,* a measure of the repeated unit length at one or more microsatellite loci and/or a classification of the MSI status for the patient's cancer). In some embodiments, one or more of the genomic features 131 are determined by a nucleic acid bioinformatics pipeline, *e.g.,* as described in detail below with reference to Figures 4A-4F. In particular, in some embodiments, the feature data 125 include circulating tumor fraction estimates 131-i, as determined using the improved methods for determining circulating tumor fraction estimates, as described in further detail below with reference to Figures 1C, 1D, and 4F. In some embodiments, one or more of the genomic features 131 are obtained from an external testing source, *e.g.,* not connected to the bioinformatics pipeline as described below.

In some embodiments, the feature data 125 further includes data 138 from other -omics fields of study. Non-limiting examples of -omics fields of study that may yield feature data useful for providing clinical support for personalized cancer therapy include transcriptomics, epigenomics, proteomics, metabolomics, metabonomics, microbiomics, lipidomics, glycomics, cellomics, and organoidomics.

In some embodiments, yet other features may include features derived from machine learning approaches, *e.g.,* based at least in part on evaluation of any relevant molecular or clinical features, considered alone or in combination, not limited to those listed above. For instance, in some embodiments, one or more latent features from evaluation of cancer patient training datasets improve the diagnostic and prognostic power of the various analysis algorithms in the feature analysis module 160.

The skilled artisan will know of other types of features useful for providing clinical support for personalized cancer therapy. The listing of features above is merely representative and should not be construed to be limiting.

In some embodiments, a test patient data store 120 includes clinical assessment data 139 for patients, *e.g.,* based on the feature data 125 collected for the subject. In some embodiments, the clinical assessment data 139 includes a catalogue of actionable variants and characteristics 139-1 (*e.g.,* genomic alterations and compound metrics based on genomic features known or believed to be targetable by one or more specific cancer therapies), matched therapies 139-2 (*e.g.,* the therapies known or believed to be particularly beneficial for treatment of subjects having actionable variants), and/or clinical reports 139-3 generated for the subject, *e.g.,* based on identified actionable variants and characteristics 139-1 and/or matched therapies 139-2.

In some embodiments, clinical assessment data 139 is generated by analysis of feature data 125 using the various algorithms of feature analysis module 160, as described in further detail below. In some embodiments, clinical assessment data 139 is generated, modified, and/or validated by evaluation of feature data 125 by a clinician, *e.g.,* an oncologist. For instance, in some embodiments, a clinician (*e.g.,* at clinical environment 220) uses feature analysis module 160, or accesses test patient data store 120 directly, to evaluate feature data 125 to make recommendations for personalized cancer treatment of a patient. Similarly, in some embodiments, a clinician (*e.g.,* at clinical environment 220) reviews recommendations determined using feature analysis module 160 and approves, rejects, or modifies the recommendations, *e.g.,* prior to the recommendations being sent to a medical professional treating the cancer patient.

### Bioinformatics Module (140)

Referring again to Figure 1A, the system (*e.g.,* system 100) includes a bioinformatics module 140 that includes a feature extraction module 145 and optional ancillary data processing constructs, such as a sequence data processing module 141 and/or one or more reference sequence constructs 158 (*e.g.,* a reference genome, exome, or targeted-panel construct that includes reference sequences for a plurality of loci targeted by a sequencing panel).

In some embodiments, bioinformatics module 140 includes a sequence data processing module 141 that includes instructions for processing sequence reads, *e.g.,* raw sequence reads 123 from one or more sequencing reactions 122, prior to analysis by the various feature extraction algorithms, as described in detail below. In some embodiments, sequence data processing module 141 includes one or more pre-processing algorithms 142 that prepare the data for analysis. In some embodiments, the pre-processing algorithms 142 include instructions for converting the file format of the sequence reads from the output of the sequencer (*e.g.,* a BCL file format) into a file format compatible with downstream analysis of the sequences (*e.g.,* a FASTQ or FASTA file format). In some embodiments, the pre-processing algorithms 142 include instructions for evaluating the quality of the sequence reads (*e.g.,* by interrogating quality metrics like Phred score, base-calling error probabilities, Quality (Q) scores, and the like) and/or removing sequence reads that do not satisfy a threshold quality (*e.g.,* an inferred base call accuracy of at least 80%, at least 90%, at least 95%, at least 99%, at least 99.5%, at least 99.9%, or higher). In some embodiments, the pre-processing algorithms 142 include instructions for filtering the sequence reads for one or more properties, *e.g.,* removing sequences failing to satisfy a lower or upper size threshold or removing duplicate sequence reads.

In some embodiments, sequence data processing module 141 includes one or more alignment algorithms 143, for aligning pre-processed sequence reads 123 to a reference sequence construct 158, *e.g.,* a reference genome, exome, or targeted-panel construct. Many algorithms for aligning sequencing data to a reference construct are known in the art, for example, BWA, Blat, SHRiMP, LastZ, and MAQ. One example of a sequence read alignment package is the Burrows-Wheeler Alignment tool (BWA), which uses a Burrows-Wheeler Transform (BWT) to align short sequence reads against a large reference construct, allowing for mismatches and gaps. Li and Durbin, Bioinformatics, 25(14):1754-60 (2009), the content of which is incorporated herein by reference, in its entirety, for all purposes. Sequence read alignment packages import raw or pre-processed sequence reads 122, *e.g.,* in BCL, FASTA, or FASTQ file formats, and output aligned sequence reads 124, *e.g.,* in SAM or BAM file formats.

In some embodiments, sequence data processing module 141 includes one or more demultiplexing algorithms 144, for dividing sequence read or sequence alignment files generated from sequencing reactions of pooled nucleic acids into separate sequence read or sequence alignment files, each of which corresponds to a different source of nucleic acids in the nucleic acid sequencing pool. For instance, because of the cost of sequencing reactions, it is common practice to pool nucleic acids from a plurality of samples into a single sequencing reaction. The nucleic acids from each sample are tagged with a sample-specific and/or molecule-specific sequence tag (*e.g.,* a UMI), which is sequenced along with the molecule. In some embodiments, demultiplexing algorithms 144 sort these sequence tags in the sequence read or sequence alignment files to demultiplex the sequencing data into separate files for each of the samples included in the sequencing reaction.

Bioinformatics module 140 includes a feature extraction module 145, which includes instructions for identifying diagnostic features, *e.g.,* genomic features 131, from sequencing data 122 of biological samples from a subject, *e.g.,* one or more of a solid tumor sample, a liquid biopsy sample, or a normal tissue (*e.g.,* control) sample. For instance, in some embodiments, a feature extraction algorithm compares the identity of one or more nucleotides at a locus from the sequencing data 122 to the identity of the nucleotides at that locus in a reference sequence construct (*e.g.,* a reference genome, exome, or targeted-panel construct) to determine whether the subject has a variant at that locus. In some embodiments, a feature extraction algorithm evaluates data other than the raw sequence, to identify a genomic alteration in the subject, *e.g.,* an allelic ratio, a relative copy number, a repeat unit distribution, *etc.*

For instance, in some embodiments, feature extraction module 145 includes one or more variant identification modules that include instructions for various variant calling processes. In some embodiments, variants in the germline of the subject are identified, *e.g.,* using a germline variant identification module 146. In some embodiments, variants in the cancer genome, *e.g.,* somatic variants, are identified, *e.g.,* using a somatic variant identification module 150. While separate germline and somatic variant identification modules are illustrated in Figure 1A, in some embodiments they are integrated into a single module. In some embodiments, the variant identification module includes instructions for identifying one or more of nucleotide variants (*e.g.,* single nucleotide variants (SNV) and multi-nucleotide variants (MNV)) using one or more SNV/MNV calling algorithms (*e.g.,* algorithms 147 and/or 151), indels (*e.g.,* insertions or deletions of nucleotides) using one or more indel calling algorithms (*e.g.,* algorithms 148 and/or 152), and genomic rearrangements (*e.g.,* inversions, translocation, and fusions of nucleotide sequences) using one or more genomic rearrangement calling algorithms (*e.g.,* algorithms 149 and/or 153).

A SNV/MNV algorithm 147 may identify a substitution of a single nucleotide that occurs at a specific position in the genome. For example, at a specific base position, or locus, in the human genome, the C nucleotide may appear in most individuals, but in a minority of individuals, the position is occupied by an A. This means that there is a SNP at this specific position and the two possible nucleotide variations, C or A, are said to be alleles for this position. SNPs underlie differences in human susceptibility to a wide range of diseases (*e.g. -* sickle-cell anemia, β-thalassemia and cystic fibrosis result from SNPs). The severity of illness and the way the body responds to treatments are also manifestations of genetic variations. For example, a single-base mutation in the APOE (apolipoprotein E) gene is associated with a lower risk for Alzheimer's disease. A single-nucleotide variant (SNV) is a variation in a single nucleotide without any limitations of frequency and may arise in somatic cells. A somatic single-nucleotide variation (*e.g.,* caused by cancer) may also be called a single-nucleotide alteration. An MNP (Multiple-nucleotide polymorphisms) module may identify the substitution of consecutive nucleotides at a specific position in the genome.

An indel calling algorithm 148 may identify an insertion or deletion of bases in the genome of an organism classified among small genetic variations. While indels usually measure from 1 to 10 000 base pairs in length, a microindel is defined as an indel that results in a net change of 1 to 50 nucleotides. Indels can be contrasted with a SNP or point mutation. An indel inserts and/or deletes nucleotides from a sequence, while a point mutation is a form of substitution that replaces one of the nucleotides without changing the overall number in the DNA. Indels, being insertions and/or deletions, can be used as genetic markers in natural populations, especially in phylogenetic studies. Indel frequency tends to be markedly lower than that of single nucleotide polymorphisms (SNP), except near highly repetitive regions, including homopolymers and microsatellites.

A genomic rearrangement algorithm 149 may identify hybrid genes formed from two previously separate genes. It can occur as a result of translocation, interstitial deletion, or chromosomal inversion. Gene fusion can play an important role in tumorigenesis. Fusion genes can contribute to tumor formation because fusion genes can produce much more active abnormal protein than non-fusion genes. Often, fusion genes are oncogenes that cause cancer; these include BCR-ABL, TEL-AML1 (ALL with t(12 ; 21)), AML1-ETO (M2 AML with t(8 ; 21)), and TMPRSS2-ERG with an interstitial deletion on chromosome 21, often occurring in prostate cancer. In the case of TMPRSS2-ERG, by disrupting androgen receptor (AR) signaling and inhibiting AR expression by oncogenic ETS transcription factor, the fusion product regulates prostate cancer. Most fusion genes are found from hematological cancers, sarcomas, and prostate cancer. BCAM-AKT2 is a fusion gene that is specific and unique to high-grade serous ovarian cancer. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. The latter is common in lymphomas, where oncogenes are juxtaposed to the promoters of the immunoglobulin genes. Oncogenic fusion transcripts may also be caused by trans-splicing or read-through events. Since chromosomal translocations play such a significant role in neoplasia, a specialized database of chromosomal aberrations and gene fusions in cancer has been created. This database is called Mitelman Database of Chromosome Aberrations and Gene Fusions in Cancer.

In some embodiments, feature extraction module 145 includes instructions for identifying one or more complex genomic alterations (*e.g.,* features that incorporate more than a change in the primary sequence of the genome) in the cancer genome of the subject. For instance, in some embodiments, feature extraction module 145 includes modules for identifying one or more of copy number variation (*e.g.,* copy number variation analysis module 153), microsatellite instability status (*e.g.,* microsatellite instability analysis module 154), tumor mutational burden (*e.g.,* tumor mutational burden analysis module 155), tumor ploidy (*e.g.,* tumor ploidy analysis module 156), and homologous recombination pathway deficiencies (*e.g.,* homologous recombination pathway analysis module 157).

For example, referring to Figure 1D, in some embodiments, feature extraction module 145 comprises a tumor fraction estimation module 145-tf. In some embodiments, the tumor fraction estimation module 145-tf comprises a sequence ratio data structure 145-tf-r including a plurality of sequence ratios (*e.g.,* coverage ratios) obtained from a sequencing of a test liquid biopsy sample of a subject. In some embodiments, the sequence ratio data structure 145-tf-r includes the sequence ratios that are used as input to determine tumor fraction estimates for the test liquid biopsy sample. In some embodiments, the tumor fraction estimation module 145-tf also comprises a tumor purity algorithm construct 145-tf-a that executes, for example, a maximum likelihood estimation (*e.g.,* an expectation-maximization algorithm) to calculate an estimate of the circulating tumor fraction. The tumor plurality algorithm construct 145-tf-a comprises an optional input data filtration construct 145-tf-k (*e.g.,* for removing one or more inputs passed from the sequence ratio data structure based on a minimum probe threshold or a position on a sex chromosome) and a plurality of model parameters 145-tf-d (*e.g.,* 145-tf-d-1, 145-tf-d-2,...) used for executing the algorithm. In some embodiments, model parameters include expected sequence ratios for a set of copy states at a given tumor purity; a distance (*e.g.,* an error) from a test sequence ratio to the closest expected sequence ratio at the given tumor purity; a minimum distance (*e.g.,* a minimum error) from a test sequence ratio to the closest expected sequence ratio at the given tumor purity (*e.g.,* an assigned test copy state selected from a minimal distance expected copy state); and/or a tumor purity score (*e.g.,* a sum of weighted errors).

In some embodiments, referring to Figure 1C, the tumor fraction estimation module 145-tf is used to obtain one or more circulating tumor fraction estimates 131-i that are included as feature data 125 in a test patient data store 120. For example, in some embodiments, a plurality of circulating tumor fraction estimates is obtained from a test liquid biopsy sample of a subject 131-i-cf (*e.g.,* 131-i-cf-1, 131-i-cf-2..., 131-i-cf-N). In some embodiments, the plurality of circulating tumor fraction estimates is obtained from a single patient at different collection times.

### Feature Analysis Module (160).

Referring again to Figure 1A, the system (*e.g.,* system 100) includes a feature analysis module 160 that includes one or more genomic alteration interpretation algorithms 161, one or more optional clinical data analysis algorithms 165, an optional therapeutic curation algorithm 165, and an optional recommendation validation module 167. In some embodiments, feature analysis module 160 identifies actionable variants and characteristics 139-1 and corresponding matched therapies 139-2 and/or clinical trials using one or more analysis algorithms (*e.g.,* algorithms 162, 163, 164, and 165) to evaluate feature data 125. The identified actionable variants and characteristics 139-1 and corresponding matched therapies 139-2, which are optionally stored in test patient data store 120, are then curated by feature analysis module 160 to generate a clinical report 139-3, which is optionally validated by a user, *e.g.,* a clinician, before being transmitted to a medical professional, *e.g.,* an oncologist, treating the patient.

In some embodiments, the genomic alteration interpretation algorithms 161 include instructions for evaluating the effect that one or more genomic features 131 of the subject, *e.g.,* as identified by feature extraction module 145, have on the characteristics of the patient's cancer and/or whether one or more targeted cancer therapies may improve the clinical outcome for the patient. For example, in some embodiments, one or more genomic variant analysis algorithms 163 evaluate various genomic features 131 by querying a database, *e.g.,* a look-up-table ("LUT") of actionable genomic alterations, targeted therapies associated with the actionable genomic alterations, and any other conditions that should be met before administering the targeted therapy to a subject having the actionable genomic alteration. For instance, evidence suggests that depatuxizumab mafodotin (an anti-EGFR mAb conjugated to monomethyl auristatin F) has improved efficacy for the treatment of recurrent glioblastomas having EGFR focal amplifications. van den Bent et al., 2017, Cancer Chemother Pharmacol., 80(6):1209-17. Accordingly, the actionable genomic alteration LUT would have an entry for the focal amplification of the EGFR gene indicating that depatuxizumab mafodotin is a targeted therapy for glioblastomas (*e.g.,* recurrent glioblastomas) having a focal gene amplification. In some instances, the LUT may also include counter indications for the associated targeted therapy, *e.g.,* adverse drug interactions or personal characteristics that are counter-indicated for administration of the particular targeted therapy.

In some embodiments, a genomic alteration interpretation algorithm 161 determines whether a particular genomic feature 131 should be reported to a medical professional treating the cancer patient. In some embodiments, genomic features 131 (*e.g.,* genomic alterations and compound features) are reported when there is clinical evidence that the feature significantly impacts the biology of the cancer, impacts the prognosis for the cancer, and/or impacts pharmacogenomics, *e.g.,* by indicating or counter-indicating particular therapeutic approaches. For instance, a genomic alteration interpretation algorithm 161 may classify a particular CNV feature 135 as "Reportable," *e.g.,* meaning that the CNV has been identified as influencing the character of the cancer, the overall disease state, and/or pharmacogenomics, as "Not Reportable," *e.g.,* meaning that the CNV has not been identified as influencing the character of the cancer, the overall disease state, and/or pharmacogenomics, as "No Evidence," *e.g.,* meaning that no evidence exists supporting that the CNV is "Reportable" or "Not Reportable," or as "Conflicting Evidence," *e.g.,* meaning that evidence exists supporting both that the CNV is "Reportable" and that the CNV is "Not Reportable."

In some embodiments, the genomic alteration interpretation algorithms 161 include one or more pathogenic variant analysis algorithms 162, which evaluate various genomic features to identify the presence of an oncogenic pathogen associated with the patient's cancer and/or targeted therapies associated with an oncogenic pathogen infection in the cancer. For instance, RNA expression patterns of some cancers are associated with the presence of an oncogenic pathogen that is helping to drive the cancer. See, for example, U.S. Patent Application Serial No. 16/802,126, filed February 26, 2020, the content of which is hereby incorporated by reference, in its entirety, for all purposes. In some instances, the recommended therapy for the cancer is different when the cancer is associated with the oncogenic pathogen infection than when it is not. Accordingly, in some embodiments, *e.g.,* where feature data 125 includes RNA abundance data for the cancer of the patient, one or more pathogenic variant analysis algorithms 162 evaluate the RNA abundance data for the patient's cancer to determine whether a signature exists in the data that indicates the presence of the oncogenic pathogen in the cancer. Similarly, in some embodiments, bioinformatics module 140 includes an algorithm that searches for the presence of pathogenic nucleic acid sequences in sequencing data 122. See, for example, U.S. Provisional Patent Application Serial No. 62/978,067, filed February 18, 2020, the content of which is hereby incorporated by reference, in its entirety, for all purposes. Accordingly, in some embodiments, one or more pathogenic variant analysis algorithms 162 evaluates whether the presence of an oncogenic pathogen in a subject is associated with an actionable therapy for the infection. In some embodiments, system 100 queries a database, *e.g.,* a look-up-table ("LUT"), of actionable oncogenic pathogen infections, targeted therapies associated with the actionable infections, and any other conditions that should be met before administering the targeted therapy to a subject that is infected with the oncogenic pathogen. In some instances, the LUT may also include counter indications for the associated targeted therapy, *e.g.,* adverse drug interactions or personal characteristics that are counter-indicated for administration of the particular targeted therapy.

In some embodiments, the genomic alteration interpretation algorithms 161 include one or more multi-feature analysis algorithms 164 that evaluate a plurality of features to classify a cancer with respect to the effects of one or more targeted therapies. For instance, in some embodiments, feature analysis module 160 includes one or more classifiers trained against feature data, one or more clinical therapies, and their associated clinical outcomes for a plurality of training subjects to classify cancers based on their predicted clinical outcomes following one or more therapies.

In some embodiments, the classifier is implemented as an artificial intelligence engine and may include gradient boosting models, random forest models, neural networks (NN), regression models, Naive Bayes models, and/or machine learning algorithms (MLA). An MLA or a NN may be trained from a training data set that includes one or more features 125, including personal characteristics 126, medical history 127, clinical features 128, genomic features 131, and/or other -omic features 138. MLAs include supervised algorithms (such as algorithms where the features/classifications in the data set are annotated) using linear regression, logistic regression, decision trees, classification and regression trees, naïve Bayes, nearest neighbor clustering; unsupervised algorithms (such as algorithms where no features/classification in the data set are annotated) using Apriori, means clustering, principal component analysis, random forest, adaptive boosting; and semi-supervised algorithms (such as algorithms where an incomplete number of features/classifications in the data set are annotated) using generative approach (such as a mixture of Gaussian distributions, mixture of multinomial distributions, hidden Markov models), low density separation, graph-based approaches (such as mincut, harmonic function, manifold regularization), heuristic approaches, or support vector machines.

NNs include conditional random fields, convolutional neural networks, attention based neural networks, deep learning, long short term memory networks, or other neural models where the training data set includes a plurality of tumor samples, RNA expression data for each sample, and pathology reports covering imaging data for each sample.

While MLA and neural networks identify distinct approaches to machine learning, the terms may be used interchangeably herein. Thus, a mention of MLA may include a corresponding NN or a mention of NN may include a corresponding MLA unless explicitly stated otherwise. Training may include providing optimized datasets, labeling these traits as they occur in patient records, and training the MLA to predict or classify based on new inputs. Artificial NNs are efficient computing models which have shown their strengths in solving hard problems in artificial intelligence. They have also been shown to be universal approximators, that is, they can represent a wide variety of functions when given appropriate parameters.

In some embodiments, system 100 includes a classifier training module that includes instructions for training one or more untrained or partially trained classifiers based on feature data from a training dataset. In some embodiments, system 100 also includes a database of training data for use in training the one or more classifiers. In other embodiments, the classifier training module accesses a remote storage device hosting training data. In some embodiments, the training data includes a set of training features, including but not limited to, various types of the feature data 125 illustrated in Figure 1B. In some embodiments, the classifier training module uses patient data 121, e.g., when test patient data store 120 also stores a record of treatments administered to the patient and patient outcomes following therapy.

In some embodiments, feature analysis module 160 includes one or more clinical data analysis algorithms 165, which evaluate clinical features 128 of a cancer to identify targeted therapies which may benefit the subject. For example, in some embodiments, *e.g.,* where feature data 125 includes pathology data 128-1, one or more clinical data analysis algorithms 165 evaluate the data to determine whether an actionable therapy is indicated based on the histopathology of a tumor biopsy from the subject, *e.g.,* which is indicative of a particular cancer type and/or stage of cancer. In some embodiments, system 100 queries a database, *e.g.,* a look-up-table ("LUT"), of actionable clinical features (*e.g.,* pathology features), targeted therapies associated with the actionable features, and any other conditions that should be met before administering the targeted therapy to a subject associated with the actionable clinical features 128 (*e.g.,* pathology features 128-1). In some embodiments, system 100 evaluates the clinical features 128 (*e.g.,* pathology features 128-1) directly to determine whether the patient's cancer is sensitive to a particular therapeutic agent. Further details on example methods, systems, and algorithms for classifying cancer and identifying targeted therapies based on clinical data, such as pathology data 128-1, imaging data 138-2, and/or tissue culture/organoid data 128-3 are discussed, for example, in U.S. Patent Application No. 16/830,186, filed on March 25, 2020, U.S. Patent Application No. 16/789,363, filed on Feb. 12, 2020, and U.S. Provisional Application No. 63/007,874, filed on April. 9, 2020, the contents of which are hereby incorporated by reference, in their entireties, for all purposes.

In some embodiments, feature analysis module 160 includes a clinical trials module that evaluates test patient data 121 to determine whether the patient is eligible for inclusion in a clinical trial for a cancer therapy, *e.g.,* a clinical trial that is currently recruiting patients, a clinical trial that has not yet begun recruiting patients, and/or an ongoing clinical trial that may recruit additional patients in the future. In some embodiments, a clinical trial module evaluates test patient data 121 to determine whether the results of a clinical trial are relevant for the patient, *e.g.,* the results of an ongoing clinical trial and/or the results of a completed clinical trial. For instance, in some embodiments, system 100 queries a database, *e.g.,* a look-up-table ("LUT") of clinical trials, *e.g.,* active and/or completed clinical trials, and compares patient data 121 with inclusion criteria for the clinical trials, stored in the database, to identify clinical trials with inclusion criteria that closely match and/or exactly match the patient's data 121. In some embodiments, a record of matching clinical trials, *e.g.,* those clinical trials that the patient may be eligible for and/or that may inform personalized treatment decisions for the patient, are stored in clinical assessment database 139.

In some embodiments, feature analysis module 160 includes a therapeutic curation algorithm 166 that assembles actionable variants and characteristics 139-1, matched therapies 139-2, and/or relevant clinical trials identified for the patient, as described above. In some embodiments, a therapeutic curation algorithm 166 evaluates certain criteria related to which actionable variants and characteristics 139-1, matched therapies 139-2, and/or relevant clinical trials should be reported and/or whether certain matched therapies, considered alone or in combination, may be counter-indicated for the patient, *e.g.,* based on personal characteristics 126 of the patient and/or known drug-drug interactions. In some embodiments, the therapeutic curation algorithm then generates one or more clinical reports 139-3 for the patient. In some embodiments, the therapeutic curation algorithm generates a first clinical report 139-3-1 that is to be reported to a medical professional treating the patient and a second clinical report 139-3-2 that will not be communicated to the medical professional, but may be used to improve various algorithms within the system.

In some embodiments, feature analysis module 160 includes a recommendation validation module 167 that includes an interface allowing a clinician to review, modify, and approve a clinical report 139-3 prior to the report being sent to a medical professional, *e.g.,* an oncologist, treating the patient.

In some embodiments, each of the one or more feature collections, sequencing modules, bioinformatics modules (including, *e.g.,* alteration module(s), structural variant calling and data processing modules), classification modules and outcome modules are communicatively coupled to a data bus to transfer data between each module for processing and/or storage. In some alternative embodiments, each of the feature collection, alteration module(s), structural variant and feature store are communicatively coupled to each other for independent communication without sharing the data bus.

Further details on systems and exemplary embodiments of modules and feature collections are discussed in PCT Publication Number WO2020/142551, which is hereby incorporated herein by reference in its entirety.

### Example Methods

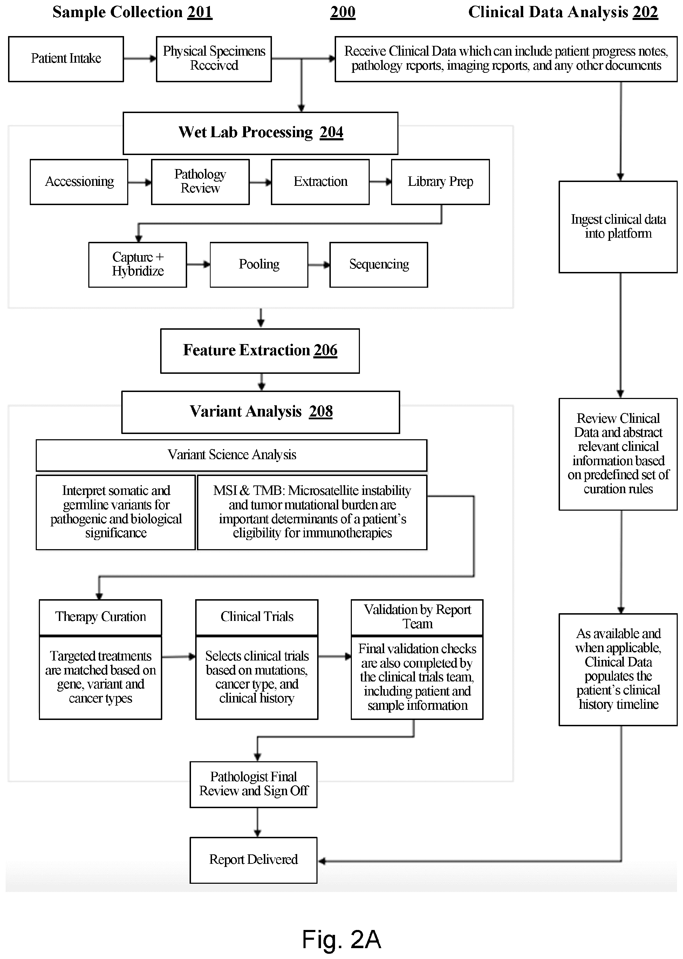

Now that details of a system 100 for detecting whether a variant in a biopsy from a subject is (a) a somatic variant derived from cell free DNA or (b) other than a somatic variant derived from cell free DNA have been disclosed, details regarding processes and features of the system, in accordance with various embodiments of the present disclosure, are disclosed below. Specifically, example processes are described below with reference to Figures 2A, 3, 4A-4E, and 5A-5G. In some embodiments, such processes and features of the system are carried out by modules 118, 120, 140, 160, and/or 170, as illustrated in Figure 1A. Referring to these methods, the systems described herein (*e.g.,* system 100) include instructions for detecting whether a variant in a biopsy from a subject is (a) a somatic variant derived from cell free DNA or (b) other than a somatic variant derived from cell free DNA that are improved compared to conventional methods.

### Figure 2B: Distributed Diagnostic and Clinical Environment

In some aspects, the methods described herein for providing clinical support for personalized cancer therapy are performed across a distributed diagnostic/clinical environment, *e.g.,* as illustrated in Figure 2B. However, in some embodiments, the improved methods described herein for detecting clonal hematopoiesis variants and/or solid tumor variants in a liquid biopsy assay, are performed at a single location, *e.g.,* at a single computing system or environment, although ancillary procedures supporting the methods described herein, and/or procedures that make further use of the results of the methods described herein, may be performed across a distributed diagnostic/clinical environment.

Figure 2B illustrates an example of a distributed diagnostic/clinical environment 210. In some embodiments, the distributed diagnostic/clinical environment is connected via communication network 105. In some embodiments, one or more biological samples, *e.g.,* one or more liquid biopsy samples, solid tumor biopsy, normal tissue samples, and/or control samples, are collected from a subject in clinical environment 220, *e.g.,* a doctor's office, hospital, or medical clinic, or at a home health care environment (not depicted). Advantageously, while solid tumor samples should be collected within a clinical setting, liquid biopsy samples can be acquired in a less invasive fashion and are more easily collected outside of a traditional clinical setting. In some embodiments, one or more biological samples, or portions thereof, are processed within the clinical environment 220 where collection occurred, using a processing device 224, *e.g.,* a nucleic acid sequencer for obtaining sequencing data, a microscope for obtaining pathology data, a mass spectrometer for obtaining proteomic data, *etc.* In some embodiments, one or more biological samples, or portions thereof are sent to one or more external environments, *e.g.,* sequencing lab 230, pathology lab 240, and/or molecular biology lab 250, each of which includes a processing device 234, 244, and 254, respectively, to generate biological data 121 for the subject. Each environment includes a communications device 222, 232, 242, and 252, respectively, for communicating biological data 121 about the subject to a processing server 262 and/or database 264, which may be located in yet another environment, *e.g.,* processing/storage center 260. Thus, in some embodiments, different portions of the systems and methods described herein are fulfilled by different processing devices located in different physical environments.

Accordingly, in some embodiments, a method for providing clinical support for personalized cancer therapy, *e.g.,* with improved detection of detecting whether a variant in a biopsy from a subject is (a) a somatic variant derived from cell free DNA or (b) other than a somatic variant derived from cell free DNA, is performed across one or more environments, as illustrated in Figure 2B. For instance, in some such embodiments, a liquid biopsy sample is collected at clinical environment 220 or in a home healthcare environment. The sample, or a portion thereof, is sent to sequencing lab 230 where raw sequence reads 123 of nucleic acids in the sample are generated by sequencer 234. The raw sequencing data 123 is communicated, *e.g.,* from communications device 232, to database 264 at processing/storage center 260, where processing server 262 extracts features from the sequence reads by executing one or more of the processes in bioinformatics module 140, thereby generating genomic features 131 for the sample. Processing server 262 may then analyze the identified features by executing one or more of the processes in feature analysis module 160, thereby generating clinical assessment 139, including a clinical report 139-3. A clinician may access clinical report 139-3, *e.g.,* at processing/storage center 260 or through communications network 105, via recommendation validation module 167. After final approval, clinical report 139-3 is transmitted to a medical professional, *e.g.,* an oncologist, at clinical environment 220, who uses the report to support clinical decision making for personalized treatment of the patient's cancer.

### Figure 2A: Example Workflow for Precision Oncology

Figure 2A is a flowchart of an example workflow 200 for collecting and analyzing data in order to generate a clinical report 139 to support clinical decision making in precision oncology. Advantageously, the methods described herein improve this process, for example, by improving various stages within feature extraction 206, including detecting clonal hematopoiesis variants and/or solid tumor variants in a liquid biopsy assay.

Briefly, the workflow begins with patient intake and sample collection 201, where one or more liquid biopsy samples, one or more tumor biopsy, and one or more normal and/or control tissue samples are collected from the patient (*e.g.,* at a clinical environment 220 or home healthcare environment, as illustrated in Figure 2B). In some embodiments, personal data 126 corresponding to the patient and a record of the one or more biological samples obtained (*e.g.,* patient identifiers, patient clinical data, sample type, sample identifiers, cancer conditions, *etc*.) are entered into a data analysis platform, *e.g.,* test patient data store 120. Accordingly, in some embodiments, the methods disclosed herein include obtaining one or more biological samples from one or more subjects, *e.g.,* cancer patients. In some embodiments, the subject is a human, *e.g.,* a human cancer patient.

Sequence reads are then generated (312) from the sequencing library or pool of sequencing libraries. Sequencing data may be acquired by any methodology known in the art. For example, next generation sequencing (NGS) techniques such as sequencing-by-synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), Ultima Sequencing (Ultima Genomics, Fremont California), or paired-end sequencing. In some embodiments, massively parallel sequencing is performed using sequencing-by-synthesis with reversible dye terminators. In some embodiments, sequencing is performed using next generation sequencing technologies, such as short-read technologies. In other embodiments, long-read sequencing or another sequencing method known in the art is used.

Referring again to Figure 2A, nucleic acid sequencing data 122 generated from the one or more patient samples is then evaluated *(e.g.,* via variant analysis 206) in a bioinformatics pipeline, *e.g.,* using bioinformatics module 140 of system 100, to identify genomic alterations and other metrics in the cancer genome of the patient. An example overview for a bioinformatics pipeline is described below with respect to Figures 4A-4E. Advantageously, in some embodiments, the present disclosure improves bioinformatics pipelines, like pipeline 206, by improving circulating tumor fraction estimates.

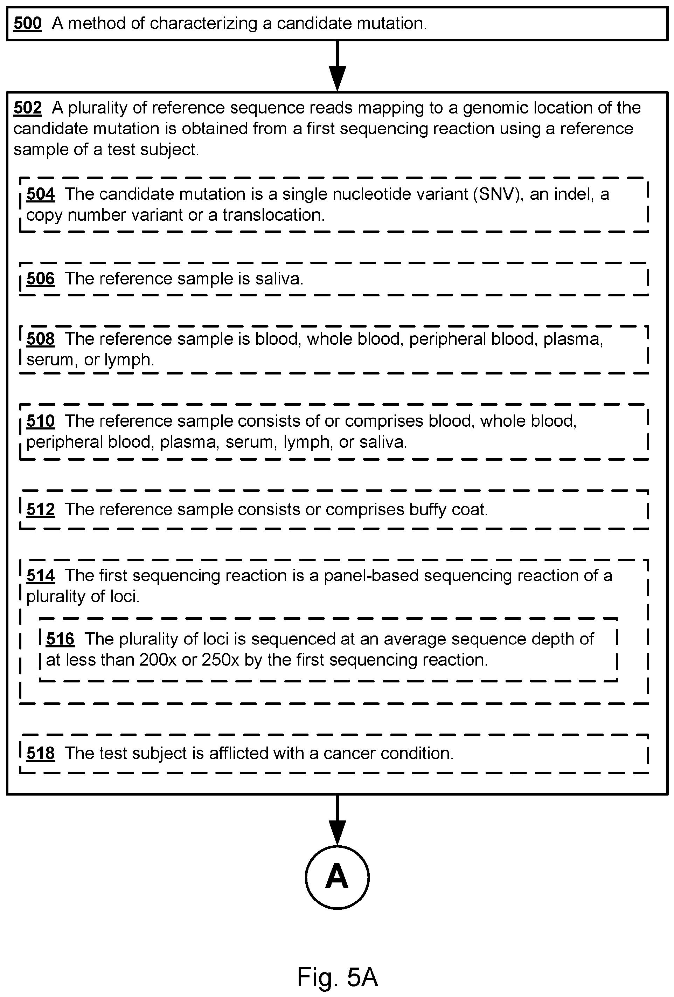

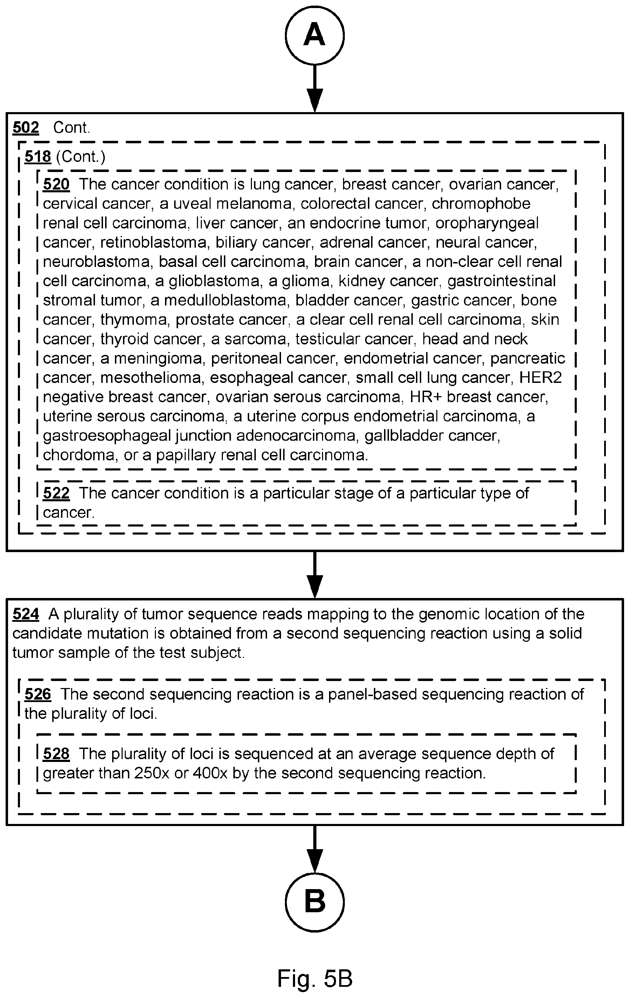

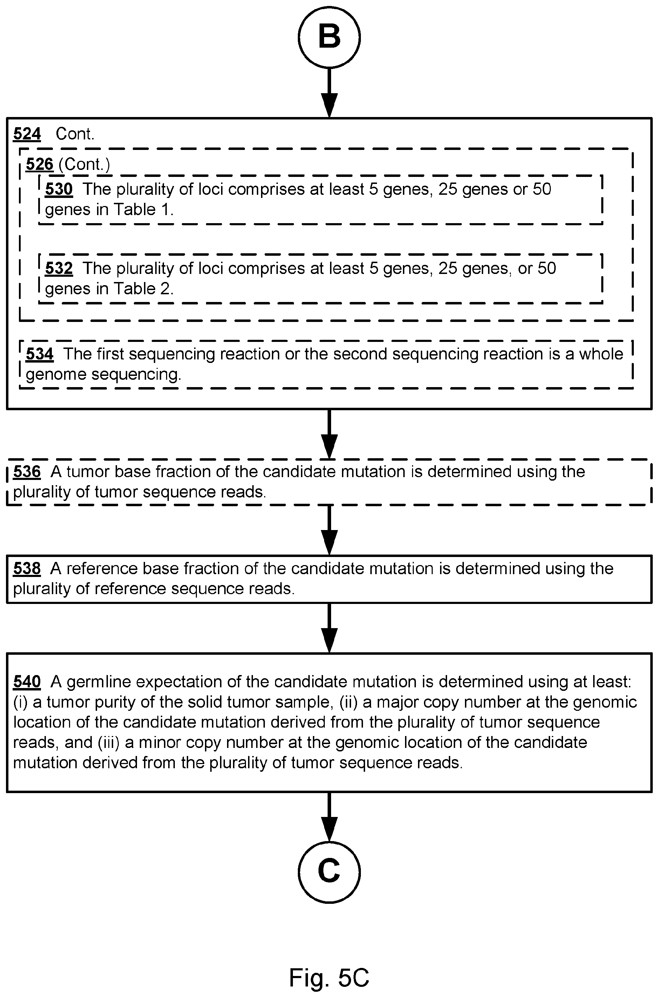

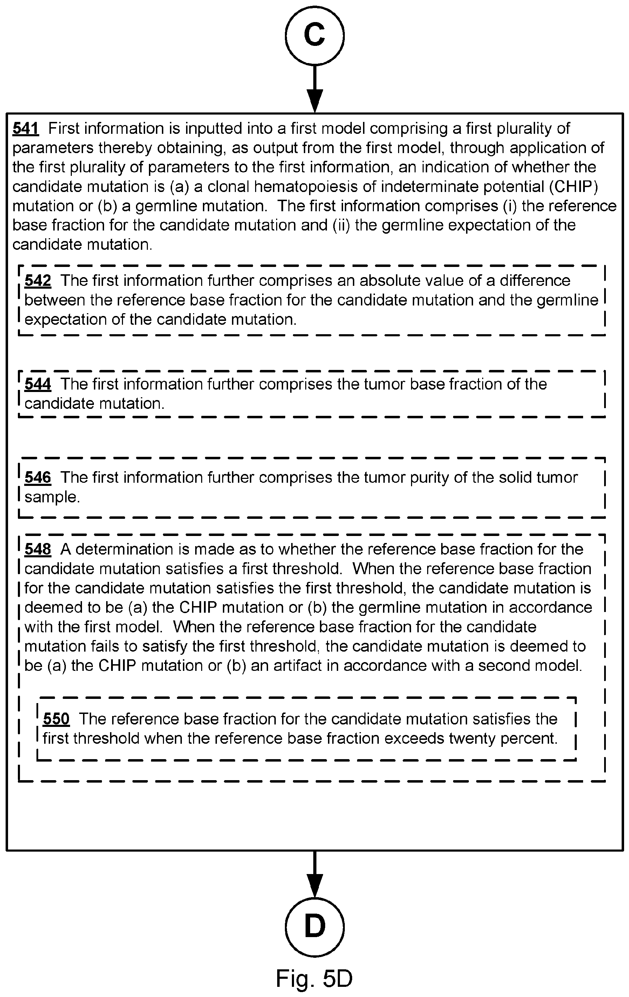

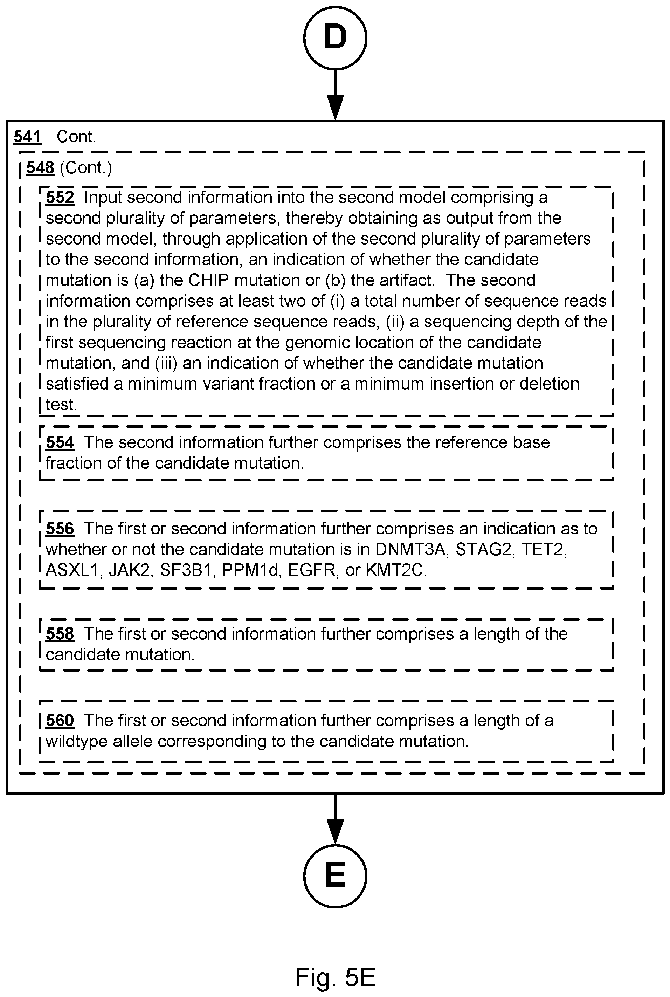

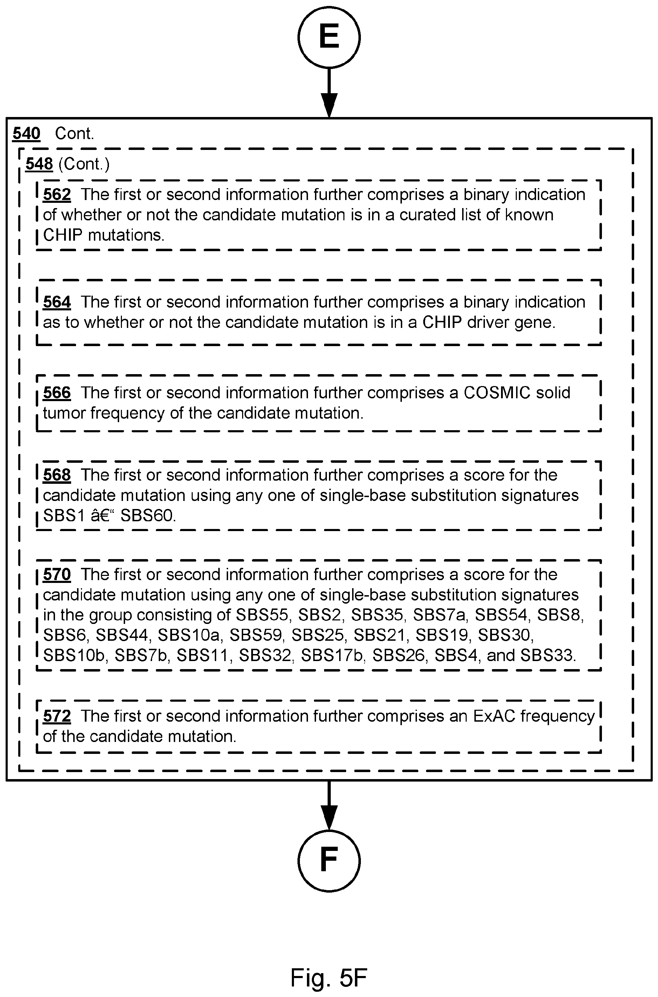

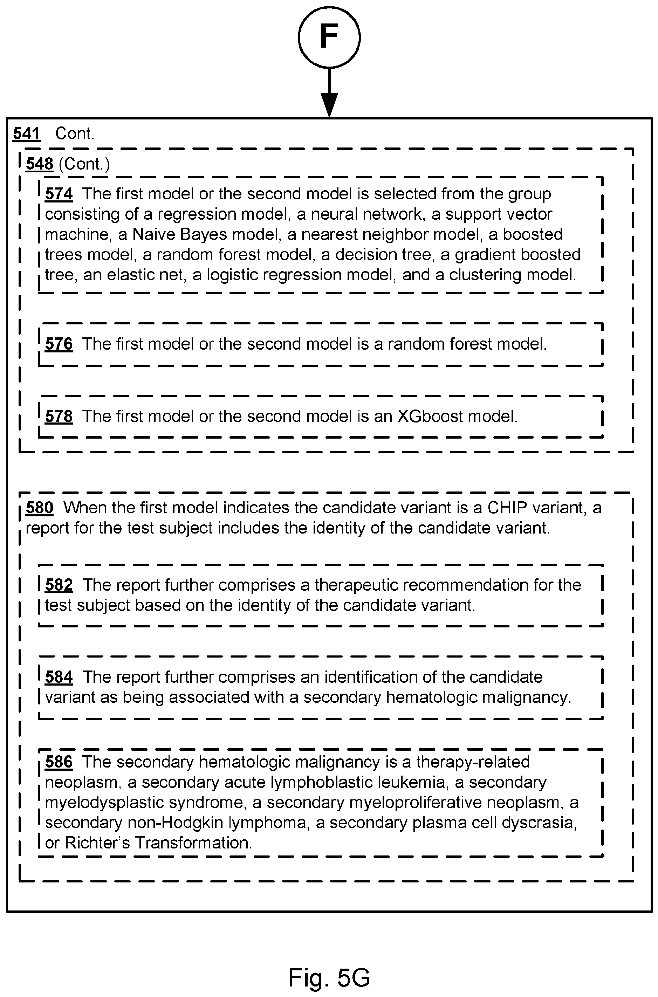

Further details of such sequencing are provided below in conjunction with Figure 5.

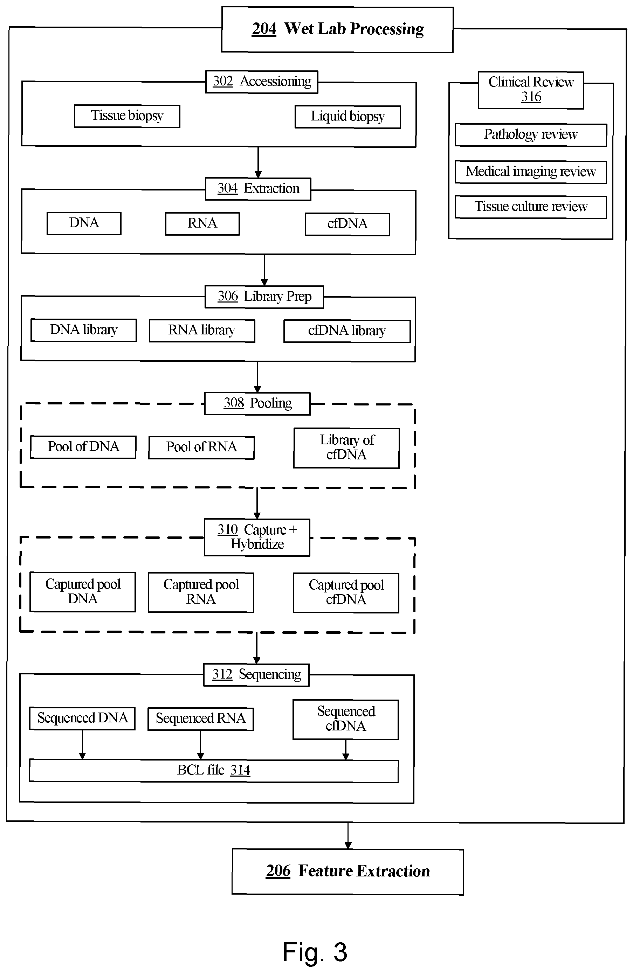

Figure 4A illustrates an example bioinformatics pipeline 206 (*e.g.,* as used for feature extraction in the workflows illustrated in Figures 2A and 3) for providing clinical support for precision oncology. As shown in Figure 4A, sequencing data 122 obtained from the wet lab processing 204 (*e.g.,* sequence reads 314) is input into the pipeline.

In various embodiments, the bioinformatics pipeline includes a circulating tumor DNA (ctDNA) pipeline for analyzing liquid biopsy samples. The pipeline may detect SNVs, INDELs, copy number amplifications/deletions and genomic rearrangements (for example, fusions). The pipeline may employ unique molecular index (UMI)-based consensus base calling as a method of error suppression as well as a Bayesian tri-nucleotide context-based position level error suppression. In various embodiments, it is able to detect variants having a 0.1%, 0.15%, 0.2%, 0.25%, 0.3%, 0.4%, or 0.5% variant allele fraction.

In some embodiments, variant analysis of aligned sequence reads, *e.g.,* in SAM or BAM format, includes identification of single nucleotide variants (SNVs), multiple nucleotide variants (MNVs), indels (*e.g.,* nucleotide additions and deletions), and/or genomic rearrangements (*e.g.,* inversions, translocations, and gene fusions) using variant identification module 146, *e.g.,* which includes a SNV/MNV calling algorithm (*e.g.,* SNV/MNV calling algorithm 147), an indel calling algorithm (*e.g.,* indel calling algorithm 148), and/or one or more genomic rearrangement calling algorithms (*e.g.,* genomic rearrangement calling algorithm 149). An overview of an example method for variant identification is shown in Figure 4E. Essentially, the module first identifies a difference between the sequence of an aligned sequence read 124 and the reference sequence to which the sequence read is aligned (*e.g.,* an SNV/MNV, an indel, or a genomic rearrangement) and makes a record of the variant, *e.g.,* in a variant call format (VCF) file. For instance, software packages such as freebayes and pindel are used to call variants using sorted BAM files and reference BED files as the input. For a review of variant calling packages see, for example, Cameron, D.L. et al., Nat. Commun., 10(3240):1-11 (2019). A raw VCF file (variant call format) file is output, showing the locations where the nucleotide base in the sample is not the same as the nucleotide base in that position in the reference sequence construct.

In some embodiments, as illustrated in Figure 4E, raw VCF data is then normalized, *e.g.,* by parsimony and left alignment. For example, software packages such as vcfbreakmulti and vt are used to normalize multi-nucleotide polymorphic variants in the raw VCF file and a variant normalized VCF file is output. See, for example, E. Garrison, "Vcflib: A C++ library for parsing and manipulating VCF files, GitHub, available on the internet at github.com/ekg/vcflib (2012), the content of which is hereby incorporated by reference, in its entirety, for all purposes. In some embodiments, a normalization algorithm is included within the architecture of a broader variant identification software package.

An algorithm is then used to annotate the variants in the (*e.g.,* normalized) VCF file, e.g., determines the source of the variation, *e.g.,* whether the variant is from the germline of the subject (*e.g.,* a germline variant), a cancerous tissue (*e.g.,* a somatic variant), a sequencing error, or of an undeterminable source. In some embodiments, an annotation algorithm is included within the architecture of a broader variant identification software package. However, in some embodiments, an external annotation algorithm is applied to (*e.g.,* normalized) VCF data obtained from a conventional variant identification software package. The choice to use a particular annotation algorithm is well within the purview of the skilled artisan, and in some embodiments is based upon the data being annotated.

For example, in some embodiments, where both a liquid biopsy sample and a normal tissue sample of the patient are analyzed, variants identified in the normal tissue sample inform annotation of the variants in the liquid biopsy sample. In some embodiments, where a particular variant is identified in the normal tissue sample, that variant is annotated as a germline variant in the liquid biopsy sample. Similarly, in some embodiments, where a particular variant identified in the liquid biopsy sample is not identified in the normal tissue sample, the variant is annotated as a somatic variant when the variant otherwise satisfies any additional criteria placed on somatic variant calling, *e.g.,* a threshold variant allele fraction (VAF) in the sample.

By contrast, in some embodiments, where only a liquid biopsy sample is being analyzed, the annotation algorithm relies on other characteristics of the variant in order to annotate the origin of the variant. For instance, in some embodiments, the annotation algorithm evaluates the VAF of the variant in the sample, *e.g.,* alone or in combination with additional characteristics of the sample, *e.g.,* tumor fraction. Accordingly, in some embodiments, where the VAF is within a first range encompassing a value that corresponds to a 1:1 distribution of variant and reference alleles in the sample, the algorithm annotates the variant as a germline variant, because it is presumably represented in cfDNA originating from both normal and cancer tissues. Similarly, in some embodiments, where the VAF is below a baseline variant threshold, the algorithm annotates the variant as undeterminable, because there is not sufficient evidence to distinguish between the possibility that the variant arose as a result of an amplification or sequencing error and the possibility that the variant originated from a cancerous tissue. Similarly, in some embodiments, where the VAF falls between the first range and the baseline variant threshold, the algorithm annotates the variant as a somatic variant derived from cell free DNA.

In some embodiments, the baseline variant threshold is a value from 0.01% VAF to 0.5% VAF. In some embodiments, the baseline variant threshold is a value from 0.05% VAF to 0.35% VAF. In some embodiments, the baseline variant threshold is a value from 0.1% VAF to 0.25% VAF. In some embodiments, the baseline variant threshold is about 0.01% VAF, 0.015% VAF, 0.02% VAF, 0.025% VAF, 0.03% VAF, 0.035% VAF, 0.04% VAF, 0.045% VAF, 0.05% VAF, 0.06% VAF, 0.07% VAF, 0.075% VAF, 0.08% VAF, 0.09% VAF, 0.1% VAF, 0.15% VAF, 0.2% VAF, 0.25% VAF, 0.3% VAF, 0.35% VAF, 0.4% VAF, 0.45% VAF, 0.5% VAF, or greater. In some embodiments, the baseline variant threshold is different for variants located in a first region, *e.g.,* a region identified as a mutational hotspot and/or having high genomic complexity, than for variants located in a second region, *e.g.,* a region that is not identified as a mutational hotspot and/or having average genomic complexity. For example, in some embodiments, the baseline variant threshold is a value from 0.01% to 0.25% for variants located in the first region and is a value from 0.1% to 0.5% for variants located in the second region.

### Homologous Recombination Status (HRD)

In some embodiments, analysis of aligned sequence reads, *e.g.,* in SAM or BAM format, includes analysis of whether the cancer is homologous recombination deficient (HRD status 137-3), using a homologous recombination pathway analysis module 157.

Homologous recombination (HR) is a normal, highly conserved DNA repair process that enables the exchange of genetic information between identical or closely related DNA molecules. It is most widely used by cells to accurately repair harmful breaks (*e.g.* damage) that occur on both strands of DNA. DNA damage may occur from exogenous (external) sources like UV light, radiation, or chemical damage; or from endogenous (internal) sources like errors in DNA replication or other cellular processes that create DNA damage. Double strand breaks are a type of DNA damage. Using poly (ADP-ribose) polymerase (PARP) inhibitors in patients with HRD compromises two pathways of DNA repair, resulting in cell death (apoptosis). The efficacy of PARP inhibitors is improved not only in ovarian cancers displaying germline or somatic BRCA mutations, but also in cancers in which HRD is caused by other underlying etiologies.

In some embodiments, HRD status can be determined by inputting features correlated with HRD status into a classifier trained to distinguish between cancers with homologous recombination pathway deficiencies and cancers without homologous recombination pathway deficiencies. For example, in some embodiments, the features include one or more of (i) a heterozygosity status for a first plurality of DNA damage repair genes in the genome of the cancerous tissue of the subject, (ii) a measure of the loss of heterozygosity across the genome of the cancerous tissue of the subject, (iii) a measure of variant alleles detected in a second plurality of DNA damage repair genes in the genome of the cancerous tissue of the subject, and (iv) a measure of variant alleles detected in the second plurality of DNA damage repair genes in the genome of the non-cancerous tissue of the subject. In some embodiments, all four of the features described above are used as features in an HRD classifier. More details about HRD classifiers using these and other features are described in U.S. Patent Application Publication No. 2020/0255909, the content of which is hereby incorporated by reference, in its entirety, for all purposes.

### Concurrent Testing

Unless stated otherwise, as used herein, the term "concurrent" as it relates to assays refers to a period of time between zero and ninety days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 90 days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 60 days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 30 days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 21 days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 14 days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 7 days. In some embodiments, concurrent tests using different biological samples from the same subject (*e.g.,* two or more of a liquid biopsy sample, cancerous tissue-such as a solid tumor sample or blood sample for a blood-based cancer-and a non-cancerous sample) are performed within a period of time (*e.g.,* the biological samples are collected within the period of time) of from 0 days to 3 days.

In some embodiments, a liquid biopsy assay may be used concurrently with a solid tumor assay to return more comprehensive information about a patient's variants. For example, a blood specimen and a solid tumor specimen may be sent to a laboratory for evaluation. The solid tumor specimen may be analyzed using a bioinformatics pipeline to produce a solid tumor result. A solid tumor assay is described, for instance, in U.S. Patent Application No. 16/657,804, the content of which is hereby incorporated by reference, in its entirety, for all purposes. The cancer type of the solid tumor may include, for example, non small cell lung cancer, colorectal cancer, or breast cancer. Alterations identified in the tumor/matched normal result may include, for example, EGFR+ for non small cell lung cancer; HER2+ for breast cancer; or KRAS G12C for several cancers.

In some embodiments, a blood specimen may be divided into a first portion and a second portion. The first portion of the blood specimen and the solid tumor specimen may be analyzed using a bioinformatics pipeline to produce a tumor/matched normal result. The second portion of the blood specimen may be analyzed using a bioinformatics pipeline to produce a liquid biopsy result. For example, the blood specimen may be analyzed using at least an improvement in somatic variant identification, *e.g.,* as described herein in the section entitled "Variant Identification." For example, the blood specimen may be analyzed using an improvement in focal copy number identification, *e.g.,* as described herein in the section entitled "Copy Number Variation." For example, the blood specimen may be analyzed using an improvement in circulating tumor fraction determination, *e.g.,* as described above in the section entitled "Systems and Methods for Improved Circulating Tumor Fraction Estimates" and/or "Systems and Methods for Improved Validation of Somatic Sequence Variants."

Therapies may be identified for further consideration in response to receiving the tumor or tumor/matched normal result along with the liquid biopsy result. For example, if the results overall indicate that the patient has HER2+ breast cancer, neratinib may be identified along with the test results for further consideration by the ordering clinician.

The solid tumor or tumor/matched normal assay may be ordered concurrently; their results may be delivered concurrently; and they may be analyzed concurrently.

*Systems and methods for detecting whether a variant in a biopsy from* a *subject is (a)* a *somatic variant derived from cell free DNA or (b) other than a somatic variant derived from cell free DNA.*

An overview of methods for providing clinical support for personalized cancer therapy is described above with reference to Figures 2-4E above. Below, systems and methods for improved detection of clonal hematopoiesis variants and/or solid tumor variants, *e.g.,* within the context of the methods and systems described above, are described with reference to Figures 5A-5G.

Many of the embodiments described below, in conjunction with Figures 5A-5G, relate to analyses performed using sequencing data for cfDNA obtained from a liquid biopsy sample of a subject, *e.g.,* a cancer patient. Generally, these embodiments are independent and, thus, not reliant upon any particular DNA sequencing methods. However, in some embodiments, the methods described below include generating the sequencing data.

As described herein, in some embodiments, the methods described herein (*e.g.,* method 500 as illustrated in Figures 5A-5G) include one or more data collection steps, in addition to data analysis and downstream steps. For example, as described herein, *e.g.,* with reference to Figures 2 and 3, in some embodiments, the methods include collection of a liquid biopsy sample and, optionally, one or more matching biological samples from the subject (*e.g.,* a matched cancerous and/or matched non-cancerous sample from the subject). Likewise, as described herein, *e.g.,* with reference to Figures 2 and 3, in some embodiments, the methods include extraction of DNA from the liquid biopsy sample (cfDNA) and, optionally, one or more matching biological samples from the subject (*e.g.,* a matched cancerous and/or matched non-cancerous sample from the subject). Similarly, as herein, *e.g.,* with reference to Figures 2 and 3, in some embodiments, the methods include nucleic acid sequencing of DNA from the liquid biopsy (cfDNA) sample and, optionally, one or more matching biological samples from the subject (*e.g.,* a matched cancerous and/or matched non-cancerous sample from the subject). Advantageously, the methods and systems described herein allow for accurate classification of variant lineage as either somatic or hematopoietic based on sequencing data from only cfDNA fragments. Accordingly, in some embodiments, a matched cancerous and/or matched non-cancerous sample from the subject is not used in the methods described herein.

However, in other embodiments, the methods described herein begin with obtaining nucleic acid sequencing results, *e.g.,* raw or collapsed sequence reads of DNA from a liquid biopsy sample (cfDNA) and, optionally, one or more matching biological samples from the subject (*e.g.,* a matched cancerous and/or matched non-cancerous sample from the subject), from which the genomic features needed for detecting clonal hematopoiesis variants and/or solid tumor variants can be determined. For example, in some embodiments, sequencing data 122 for a patient 121 is accessed and/or downloaded over network 105 by system 100.

In some embodiments, the method further comprises isolating the plurality of cell-free nucleic acids from the liquid biopsy sample of the test subject prior to the sequencing. In some embodiments, the sequencing is multiplexed sequencing. In some embodiments, the sequencing is short-read sequencing or long-read sequencing.

Similarly, in some embodiments, the methods described herein begin with obtaining the genomic features needed for filtering of clonal hematopoiesis variants from a sequencing of a liquid biopsy sample and, optionally, one or more matching biological samples from the subject (*e.g.,* a matched cancerous and/or matched non-cancerous sample from the subject). For example, in some embodiments, (i) one or more fragment length metrics, (ii) a variant allele fraction for the candidate somatic variant and a ctFE for the liquid biopsy sample or one or more features determined from the variant allele fraction for the candidate somatic variant and the ctFE for the liquid biopsy sample, and (iii) one or more metrics of clonal hematopoiesis prevalence for the first nucleotide position, is accessed and/or downloaded over network 105 by system 100.

*Block 500.* Figures 5A-5G collectively provide a flow chart of processes and features for characterizing a candidate mutation, in accordance with some embodiments of the present disclosure.

*Block 502.* Referring to block 502, a plurality of reference sequence reads mapping to a genomic location of the candidate mutation is obtained from a first sequencing reaction using a reference sample of a test subject.

In some embodiments the reference sample is a liquid biopsy sample.

In some embodiments the method includes obtaining a corresponding nucleic acid sequence of each cell-free DNA (cfDNA) fragment in a plurality of DNA fragments (*e.g.,* cfDNA fragments), from a plurality of sequence reads of a sequencing reaction of the plurality of DNA fragments from one or more liquid biological samples from a subject.

With reference to Figure 2B, nucleic acid sequencing of one or more samples collected from the subject is performed, *e.g.,* at sequencing lab 230, during wet lab processing 204. An example workflow for nucleic acid sequencing is illustrated in Figure 3. In some embodiments, the one or more biological samples obtained at the sequencing lab 230 are accessioned (302), to track the sample and data through the sequencing process.

Next, nucleic acids, *e.g.,* RNA and/or DNA are extracted (304) from the one or more biological samples. Methods for isolating nucleic acids from biological samples are known in the art, and are dependent upon the type of nucleic acid being isolated (*e.g.,* cfDNA, DNA, and/or RNA) and the type of sample from which the nucleic acids are being isolated (*e.g.*, liquid biopsy samples, white blood cell buffy coat preparations, formalin-fixed paraffin-embedded (FFPE) solid tissue samples, and fresh frozen solid tissue samples). The selection of any particular nucleic acid isolation technique for use in conjunction with the embodiments described herein is well within the skill of the person having ordinary skill in the art, who will consider the sample type, the state of the sample, the type of nucleic acid being sequenced and the sequencing technology being used.

For instance, many techniques for DNA isolation, *e.g.,* genomic DNA isolation, from a tissue sample are known in the art, such as organic extraction, silica adsorption, and anion exchange chromatography. Likewise, many techniques for RNA isolation, *e.g.,* mRNA isolation, from a tissue sample are known in the art. For example, acid guanidinium thiocyanate-phenol-chloroform extraction (see, for example, Chomczynski and Sacchi, 2006, Nat Protoc, 1(2):581-85, which is hereby incorporated by reference herein), and silica bead/glass fiber adsorption (see, for example, Poeckh et al., 2008, Anal Biochem., 373(2):253-62, which is hereby incorporated by reference herein). The selection of any particular DNA or RNA isolation technique for use in conjunction with the embodiments described herein is well within the skill of the person having ordinary skill in the art, who will consider the tissue type, the state of the tissue, *e.g.,* fresh, frozen, formalin-fixed, paraffin-embedded (FFPE), and the type of nucleic acid analysis that is to be performed.

In some embodiments where the biological sample is a liquid biopsy sample, *e.g.,* a blood or blood plasma sample, cfDNA is isolated from blood samples using commercially available reagents, including proteinase K, to generate a liquid solution of cfDNA.

In some embodiments, isolated DNA molecules are mechanically sheared to an average length using an ultrasonicator (for example, a Covaris ultrasonicator). In some embodiments, isolated nucleic acid molecules are analyzed to determine their fragment size, *e.g.,* through gel electrophoresis techniques and/or the use of a device such as a LabChip GX Touch. The skilled artisan will know of an appropriate range of fragment sizes, based on the sequencing technique being employed, as different sequencing techniques have differing fragment size requirements for robust sequencing. In some embodiments, quality control testing is performed on the extracted nucleic acids (*e.g.,* DNA and/or RNA), *e.g.,* to assess the nucleic acid concentration and/or fragment size. For example, sizing of DNA fragments provides valuable information used for downstream processing, such as determining whether DNA fragments require additional shearing prior to sequencing.

Wet lab processing 204 then includes preparing a nucleic acid library from the isolated nucleic acids (*e.g.,* cfDNA, DNA, and/or RNA). For example, in some embodiments, DNA libraries (*e.g.,* gDNA and/or cfDNA libraries) are prepared from isolated DNA from the one or more biological samples. In some embodiments, the DNA libraries are prepared using a commercial library preparation kit, *e.g.,* the KAPA Hyper Prep Kit, a New England Biolabs (NEB) kit, or a similar kit.

In some embodiments, during library preparation, adapters (*e.g.,* UDI adapters, such as Roche SeqCap dual end adapters, or UMI adapters such as full length or stubby Y adapters) are ligated onto the nucleic acid molecules. In some embodiments, the adapters include unique molecular identifiers (UMIs), which are short nucleic acid sequences (*e.g.,* 3-10 base pairs) that are added to ends of DNA fragments during adapter ligation. In some embodiments, UMIs are degenerate base pairs that serve as a unique tag that can be used to identify sequence reads originating from a specific DNA fragment. In some embodiments, *e.g.,* when multiplex sequencing will be used to sequence DNA from a plurality of samples (*e.g.,* from the same or different subjects) in a single sequencing reaction, a patient-specific index is also added to the nucleic acid molecules. In some embodiments, the patient specific index is a short nucleic acid sequence (*e.g.,* 3-20 nucleotides) that are added to ends of DNA fragments during library construction, that serve as a unique tag that can be used to identify sequence reads originating from a specific patient sample. Examples of identifier sequences are described, for example, in Kivioja et al., 2011, Nat. Methods 9(1):72-74 and Islam et al., 2014, Nat. Methods 11(2):163-66, the contents of which are hereby incorporated by reference, in their entireties, for all purposes.

In some embodiments, an adapter includes a PCR primer landing site, designed for efficient binding of a PCR or second-strand synthesis primer used during the sequencing reaction. In some embodiments, an adapter includes an anchor binding site, to facilitate binding of the DNA molecule to anchor oligonucleotide molecules on a sequencer flow cell, serving as a seed for the sequencing process by providing a starting point for the sequencing reaction. During PCR amplification following adapter ligation, the UMIs, patient indexes, and binding sites are replicated along with the attached DNA fragment. This provides a way to identify sequence reads that came from the same original fragment in downstream analysis.

In some embodiments, DNA libraries are amplified and purified using commercial reagents, (*e.g.,* Axygen MAG PCR clean up beads). In some such embodiments, the concentration and/or quantity of the DNA molecules are then quantified using a fluorescent dye and a fluorescence microplate reader, standard spectrofluorometer, or filter fluorometer. In some embodiments, library amplification is performed on a device (*e.g.,* an Illumina C-Bot2) and the resulting flow cell containing amplified target-captured DNA libraries is sequenced on a next generation sequencer (*e.g.,* an Illumina HiSeq 4000 or an Illumina NovaSeq 6000) to a unique on-target depth selected by the user. In some embodiments, DNA library preparation is performed with an automated system, using a liquid handling robot (*e.g.,* a SciClone NGSx).

In some embodiments, where feature data 125 includes methylation states 132 for one or more genomic locations, nucleic acids isolated from the biological sample (*e.g.,* cfDNA) are treated to convert unmethylated cytosines to uracils, *e.g.,* prior to generating the sequencing library. Accordingly, when the nucleic acids are sequenced, all cytosines called in the sequencing reaction were necessarily methylated, since the unmethylated cytosines were converted to uracils and accordingly would have been called as thymidines, rather than cytosines, in the sequencing reaction. Commercial kits are available for bisulfite-mediated conversion of methylated cytosines to uracils. Commercial kits are also available for enzymatic conversion of methylated cytosines to uracils.

In some embodiments, wet lab processing 204 includes pooling (308) DNA molecules from a plurality of libraries, corresponding to different samples from the same and/or different patients, to forming a sequencing pool of DNA libraries. When the pool of DNA libraries is sequenced, the resulting sequence reads correspond to nucleic acids isolated from multiple samples. The sequence reads can be separated into different sequence read files, corresponding to the various samples represented in the sequencing read based on the unique identifiers present in the added nucleic acid fragments. In this fashion, a single sequencing reaction can generate sequence reads from multiple samples. Advantageously, this allows for the processing of more samples per sequencing reaction.

In some embodiments, the plurality of sequence reads that is obtained from the above described sequencing includes at least 10,000 sequence reads, at least 50,000 sequence reads, at least 100,000 sequence reads, at least 500,000 sequence reads, at least 1 million sequence reads, at least 5 million sequence reads, at least 10 million sequence reads, or more. In some embodiments, the plurality of sequence reads includes no more than 1 billion sequence reads, no more than 500 million sequence reads, no more than 100 million sequence reads, no more than 50 million sequence reads, no more than 10 million sequence reads, no more than 5 million sequence reads, no more than 1 million sequence reads, or less. In some embodiments, the plurality of sequence reads is from 10,000 sequence reads to 1 billion sequence reads, from 10,000 sequence reads to 500 million sequence reads, from 10,000 sequence reads to 100 million sequence reads, from 10,000 sequence reads to 50 million sequence reads, from 10,000 sequence reads to 10 million sequence reads, from 10,000 sequence reads to 5 million sequence reads, or from 10,000 sequence reads to 1 million sequence reads. In some embodiments, the plurality of sequence reads is from 100,000 sequence reads to 1 billion sequence reads, from 100,000 sequence reads to 500 million sequence reads, from 100,000 sequence reads to 100 million sequence reads, from 100,000 sequence reads to 50 million sequence reads, from 100,000 sequence reads to 10 million sequence reads, from 100,000 sequence reads to 5 million sequence reads, or from 100,000 sequence reads to 1 million sequence reads. In some embodiments, the plurality of sequence reads is from 500,000 sequence reads to 1 billion sequence reads, from 500,000 sequence reads to 500 million sequence reads, from 500,000 sequence reads to 100 million sequence reads, from 500,000 sequence reads to 50 million sequence reads, from 500,000 sequence reads to 10 million sequence reads, from 500,000 sequence reads to 5 million sequence reads, or from 500,000 sequence reads to 1 million sequence reads. In some embodiments, the plurality of sequence reads is from 1 million sequence reads to 1 billion sequence reads, from 1 million sequence reads to 500 million sequence reads, from 1 million sequence reads to 100 million sequence reads, from 1 million sequence reads to 50 million sequence reads, from 1 million sequence reads to 10 million sequence reads, or from 1 million sequence reads to 5 million sequence reads.

In some embodiments, the plurality of DNA (*e.g.,* cfDNA) fragments includes at least 1000 DNA (*e.g.,* cfDNA) fragments, at least 5000 DNA (*e.g.,* cfDNA) fragments, at least 10,000 DNA (*e.g.,* cfDNA) fragments, at least 50,000 DNA (*e.g.,* cfDNA) fragments, at least 100,000 DNA *(e.g.,* cfDNA) fragments, at least 500,000 DNA (*e.g.,* cfDNA) fragments, at least 1 million DNA (*e.g.,* cfDNA) fragments, at least 5 million DNA (*e.g.,* cfDNA) fragments, or more. In some embodiments, the plurality of DNA (*e.g.,* cfDNA) fragments includes no more than no more than 100 million DNA (*e.g.,* cfDNA) fragments, no more than 50 million DNA (*e.g.,* cfDNA) fragments, no more than 10 million DNA (*e.g.,* cfDNA) fragments, no more than 5 million DNA (*e.g.,* cfDNA) fragments, no more than 1 million DNA (*e.g.,* cfDNA) fragments, no more than 500,000 DNA (*e.g.,* cfDNA) fragments, no more than 100,000 DNA (*e.g.,* cfDNA) fragments or less. In some embodiments, the plurality of DNA (*e.g.,* cfDNA) fragments is from 1000 DNA (*e.g.,* cfDNA) fragments to 500 million DNA (*e.g.,* cfDNA) fragments, from 1000 DNA (*e.g.,* cfDNA) fragments to 100 million DNA (*e.g.,* cfDNA) fragments, from 1000 DNA (*e.g.,* cfDNA) fragments to 50 million DNA (*e.g.,* cfDNA) fragments, from 1000 DNA (*e.g.,* cfDNA) fragments to 10 million DNA (*e.g.,* cfDNA) fragments, from 1000 DNA (*e.g.,* cfDNA) fragments to 5 million DNA (*e.g.,* cfDNA) fragments, from 1000 DNA (*e.g.,* cfDNA) fragments to 1 million DNA (*e.g.,* cfDNA) fragments, from 1000 DNA (*e.g.,* cfDNA) fragments to 500,000 DNA (*e.g.,* cfDNA) fragments, from 1000 DNA (*e.g.,* cfDNA) fragments to 250,000 DNA (*e.g.,* cfDNA) fragments, or from 1000 DNA (*e.g.,* cfDNA) fragments to 100,000 DNA (*e.g.,* cfDNA) fragments. In some embodiments, the plurality of DNA (*e.g.,* cfDNA) fragments is from 5000 DNA (*e.g.,* cfDNA) fragments to 500 million DNA (*e.g.,* cfDNA) fragments, from 5000 DNA (*e.g.,* cfDNA) fragments to 100 million DNA (*e.g.,* cfDNA) fragments, from 5000 DNA (*e.g.,* cfDNA) fragments to 50 million DNA *(e.g.,* cfDNA) fragments, from 5000 DNA (*e.g.,* cfDNA) fragments to 10 million DNA (*e.g.,* cfDNA) fragments, from 5000 DNA (*e.g.,* cfDNA) fragments to 5 million DNA (*e.g.,* cfDNA) fragments, from 5000 DNA (*e.g.,* cfDNA) fragments to 1 million DNA (*e.g*., cfDNA) fragments, from 5000 DNA (*e.g*., cfDNA) fragments to 500,000 DNA (*e.g.*, cfDNA) fragments, from 5000 DNA (*e.g.,* cfDNA) fragments to 250,000 DNA (*e.g*., cfDNA) fragments, or from 5000 DNA (*e.g.,* cfDNA) fragments to 100,000 DNA (*e.g*., cfDNA) fragments. In some embodiments, the plurality of DNA (*e.g.,* cfDNA) fragments is from 10,000 DNA (*e.g*.*,* cfDNA) fragments to 500 million DNA (*e.g*., cfDNA) fragments, from 10,000 DNA (*e.g.,* cfDNA) fragments to 100 million DNA (*e.g*., cfDNA) fragments, from 10,000 DNA (*e.g.*, cfDNA) fragments to 50 million DNA (*e.g.,* cfDNA) fragments, from 10,000 DNA (*e.g.,* cfDNA) fragments to 10 million DNA (*e.g.,* cfDNA) fragments, from 10,000 DNA (*e.g*., cfDNA) fragments to 5 million DNA (*e.g*., cfDNA) fragments, from 10,000 DNA (*e.g.,* cfDNA) fragments to 1 million DNA (*e.g.*, cfDNA) fragments, from 10,000 DNA (*e.g.,* cfDNA) fragments to 500,000 DNA (*e.g.*, cfDNA) fragments, from 10,000 DNA (*e.g.*, cfDNA) fragments to 250,000 DNA (*e.g.,* cfDNA) fragments, or from 10,000 DNA (*e.g.*, cfDNA) fragments to 100,000 DNA (*e.g*., cfDNA) fragments. In some embodiments, the plurality of DNA (*e.g.,* cfDNA) fragments is from 25,000 DNA (*e.g.,* cfDNA) fragments to 500 million DNA (*e.g.,* cfDNA) fragments, from 25,000 DNA (*e.g.,* cfDNA) fragments to 100 million DNA (*e.g.,* cfDNA) fragments, from 25,000 DNA (*e.g.,* cfDNA) fragments to 50 million DNA (*e.g.,* cfDNA) fragments, from 25,000 DNA (*e.g.,* cfDNA) fragments to 10 million DNA (*e.g.,* cfDNA) fragments, from 25,000 DNA (*e.g.,* cfDNA) fragments to 5 million DNA (*e.g*., cfDNA) fragments, from 25,000 DNA (*e.g*., cfDNA) fragments to 1 million DNA (*e.g.,* cfDNA) fragments, from 25,000 DNA (*e.g*., cfDNA) fragments to 500,000 DNA (*e.g.,* cfDNA) fragments, from 25,000 DNA (*e.g*., cfDNA) fragments to 250,000 DNA (*e.g*., cfDNA) fragments, or from 25,000 DNA (*e.g*., cfDNA) fragments to 100,000 DNA (*e.g*., cfDNA) fragments.

In some embodiments, the obtaining, accessioning, storing, preparing, processing and/or analyzing the biopsy sample from the test subject comprises any of the methods and/or embodiments described above in the present disclosure. In some embodiments, the sequencing reaction comprises any of the methods and/or embodiments described above in the present disclosure.

In some embodiments, all, or nearly all, of the aligned sequence reads are evaluated to identify candidate sequence variants (*e.g*., candidate somatic sequence variants and/or candidate germline sequence variants). In other embodiments, a subset of the aligned sequence reads is evaluated to identify candidate sequence variants. For example, in one embodiment, targeted-panel sequencing reaction is used to generate sequencing data 122 and only sequence reads corresponding to the target panel (on-target reads) are evaluated to identify candidate sequence variants. In some embodiments, targeted-panel sequencing reaction is used to generate sequencing data 122 and a subset of sequence reads corresponding to a subset of the target panel are evaluated to identify candidate sequence variants. In some embodiments, a subset of the sequence reads corresponding to a subset of genes, regardless of whether the sequencing reaction is a targeted-panel sequencing reaction, a whole exome sequencing reaction, or a whole genome sequencing reaction, are evaluated to identify candidate sequence variants. In some embodiments, a subset of sequence reads corresponding to a defined set of regions within the genome, e.g., one or more genes, one or more introns, one or more exons, one or more subregion of an intron and/or exon associated with cancer etiology, etc., are evaluated to identify candidate sequence variants.

Alternatively, in some embodiments, regardless of what subset of aligned sequence reads are evaluated to identify candidate sequence variants, only a subset of candidate sequence variants is further validated. For example, in some embodiments, only candidate sequence variants corresponding to the target panel (on-target reads) are validated. Similarly, in some embodiments, only candidate sequence variants corresponding to a subset of the target panel are validated. Likewise, in some embodiments, only candidate sequence variants corresponding to a subset of genes, regardless of whether the sequencing reaction is a targeted-panel sequencing reaction, a whole exome sequencing reaction, or a whole genome sequencing reaction, are validated. Similarly, in some embodiments, only candidate mutations corresponding to a defined set of regions within the genome, *e.g.,* one or more genes, one or more introns, one or more exons, one or more subregion of an intron and/or exon associated with cancer etiology, etc., are validated.

In some embodiments, *e.g.,* where a whole genome sequencing method is used, nucleic acid sequencing libraries are not target-enriched prior to sequencing, in order to obtain sequencing data on substantially all of the competent nucleic acids in the sequencing library. Similarly, in some embodiments, *e.g.,* where a whole genome sequencing method will be used, nucleic acid sequencing libraries are not mixed, because of bandwidth limitations related to obtaining significant sequencing depth across an entire genome. However, in other embodiments, *e.g.,* where a low pass whole genome sequencing (LPWGS) methodology will be used, nucleic acid sequencing libraries can still be pooled, because very low average sequencing coverage is achieved across a respective genome, *e.g.,* between about 0.5x and about 5x.

In some embodiments, the sequence reads are obtained by a whole genome or whole exome sequencing methodology. In some such embodiments, whole exome capture is performed with an automated system, using a liquid handling robot (for example, a SciClone NGSx). Whole genome sequencing, and to some extent whole exome sequencing, is typically performed at lower sequencing depth than smaller target-panel sequencing reactions, because many more loci are being sequenced. For example, in some embodiments, whole genome or whole exome sequencing is performed to an average sequencing depth of at least 3x, at least 5x, at least 10x, at least 15x, at least 20x, or greater. In some embodiments, low-pass whole genome sequencing (LPWGS) techniques are used for whole genome or whole exome sequencing. LPWGS is typically performed to an average sequencing depth of about 0.25x to about 5x, more typically to an average sequencing depth of about 0.5x to about 3x.

Because of the differences in the sequencing methodologies, data obtained from targeted-panel sequencing is better suited for certain analyses than data obtained from whole genome/whole exome sequencing, and vice versa. For instance, because of the higher sequencing depth achieved by targeted-panel sequencing, the resulting sequence data is better suited for the identification of variant alleles present at low allelic fractions in the sample, *e.g.,* less than 20%. By contrast, data generated from whole genome/whole exome sequencing is better suited for the estimation of genome-wide metrics, such as tumor mutational burden, because the entire genome is better represented in the sequencing data. Accordingly, in some embodiments, a nucleic acid sample, *e.g.,* a cfDNA, gDNA, or mRNA sample, is evaluated using both targeted-panel sequencing and whole genome/whole exome sequencing (*e.g.,* LPWGS).

In some embodiments, the raw sequence reads resulting from the sequencing reaction are output from the sequencer in a native file format, *e.g.,* a BCL file. In some embodiments, the native file is passed directly to a bioinformatics pipeline (*e.g.,* variant analysis 206), components of which are described in detail below. In other embodiments, pre-processing is performed prior to passing the sequences to the bioinformatics platform. For instance, in some embodiments, the format of the sequence read file is converted from the native file format (*e.g.,* BCL) to a file format compatible with one or more algorithms used in the bioinformatics pipeline (*e.g.,* FASTQ or FASTA). In some embodiments, the raw sequence reads are filtered to remove sequences that do not meet one or more quality thresholds. In some embodiments, raw sequence reads generated from the same unique nucleic acid molecule in the sequencing read are collapsed into a single sequence read representing the molecule, *e.g.,* using UMIs as described above. In some embodiments, one or more of these pre-processing activities is performed within the bioinformatics pipeline itself.

In one example, a sequencer may generate a BCL file. A BCL file may include raw image data of a plurality of patient specimens which are sequenced. BCL image data is an image of the flow cell across each cycle during sequencing. A cycle may be implemented by illuminating a patient specimen with a specific wavelength of electromagnetic radiation, generating a plurality of images which may be processed into base calls via BCL to FASTQ processing algorithms which identify which base pairs are present at each cycle. The resulting FASTQ file includes the entirety of reads for each patient specimen paired with a quality metric, *e.g.,* in a range from 0 to 64 where a 64 is the best quality and a 0 is the worst quality. In embodiments where both a liquid biopsy sample and a normal tissue sample are sequenced, sequence reads in the corresponding FASTQ files may be matched, such that a liquid biopsy-normal analysis may be performed.

FASTQ format is a text-based format for storing both a biological sequence, such as a nucleotide sequence, and its corresponding quality scores. These FASTQ files are analyzed to determine what genetic variants or copy number changes are present in the sample. Each FASTQ file contains reads that may be paired-end or single reads, and may be short-reads or long-reads, where each read represents one detected sequence of nucleotides in a nucleic acid molecule that was isolated from the patient sample or a copy of the nucleic acid molecule, detected by the sequencer. Each read in the FASTQ file is also associated with a quality rating. The quality rating may reflect the likelihood that an error occurred during the sequencing procedure that affected the associated read. In some embodiments, the results of paired-end sequencing of each isolated nucleic acid sample are contained in a split pair of FASTQ files, for efficiency. Thus, in some embodiments, forward (Read 1) and reverse (Read 2) sequences of each isolated nucleic acid sample are stored separately but in the same order and under the same identifier.

In various embodiments, the bioinformatics pipeline may filter FASTQ data from the corresponding sequence data file for each respective biological sample. Such filtering may include correcting or masking sequencer errors and removing (trimming) low quality sequences or bases, adapter sequences, contaminations, chimeric reads, overrepresented sequences, biases caused by library preparation, amplification, or capture, and other errors.

While workflow 200 illustrates obtaining a biological sample, extracting nucleic acids from the biological sample, and sequencing the isolated nucleic acids, in some embodiments, sequencing data used in the improved systems and methods described herein (*e.g.,* which include improved methods for determining accurate circulating tumor fraction estimates) is obtained by receiving previously generated sequence reads, in electronic form.

In some embodiments, sequencing of the plurality of cell-free nucleic acids in the liquid biopsy sample of the subject is performed at a central laboratory or sequencing facility. In some such embodiments, the method comprises accessing one or more sequencing datasets and/or one or more auxiliary files, in electronic form, through a cloud-based interface. For example, a dataset can be obtained by performing a bioinformatics pipeline using tumor BAM files, normal BAM files, a human reference genome file, a target region BED file, a list of mappable regions of the genome, and/or a blacklist of recurrent problematic areas of the genome.

In some embodiments, the obtaining the dataset comprises accessing the dataset, in electronic form, through a cloud-based interface. For example, a dataset can comprise one or more outputs from a bioinformatics pipeline (*e.g.,* CNVkit outputs ".cns" and/or ".cnr").

In some embodiments, the sequencing data is processed (*e.g.,* using sequence data processing module 141) to prepare it for genomic feature identification 385. For instance, in some embodiments as described above, the sequencing data is present in a native file format provided by the sequencer. Accordingly, in some embodiments, the system (*e.g.,* system 100) applies a pre-processing algorithm 142 to convert the file format (318) to one that is recognized by one or more upstream processing algorithms. For example, BCL file outputs from a sequencer can be converted to a FASTQ file format using the bcl2fastq or bcl2fastq2 conversion software (Illumina^{®}). FASTQ format is a text-based format for storing both a biological sequence, such as nucleotide sequence, and its corresponding quality scores. These FASTQ files are analyzed to determine what genetic variants, copy number changes, *etc.,* are present in the sample.

In some embodiments, other preprocessing functions are performed, *e.g.,* filtering sequence reads 122 based on a desired quality, *e.g.,* size and/or quality of the base calling. In some embodiments, quality control checks are performed to ensure the data is sufficient for variant calling. For instance, entire reads, individual nucleotides, or multiple nucleotides that are likely to have errors may be discarded based on the quality rating associated with the read in the FASTQ file, the known error rate of the sequencer, and/or a comparison between each nucleotide in the read and one or more nucleotides in other reads that has been aligned to the same location in the reference genome. Filtering may be done in part or in its entirety by various software tools, for example, a software tool such as Skewer. See, Jiang, H. et al., BMC Bioinformatics 15(182):1-12 (2014). FASTQ files may be analyzed for rapid assessment of quality control and reads, for example, by a sequencing data QC software such as AfterQC, Kraken, RNA-SeQC, FastQC, or another similar software program. For paired end reads, reads may be merged.

In some embodiments, when both a liquid biopsy sample and a normal tissue sample from the patient are sequenced, two FASTQ output files are generated, one for the liquid biopsy sample and one for the normal tissue sample. A 'matched' (*e.g.,* panel-specific) workflow is run to jointly analyze the liquid biopsy-normal matched FASTQ files. When a matched normal sample is not available from the patient, FASTQ files from the liquid biopsy sample are analyzed in the 'tumor-only' mode. See, for example, Figure 4B. If two or more patient samples are processed simultaneously on the same sequencer flow cell, *e.g.,* a liquid biopsy sample and a normal tissue sample, a difference in the sequence of the adapters used for each patient sample barcodes nucleic acids extracted from both samples, to associate each read with the correct patient sample and facilitate assignment to the correct FASTQ file.

For efficiency, in some embodiments, the results of paired-end sequencing of each isolate are contained in a split pair of FASTQ files. Forward (Read 1) and reverse (Read 2) sequences of each tumor and normal isolate are stored separately but in the same order and under the same identifier. See, for example, Figure 4C. In various embodiments, the bioinformatics pipeline may filter FASTQ data from each isolate. Such filtering may include correcting or masking sequencer errors and removing (trimming) low quality sequences or bases, adapter sequences, contaminations, chimeric reads, overrepresented sequences, biases caused by library preparation, amplification, or capture, and other errors. See, for example, Figure 4D.

Similarly, in some embodiments, sequencing (312) is performed on a pool of nucleic acid sequencing libraries prepared from different biological samples, *e.g.,* from the same or different patients. Accordingly, in some embodiments, the system demultiplexes (320) the data (*e.g.,* using demultiplexing algorithm 144) to separate sequence reads into separate files for each sequencing library included in the sequencing pool, *e.g.,* based on UMI or patient identifier sequences added to the nucleic acid fragments during sequencing library preparation, as described above. In some embodiments, the demultiplexing algorithm is part of the same software package as one or more pre-processing algorithms 142. For instance, the bcl2fastq or bcl2fastq2 conversion software (Illumina^{®}) include instructions for both converting the native file format output from the sequencer and demultiplexing sequence reads 122 output from the reaction.

The sequence reads are then aligned (322), *e.g.,* using an alignment algorithm 143, to a reference sequence construct 158, *e.g.,* a reference genome, reference exome, or other reference construct prepared for a particular targeted-panel sequencing reaction. For example, in some embodiments, individual sequence reads 123, in electronic form (*e.g.,* in FASTQ files), are aligned against a reference sequence construct for the species of the subject (*e.g.,* a reference human genome) by identifying a sequence in a region of the reference sequence construct that best matches the sequence of nucleotides in the sequence read. In some embodiments, the sequence reads are aligned to a reference exome or reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene. Any of a variety of alignment tools can be used for this task.

For instance, local sequence alignment algorithms compare subsequences of different lengths in the query sequence (*e.g.,* sequence read) to subsequences in the subject sequence (*e.g.,* reference construct) to create the best alignment for each portion of the query sequence. In contrast, global sequence alignment algorithms align the entirety of the sequences, *e.g.,* end to end. Examples of local sequence alignment algorithms include the Smith-Waterman algorithm (see, for example, Smith and Waterman, J Mol. Biol., 147(1):195-97 (1981), which is incorporated herein by reference), Lalign (see, for example, Huang and Miller, Adv. Appl. Math, 12:337-57 (1991), which is incorporated by reference herein), and PatternHunter (see, for example, Ma et al., Bioinformatics, 18(3):440-45 (2002), which is incorporated by reference herein).

In some embodiments, the read mapping process starts by building an index of either the reference genome or the reads, which is then used to retrieve the set of positions in the reference sequence where the reads are more likely to align. Once this subset of possible mapping locations has been identified, alignment is performed in these candidate regions with slower and more sensitive algorithms. See, for example, Hatem et al., 2013, "Benchmarking short sequence mapping tools," BMC Bioinformatics 14: p. 184; and Flicek and Birney, 2009, "Sense from sequence reads: methods for alignment and assembly," Nat Methods 6(Suppl. 11), S6-S12, each of which is hereby incorporated by reference. In some embodiments, the mapping tools methodology makes use of a hash table or a Burrows-Wheeler transform (BWT). See, for example, Li and Homer, 2010, "A survey of sequence alignment algorithms for next-generation sequencing," Brief Bioinformatics 11, pp. 473-483, which is hereby incorporated by reference.

Other software programs designed to align reads include, for example, Novoalign (Novocraft, Inc.), Bowtie, Burrows Wheeler Aligner (BWA), and/or programs that use a Smith-Waterman algorithm. Candidate reference genomes include, for example, hg19, GRCh38, hg38, GRCh37, and/or other reference genomes developed by the Genome Reference Consortium. In some embodiments, the alignment generates a SAM file, which stores the locations of the start and end of each read according to coordinates in the reference genome and the coverage (number of reads) for each nucleotide in the reference genome.

For example, in some embodiments, each read of a FASTQ file is aligned to a location in the human genome having a sequence that best matches the sequence of nucleotides in the read. There are many software programs designed to align reads, for example, Novoalign (Novocraft, Inc.), Bowtie, Burrows Wheeler Aligner (BWA), programs that use a Smith-Waterman algorithm, *etc.* Alignment may be directed using a reference genome (for example, hg19, GRCh38, hg38, GRCh37, other reference genomes developed by the Genome Reference Consortium, *etc*.) by comparing the nucleotide sequences in each read with portions of the nucleotide sequence in the reference genome to determine the portion of the reference genome sequence that is most likely to correspond to the sequence in the read. In some embodiments, one or more SAM files are generated for the alignment, which store the locations of the start and end of each read according to coordinates in the reference genome and the coverage (number of reads) for each nucleotide in the reference genome. The SAM files may be converted to BAM files. In some embodiments, the BAM files are sorted, and duplicate reads are marked for deletion, resulting in de-duplicated BAM files.

In some embodiments, adapter-trimmed FASTQ files are aligned to the 19th edition of the human reference genome build (HG19). Following alignment, reads are grouped by alignment position and UMI family and collapsed into consensus sequences. Bases with insufficient quality or significant disagreement among family members (for example, when it is uncertain whether the base is an adenine, cytosine, guanine, *etc*.) may be replaced by N's to represent a wildcard nucleotide type. PHRED scores are then scaled based on initial base calling estimates combined across all family members. Following single-strand consensus generation, duplex consensus sequences are generated by comparing the forward and reverse oriented PCR products with mirrored UMI sequences. In various embodiments, a consensus can be generated across read pairs. Otherwise, single-strand consensus calls will be used. Following consensus calling, filtering is performed to remove low-quality consensus fragments. The consensus fragments are then re-aligned to the human reference genome using BWA. A BAM output file is generated after the re-alignment, then sorted by alignment position, and indexed.

In some embodiments, where both a liquid biopsy sample and a normal tissue sample are analyzed, this process produces a liquid biopsy BAM file (*e.g.,* Liquid BAM 124-1-i-cf) and a normal BAM file (*e.g.,* Germline BAM 124-1-i-g), as illustrated in Figure 4A. In various embodiments, BAM files may be analyzed to detect genetic variants and other genetic features, including single nucleotide variants (SNVs), copy number variants (CNVs), gene rearrangements, *etc.*

In some embodiments, the sequencing data is normalized, *e.g.,* to account for pull-down, amplification, and/or sequencing bias (*e.g.,* mappability, GC bias *etc*.).

In some embodiments, SAM files generated after alignment are converted to BAM files 124. Thus, after preprocessing sequencing data generated for a pooled sequencing reaction, BAM files are generated for each of the sequencing libraries present in the master sequencing pools. For example, as illustrated in Figure 4A, separate BAM files are generated for each of three samples acquired from subject 1 at time i (*e.g.,* tumor BAM 124-1-i-t corresponding to alignments of sequence reads of nucleic acids isolated from a solid tumor sample from subject 1, Liquid BAM 124-1-i-cf corresponding to alignments of sequence reads of nucleic acids isolated from a liquid biopsy sample from subject 1, and Germline BAM 124-1-i-g corresponding to alignments of sequence reads of nucleic acids isolated from a normal tissue sample from subject 1), and one or more samples acquired from one or more additional subjects at time j (*e.g.,* Tumor BAM 124-2-j-t corresponding to alignments of sequence reads of nucleic acids isolated from a solid tumor sample from subject 2). In some embodiments, BAM files are sorted, and duplicate reads are marked for deletion, resulting in de-duplicated BAM files. For example, tools like SamBAMBA mark and filter duplicate alignments in the sorted BAM files.

Many of the embodiments described below, in conjunction with Figure 4, relate to analyses performed using sequencing data from cfDNA of a cancer patient, *e.g.,* obtained from a liquid biopsy sample of the patient. Generally, these embodiments are independent and, thus, not reliant upon any particular sequencing data generation methods, *e.g.,* sample preparation, sequencing, and/or data pre-processing methodologies. However, in some embodiments, the methods described below include one or more features 204 of generating sequencing data, as illustrated in Figures 2A and 3.

Alignment files prepared as described above (*e.g.,* BAM files 124) are then passed to a feature extraction module 145, where the sequences are analyzed (324) to identify genomic alterations (*e.g.,* SNVs/MNVs, indels, genomic rearrangements, copy number variations, *etc.*) and/or determine various characteristics of the patient's cancer (*e.g.,* MSI status, TMB, tumor ploidy, HRD status, tumor fraction, tumor purity, methylation patterns, *etc*.). Many software packages for identifying genomic alterations are known in the art. For a review of many of these variant calling packages see, for example, Cameron et al., Nat. Commun., 10(3240):1-11 (2019), the content of which is hereby incorporated by reference, in its entirety, for all purposes. Generally, these software packages identify variants in sorted SAM or BAM files 124, relative to one or more reference sequence constructs 158. The software packages then output a file *e.g.,* a raw VCF (variant call format), listing the variants (*e.g.,* genomic features 131) called and identifying their location relevant to the reference sequence construct (*e.g.,* where the sequence of the sample nucleic acids differ from the corresponding sequence in the reference construct). In some embodiments, system 100 digests the contents of the native output file to populate feature data 125 in test patient data store 120. In other embodiments, the native output file serves as the record of these genomic features 131 in test patient data store 120.

Generally, the systems described herein can employ any combination of available variant calling software packages and internally developed variant identification algorithms. In some embodiments, the output of a particular algorithm of a variant calling software is further evaluated, *e.g.,* to improve variant identification. Accordingly, in some embodiments, system 100 employs an available variant calling software package to perform some of all of the functionality of one or more of the algorithms shown in feature extraction module 145.

In some embodiments, as illustrated in Figure 1A, separate algorithms (or the same algorithm implemented using different parameters) are applied to identify variants unique to the cancer genome of the patient and variants existing in the germline of the subject. In other embodiments, variants are identified indiscriminately and later classified as either germline or somatic, *e.g.,* based on sequencing data, population data, or a combination thereof. In some embodiments, variants are classified as germline variants, and/or non-actionable variants, when they are represented in the population above a threshold level, *e.g.,* as determined using a population database such as ExAC or gnomAD. For instance, in some embodiments, variants that are represented in at least 1% of the alleles in a population are annotated as germline and/or non-actionable. In other embodiments, variants that are represented in at least 2%, at least 3%, at least 4%, at least 5%, at least 7.5%, at least 10%, or more of the alleles in a population are annotated as germline and/or non-actionable. In some embodiments, sequencing data from a matched sample from the patient, *e.g.,* a normal tissue sample, is used to annotate variants identified in a cancerous sample from the subject. That is, variants that are present in both the cancerous sample and the normal sample represent those variants that were in the germline prior to the patient developing cancer and can be annotated as germline variants.

In various aspects, the detected genetic variants and genetic features are analyzed as a form of quality control. For example, a pattern of detected genetic variants or features may indicate an issue related to the sample, sequencing procedure, and/or bioinformatics pipeline (*e.g.,* example, contamination of the sample, mislabeling of the sample, a change in reagents, a change in the sequencing procedure and/or bioinformatics pipeline, *etc*.).

In some embodiments, one or more additional criteria are required to be satisfied before a variant can be annotated as a somatic variant derived from cell free DNA. For instance, in some embodiments, a threshold number of unique sequence reads encompassing the variant must be present to annotate the variant as a somatic variant derived from cell free DNA. In some embodiments, the threshold number of unique sequence reads is 2, 3, 4, 5, 7, 10, 12, 15, or greater. In some embodiments, the threshold number of unique sequence reads is only applied when certain conditions are met, *e.g.,* when the variant allele is located in a region of a certain genomic complexity. In some embodiments, the certain genomic complexity is a low genomic complexity. In some embodiments, the certain genomic complexity is an average genomic complexity. In some embodiments, the certain genomic complexity is a high genomic complexity.

In some embodiments, a threshold sequencing coverage, *e.g.,* a locus-specific and/or an average sequencing depth (*e.g.,* across a targeted panel and/or complete reference sequence construct) must be satisfied to annotate the variant as a somatic variant derived from cell free DNA. In some embodiments, the threshold sequencing coverage is 50X, 100X, 150X, 200X, 250X, 300X, 350X, 400X or greater. In some embodiments, the variant is located in a microsatellite instable (MSI) region. In some embodiments, the variant is not located in a microsatellite instable (MSI) region. In some embodiments, the variant has sufficient signal-to-noise ratio.

In some embodiments, bases contributing to the variant satisfy a threshold mapping quality to annotate the variant as a somatic variant derived from cell free DNA. In some embodiments, alignments contributing to the variant must satisfy a threshold alignment quality to annotate the variant as a somatic variant derived from cell free DNA. In some embodiments, a threshold value is determined for a variant detected in a somatic (cancer) sample by analyzing the threshold metric (for example, the baseline variant threshold is determined by analyzing VAF, or the threshold sequencing coverage is determined by analyzing coverage) associated with that variant in a group of germline (normal) samples that were each processed by the same sample processing and sequencing protocol as the somatic sample (process-matched). This may be used to ensure the variants are not caused by observed artifact generating processes.

In some embodiments, the threshold value is set above the median base fraction of the threshold metric value associated with the variant in more than a specified percentage of process-matched germline samples, *e.g.,* 1, 2, 3, 4, 5, 6, 7, 8, 9, 10, or more standard deviations above the median base fraction of the threshold metric value associated with 25%, 30, 40, 50, 60, 70, 75, or more of the processed-matched germline samples. For example, in one embodiment, the threshold value is set to a value 5 standard deviations above the median base fraction of the threshold metric value associated with the variant in more than 50% of the process matched germline samples.

In some embodiments, variants around homopolymer and multimer regions known to generate artifacts may be specifically filtered to avoid such artifacts. For example, in some embodiments, strand specific filtering is performed in the direction of the read in order to minimize stranded artifacts. Similarly, in some embodiments, variants that do not exceed the stranded minimum deviation for their specific locus within a known artifact-generating region may be filtered to avoid artifacts.

Variants may be filtered using dynamic methods, such as through the application of Bayes' Theorem through a likelihood ratio test. The dynamic threshold may be based on, for example, factors such as sample specific error rate, the error rate from a healthy reference pool, and information from internal human solid tumors. Accordingly, in some embodiments, the dynamic filtering method employs a tri-nucleotide context-based Bayesian model. That is, in some embodiments, the threshold for filtering any particular putative variant is dynamically calibrated using a context-based Bayesian model that considers one or more of a sample-specific sequencing error rate, a process-matched control sequencing error rate, and/or a variant-specific frequency (*e.g.,* determined from similar cancers). In this fashion, a minimum number of alternative alleles required to positively identify a true variant is determined for individual alleles and/or loci. An example of methods and systems for applying a variable threshold that consider one or more of these factors is described in U.S. Patent No. 11,475,981, the disclosure of which is disclosed herein by reference in its entirety for all purposes.

In some embodiments, certain variants pre-identified on a list may be rescued, *e.g.,* not filtered out, when they fail to pass selective filters, *e.g.,* MSI/SN, a Bayesian filtering method, and/or a coverage, VAF or region-based filter. The rationale for affirmatively listing a variant is to apply less stringent filtering criteria to such a variant so that it can be reviewed and/or reported. In some embodiments, one or more variant on the list is a common pathogenic variant, *e.g.,* with high clinical relevance. In this fashion, when a variant on the list fails to pass certain filters, it will be rescued and not filtered out. As used herein, MSI/SN refers to a variant filter for filtering out potential artifactual variants based on the MSI (microsatellite instable) and SN (signal-to-noise ratio) values calculated by the variant caller VarDict. See, for example, VarDict documentation, available on the internet at github.com/AstraZeneca-NGS/VarDictJava.

In some embodiments, one or more locus and/or genomic region is blacklisted, preventing somatic variant annotation for variants identified at the locus or region. In some embodiments, the variant has a length of 120, 100, 80, 60, 40, 20, 10, 5 or less base pairs. In various embodiments, any combination of the additional criteria, as well as additional criteria not listed above, may be applied to the variant calling process. Again, in some embodiments, different criteria are applied to the annotation of different types of variants.

In some embodiments, liquid biopsy assays are used to detect variant alterations present at low circulating fractions in the patient's blood. In such circumstances, it may be warranted to lower the requirements for positively identifying a variant as a somatic variant derived from cell free DNA. That is, in some embodiments, low levels of support may be sufficient to call a variant as a somatic variant derived from cell free DNA, dependent upon the reason for using the liquid biopsy assay.

In some embodiments, SNV/INDEL detection is accomplished using VarDict (available on the internet at github.com/AstraZeneca-NGS/VarDictJava). Both SNVs and INDELs are called and then sorted, deduplicated, normalized and annotated. The annotation uses SnpEff to add transcript information, 1000 genomes minor allele frequencies, COSMIC reference names and counts, ExAC allele frequencies, and Kaviar population allele frequencies. The annotated variants are then classified as germline, somatic, or uncertain using a Bayesian model based on prior expectations informed by databases of germline and cancer variants. In some embodiments, uncertain variants are treated as somatic for filtering and reporting purposes.

In some embodiments, genomic rearrangements (*e.g.,* inversions, translocations, and gene fusions) are detected following de-multiplexing by aligning tumor FASTQ files against a human reference genome using a local alignment algorithm, such as BWA. In some embodiments, DNA reads are sorted, and duplicates may be marked with a software, for example, SAMBlaster. Discordant and split reads may be further identified and separated. These data may be read into a software, for example, LUMPY, for structural variant detection. In some embodiments, structural alterations are grouped by type, recurrence, and presence and stored within a database and displayed through a fusion viewer software tool. The fusion viewer software tool may reference a database, for example, Ensembl, to determine the gene and proximal exons surrounding the breakpoint for any possible transcript generated across the breakpoint. The fusion viewer tool may then place the breakpoint 5' or 3' to the subsequent exon in the direction of transcription. For inversions, this orientation may be reversed for the inverted gene. After positioning of the breakpoint, the translated amino acid sequences may be generated for both genes in the chimeric protein, and a plot may be generated containing the remaining functional domains for each protein, as returned from a database, for example, Uniprot.

For instance, in an example implementation, gene rearrangements are detected using the SpeedSeq analysis pipeline. Chiang et al., 2015, "SpeedSeq: ultra-fast personal genome analysis and interpretation," Nat Methods, (12), pg. 966. Briefly, FASTQ files are aligned to hg19 using BWA. Split reads mapped to multiple positions and read pairs mapped to discordant positions are identified and separated, then utilized to detect gene rearrangements by LUMPY. Layer et al., 2014, "LUMPY: a probabilistic framework for structural variant discovery," Genome Biol, (15), pg. 84. Fusions can then be filtered according to the number of supporting reads.

In some embodiments, putative fusion variants supported by fewer than a minimum number of unique sequence reads are filtered. In some embodiments, the minimum number of unique sequence reads is 2, 3, 4, 5, 6, 7, 8, 9, 10, 12, 15, or 20 unique sequence reads.

Figure 4E illustrates an example workflow for genomic feature identification (324). This particular workflow is only an example of one possible collection and arrangement of algorithms for feature extraction from sequencing data 124. Generally, any combination of the modules and algorithms of feature extraction module 145, *e.g.,* illustrated in Figure 1A, can be used for a bioinformatics pipeline, and particularly for a bioinformatics pipeline for analyzing liquid biopsy samples. For instance, in some embodiments, an architecture useful for the methods and systems described herein includes at least one of the modules or variant calling algorithms shown in feature extraction module 145. In some embodiments, an architecture includes at least 2, 3, 4, 5, 6, 7, 8, 9, 10, or more of the modules or variant calling algorithms shown in feature extraction module 145. Further, in some embodiments, feature extraction modules and/or algorithms not illustrated in Figure 1A find use in the methods and systems described herein.

*Block 504.* Referring to block 504, in some embodiments, the candidate mutation is a single nucleotide variant (SNV), an insertion and/or deletion variant (indel), a copy number variant or a translocation. In some embodiments, the candidate mutation is an indel that has a size of no more than three nucleotides. In some embodiments the indel has a size of no more than 4, 5, 6, 7, 8, 9, or 10 nucleotides. In some embodiments the indel has a size of no more than 10, 20, 30, 40, 50, 60, 70, 80, 90, or 100 nucleotides. In some embodiments there is no size limit on the indel.

*Blocks 506 - 510.* Referring to block 506, in some embodiments, the reference sample is saliva. Referring to block 508, in some embodiments, the reference sample is blood, whole blood, peripheral blood, plasma, serum, or lymph. Referring to block 510, in some embodiments, the reference sample consists of or comprises blood, whole blood, peripheral blood, plasma, serum, lymph, or saliva. In some alternative embodiments, the biopsy sample is any of the embodiments described above (*see, e.g.,* Definitions: Liquid Biopsy).

In some embodiments, one or more of the biological samples obtained from the patient are a biological liquid sample, also referred to as a liquid biopsy sample. In some embodiments, one or more of the biological samples obtained from the patient are selected from blood, plasma, serum, urine, vaginal fluid, fluid from a hydrocele (*e.g.,* of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (*e.g.,* thyroid, breast), *etc.* In some embodiments, the liquid biopsy sample includes blood and/or saliva. In some embodiments, the liquid biopsy sample is peripheral blood. In some embodiments, blood samples are collected from patients in commercial blood collection containers, *e.g.,* using a PAXGENE^{®} Blood DNA Tubes. In some embodiments, saliva samples are collected from patients in commercial saliva collection containers, *e.g.,* using an ORAGENE^{®} DNA Saliva Kit.

In some embodiments, the liquid biopsy sample has a volume of from about 1 mL to about 50 mL For example, in some embodiments, the liquid biopsy sample has a volume of about 1 mL, about 2 mL, about 3 mL, about 4 mL, about 5 mL, about 6 mL, about 7 mL, about 8 mL, about 9 mL, about 10 mL, about 11 mL, about 12 mL, about 13 mL, about 14 mL, about 15 mL, about 16 mL, about 17 mL, about 18 mL, about 19 mL, about 20 mL, or greater.

Liquid biopsy samples include cell free nucleic acids, including cell-free DNA (cfDNA). As described above, cfDNA isolated from cancer patients includes DNA originating from cancerous cells, also referred to as circulating tumor DNA (ctDNA), cfDNA originating from germline (*e.g*., healthy or non-cancerous) cells, and cfDNA originating from hematopoietic cells (*e.g.,* white blood cells). The relative proportions of cancerous and non-cancerous cfDNA present in a liquid biopsy sample varies depending on the characteristics (*e.g.,* the type, stage, lineage, genomic profile, *etc*.) of the patient's cancer. As used herein, the 'tumor burden' of the subject refers to the percentage cfDNA that originated from cancerous cells.

As described herein, cfDNA is a source of biological data for various implementations of the methods and systems described herein, because it is readily obtained from various body fluids. Further, because bodily fluids, such as blood, circulate throughout the body, the cfDNA population represents a sampling of many different tissue types from many different locations.

In some embodiments, a liquid biopsy sample is separated into two different samples. For example, in some embodiments, a blood sample is separated into a blood plasma sample, containing cfDNA, and a buffy coat preparation, containing white blood cells.

In some embodiments, a plurality of liquid biopsy samples is obtained from a respective subject at intervals over a period of time (*e.g.,* using serial testing). For example, in some such embodiments, the time between obtaining liquid biopsy samples from a respective subject is at least 1 day, at least 2 days, at least 1 week, at least 2 weeks, at least 1 month, at least 2 months, at least 3 months, at least 4 months, at least 6 months, or at least 1 year.

In some alternative embodiments, one or more biological samples collected from the patient are solid tissue samples, *e.g.,* a solid tumor sample or a solid normal tissue sample. Methods for obtaining solid tissue samples, *e.g.,* of cancerous and/or normal tissue are known in the art and are dependent upon the type of tissue being sampled. For example, bone marrow biopsies and isolation of circulating tumor cells can be used to obtain samples of blood cancers, endoscopic biopsies can be used to obtain samples of cancers of the digestive tract, bladder, and lungs, needle biopsies (*e.g.,* fine-needle aspiration, core needle aspiration, vacuum-assisted biopsy, and image-guided biopsy, can be used to obtain samples of subdermal tumors, skin biopsies, *e.g.,* shave biopsy, punch biopsy, incisional biopsy, and excisional biopsy, can be used to obtain samples of dermal cancers, and surgical biopsies can be used to obtain samples of cancers affecting internal organs of a patient. In some embodiments, a solid tissue sample is a formalin-fixed tissue (FFT). In some embodiments, a solid tissue sample is a macro-dissected formalin fixed paraffin embedded (FFPE) tissue. In some embodiments, a solid tissue sample is a fresh frozen tissue sample.

In some embodiments, a dedicated normal sample is collected from the patient, for co-processing with a liquid biopsy sample. Generally, the normal sample is of a non-cancerous tissue, and can be collected using any tissue collection means described above. In some embodiments, buccal cells collected from the inside of a patient's cheeks are used as a normal sample. Buccal cells can be collected by placing an absorbent material, *e.g.,* a swab, in the subject's mouth and rubbing it against their cheek, *e.g.,* for at least 15 second or for at least 30 seconds. The swab is then removed from the patient's mouth and inserted into a tube, such that the tip of the tube is submerged into a liquid that serves to extract the buccal cells off of the absorbent material. An example of buccal cell recovery and collection devices is provided in U.S. Patent No. 9,138,205, the content of which is hereby incorporated by reference, in its entirety, for all purposes. In some embodiments, the buccal swab DNA is used as a source of normal DNA in circulating heme malignancies.

Referring to Figure 2, in some embodiments the biological samples collected from the patient are, optionally, sent to various analytical environments (*e.g.,* sequencing lab 230, pathology lab 240, and/or molecular biology lab 250) for processing (*e.g*., data collection) and/or analysis (*e.g.,* feature extraction). Wet lab processing 204 may include cataloguing samples (*e.g.,* accessioning), examining clinical features of one or more samples (*e.g.,* pathology review), and nucleic acid sequence analysis (*e.g.,* extraction, library prep, capture + hybridize, pooling, and sequencing). In some embodiments, the workflow includes clinical analysis of one or more biological samples collected from the subject, *e.g.,* at a pathology lab 240 and/or a molecular and cellular biology lab 250, to generate clinical features such as pathology features 128-3, imaging data 128-3, and/or tissue culture / organoid data 128-3.

In some embodiments, the pathology data 128-1 collected during clinical evaluation includes visual features identified by a pathologist's inspection of a specimen (*e.g.,* a solid tumor biopsy), *e.g.,* of stained H&E or IHC slides. In some embodiments, the sample is a solid tissue biopsy sample. In some embodiments, the tissue biopsy sample is a formalin-fixed tissue (FFT), *e.g.,* a formalin-fixed paraffin-embedded (FFPE) tissue. In some embodiments, the tissue biopsy sample is an FFPE or FFT block. In some embodiments, the tissue biopsy sample is a fresh-frozen tissue biopsy. The tissue biopsy sample can be prepared in thin sections (*e.g.,* by cutting and/or affixing to a slide), to facilitate pathology review (*e.g*., by staining with immunohistochemistry stain for IHC review and/or with hematoxylin and eosin stain for H&E pathology review). For instance, analysis of slides for H&E staining or IHC staining may reveal features such as tumor infiltration, programmed death-ligand 1 (PD-L1) status, human leukocyte antigen (HLA) status, or other immunological features.

In some embodiments, a liquid sample (*e.g.,* blood) collected from the patient (*e.g.,* in EDTA-containing collection tubes) is prepared on a slide (*e.g*., by smearing) for pathology review. In some embodiments, macrodissected FFPE tissue sections, which may be mounted on a histopathology slide, from solid tissue samples (*e.g.,* tumor or normal tissue) are analyzed by pathologists. In some embodiments, tumor samples are evaluated to determine, *e.g.,* the tumor purity of the sample, the percent tumor cellularity as a ratio of tumor to normal nuclei, etc. For each section, background tissue may be excluded or removed such that the section meets a tumor purity threshold, *e.g.,* where at least 20% of the nuclei in the section are tumor nuclei, or where at least 25%, 30%, 40%, 50%, 60%, 70%, 80%, 90%, or more of the nuclei in the section are tumor nuclei.

In some embodiments, pathology data 128-1 is extracted, in addition to or instead of visual inspection, using computational approaches to digital pathology, *e.g.,* providing morphometric features extracted from digital images of stained tissue samples. In some embodiments, pathology data 128-1 includes features determined using machine learning algorithms to evaluate pathology data collected as described above.

Further details on methods, systems, and algorithms for using pathology data to classify cancer and identify targeted therapies are discussed, for example, in U.S. Patent No. 10,957,041, 11,244,763, 11,848,107, and 11,145,416, the contents of which are each hereby incorporated by reference, in their entireties, for all purposes.

In some embodiments, imaging data 128-2 collected during clinical evaluation includes features identified by review of in-vitro and/or in-vivo imaging results (*e.g.,* of a tumor site), for example a size of a tumor, tumor size differentials over time (such as during treatment or during other periods of change). In some embodiments, imaging data 128-2 includes features determined using machine learning algorithms to evaluate imaging data collected as described above.

Further details on methods, systems, and algorithms for using medical imaging to classify cancer and identify targeted therapies are discussed, for example, in U.S. Patent No. 10,957,041, 11,244,763, 11,848,107, and 11,145,416, the contents of which are each hereby incorporated by reference, in their entireties, for all purposes.

In some embodiments, tissue culture / organoid data 128-3 collected during clinical evaluation includes features identified by evaluation of cultured tissue from the subject. For instance, in some embodiments, tissue samples obtained from the patients (*e.g.,* tumor tissue, normal tissue, or both) are cultured (*e.g.,* in liquid culture, solid-phase culture, and/or organoid culture) and various features, such as cell morphology, growth characteristics, genomic alterations, and/or drug sensitivity, are evaluated. In some embodiments, tissue culture / organoid data 128-3 includes features determined using machine learning algorithms to evaluate tissue culture / organoid data collected as described above. Examples of tissue organoid (*e.g.,* personal tumor organoid) culturing and feature extractions thereof are described in PCT publication No. WO2021/081253 and U.S. Patent No. 11,629,385, the contents of which are each hereby incorporated by reference, in their entireties, for all purposes.

In some embodiments, the method further comprises obtaining the liquid biopsy sample from a sample repository or database (*e.g.,* BiolVT, TSC Biosample Repository, BioLINCC, *etc.*). In some embodiments, the liquid biopsy sample is obtained from the subject at least 1 hour, at least 2 hours, at least 12 hours, at least 1 day, at least 2 days, at least 1 week, at least 1 month, or at least 1 year prior to processing and/or sequencing the liquid biopsy sample. In some such embodiments, the liquid biopsy sample is fresh, frozen, dried, and/or fixed. In some embodiments, the liquid biopsy sample is processed and/or sequenced at least 1 day, at least 2 days, at least 1 week, at least 1 month, or at least 1 year prior to obtaining the first dataset. For example, in some embodiments, the sequencing data for the liquid biopsy sample are obtained from a data repository (*e.g.,* GenBank, NCBI Assembly, DNA DataBank of Japan, European Nucleotide Archive, European Variation Archive, *etc*.).

*Block 512.* Referring to block 512, in some embodiments, the reference sample consists or comprises buffy coat obtained from the whole blood of the test subject. The buffy coat is the thin, whitish layer found between the red blood cells and plasma when whole blood is centrifuged. It comprises primarily white blood cells (leukocytes) and platelets, making it a concentrated source of these components.

To obtain the buffy coat, in some embodiments, blood is drawn from the test subject into a tube containing an anticoagulant to prevent clotting. The blood sample is spun at high speed in a centrifuge. This separates its components based on density: the plasma (least dense) remains at the top, the buffy coat (middle layer) forms as a thin, white layer, and the red blood cells (most dense) settle at the bottom. The buffy coat is pipetted or removed from the tube for further analysis in accordance with the present disclosure.

*Block 514.* Referring to block 514, in some embodiments, the first sequencing reaction is a panel-based sequencing reaction of a plurality of loci.

In some embodiments, the plurality of sequence reads is from a panel-enriched sequencing reaction that includes a first subset of sequence reads corresponding to cfDNA fragments targeted by one or more probes in a targeted enrichment panel. In some embodiments, the sequencing reaction is a total cfDNA fragment sequencing reaction. That is, in some embodiments, cfDNA fragments are not enriched using probes in a targeted enrichment panel prior to sequencing.

In some embodiments, wet lab processing 204 includes enriching (310) a sequencing library, or pool of sequencing libraries, for target nucleic acids, *e.g.,* nucleic acids encompassing loci that are informative for precision oncology and/or used as internal controls for the sequencing or bioinformatics processes. In some embodiments, enrichment is achieved by hybridizing target nucleic acids in the sequencing library to probes that hybridize to the target sequences, and then isolating the captured nucleic acids away from off-target nucleic acids that are not bound by the capture probes. Of course, some off-target nucleic acids will remain in the final sequencing pool.

Advantageously, enriching for target sequences prior to sequencing nucleic acids significantly reduces the costs and time associated with sequencing, facilitates multiplex sequencing by allowing multiple samples to be mixed together for a single sequencing reaction, and significantly reduces the computation burden of aligning the resulting sequence reads, as a result of significantly reducing the total amount of nucleic acids analyzed from each sample.

Accordingly, in some embodiments, a panel-enriched sequencing reaction is performed at a read depth of at least 1,000x. In some embodiments, a panel-enriched sequencing reaction is performed at a read depth of at least 100x, at least 500x, at least 1000x, at least 5000x, at least 10,000x, at least 50,000x, or greater. In some embodiments, the panel-enriched sequencing reaction is performed at a read depth of no more than 100,000x, no more than 50,000x, no more than 10,000x, no more than 5000x, or less. In some embodiments, a panel-enriched sequencing reaction is performed at a read depth of from 100x to 50,000x, from 100X to 10,000x, from 100x to 5000x, from 100x to 1000x, or from 100x to 500x. In some embodiments, a panel-enriched sequencing reaction is performed at a read depth of from 500x to 50,000x, from 500x to 10,000x, from 500x to 5000x, or from 500x to 1000x. In some embodiments, a panel-enriched sequencing reaction is performed at a read depth of from 1000x to 50,000x, from 1000x to 10,000x, or from 1000x to 5000x.

In some embodiments, the enrichment is performed prior to pooling multiple nucleic acid sequencing libraries. However, in other embodiments, the enrichment is performed after pooling nucleic acid sequencing libraries, which has the advantage of reducing the number of enrichment assays that have to be performed.

In some embodiments, the enrichment is performed prior to generating a nucleic acid sequencing library. This has the advantage that fewer reagents are needed to perform both the enrichment (because there are fewer target sequences at this point, prior to library amplification) and the library production (because there are fewer nucleic acid molecules to tag and amplify after the enrichment). However, this raises the possibility of pull-down bias and/or that small variations in the enrichment protocol will result in less consistent results.

In some embodiments, nucleic acid libraries are pooled (two or more DNA libraries may be mixed to create a pool) and treated with reagents to reduce off-target capture, for example Human COT-1 and/or IDT xGen Universal Blockers. Pools may be dried in a vacufuge and resuspended. DNA libraries or pools may be hybridized to a probe set (for example, a probe set specific to a panel that includes loci from at least 100, 600, 1,000, 10,000, etc. of the 19,000 known human genes) and amplified with commercially available reagents (for example, the KAPA HiFi HotStart ReadyMix). For example, in some embodiments, a pool is incubated in an incubator, PCR machine, water bath, or other temperature-modulating device to allow probes to hybridize. Pools may then be mixed with Streptavidin-coated beads or another means for capturing hybridized DNA-probe molecules, such as DNA molecules representing exons of the human genome and/or genes selected for a genetic panel.

Pools may be amplified and purified more than once using commercially available reagents, for example, the KAPA HiFi Library Amplification kit and Axygen MAG PCR clean up beads, respectively. The pools or DNA libraries may be analyzed to determine the concentration or quantity of DNA molecules, for example by using a fluorescent dye (for example, PicoGreen pool quantification) and a fluorescence microplate reader, standard spectrofluorometer, or filter fluorometer. In one example, the DNA library preparation and/or capture is performed with an automated system, using a liquid handling robot (for example, a SciClone NGSx).

In some embodiments, a plurality of nucleic acid probes (*e.g.,* a probe set) is used to enrich one or more target sequences in a nucleic acid sample (*e.g.,* an isolated nucleic acid sample or a nucleic acid sequencing library), *e.g.,* where one or more target sequences is informative for precision oncology. For instance, in some embodiments, one or more of the target sequences encompasses a locus that is associated with an actionable allele. That is, variations of the target sequence are associated with targeted therapeutic approaches. In some embodiments, one or more of the target sequences and/or a property of one or more of the target sequences is used in a classifier trained to distinguish two or more cancer states.

In some embodiments, the probe set includes probes targeting one or more gene loci, *e.g.,* exon or intron loci. In some embodiments, the probe set includes probes targeting one or more loci not encoding a protein, *e.g.,* regulatory loci, miRNA loci, and other non-coding loci, *e.g.,* that have been found to be associated with cancer. In some embodiments, the plurality of loci includes at least 25, 50, 100, 150, 200, 250, 300, 350, 400, 500, 750, 1000, 2500, 5000, or more human genomic loci.

In some embodiments, a panel-enriched sequencing reaction uses a sequencing panel that enriches for at least 50 genes. In some embodiments, a panel-enriched sequencing reaction uses a sequencing panel that enriches for at least 25 genes, at least 50 genes, at least 100 genes, at least 250 genes, at least 500 genes, at least 1000 genes, at least 2500 genes, at least 5000 genes, or more. In some embodiments, a panel-enriched sequencing reaction uses a sequencing panel that enriches for no more than 40,000 genes, no more than 20,000 genes, no more than 10,000 genes, no more than 5000 genes, no more than 2500 genes, no more than 1000 genes, or less. In some embodiments, a panel-enriched sequencing reaction uses a sequencing panel that enriches for from 25 genes to 10,000 genes, from 25 genes to 5000 genes, from 25 genes to 2500 genes, from 25 genes to 1000 genes, from 25 genes to 500 genes, or from 25 genes to 250 genes. In some embodiments, a panel-enriched sequencing reaction uses a sequencing panel that enriches for from 50 genes to 10,000 genes, from 50 genes to 5000 genes, from 50 genes to 2500 genes, from 50 genes to 1000 genes, from 50 genes to 500 genes, or from 50 genes to 250 genes. In some embodiments, a panel-enriched sequencing reaction uses a sequencing panel that enriches for from 100 genes to 10,000 genes, from 100 genes to 5000 genes, from 100 genes to 2500 genes, from 100 genes to 1000 genes, from 100 genes to 500 genes, or from 100 genes to 250 genes.

In some embodiments, the plurality of probe sequences used to enrich cell-free DNA fragments in the liquid biopsy sample in a panel-enriched sequencing reaction collectively map to at least 25 different genes in a human reference genome. In some embodiments, a panel-enriched sequencing reaction uses a sequencing panel that enriches for at least 25 human genes, at least 50 human genes, at least 100 human genes, at least 250 human genes, at least 500 human genes, at least 1000 human genes, at least 2500 human genes, at least 5000 human genes, or more. In some embodiments, a panel-enriched sequencing reaction uses a sequencing panel that enriches for no more than 40,000 human genes, no more than 20,000 human genes, no more than 10,000 human genes, no more than 5000 human genes, no more than 2500 human genes, no more than 1000 human genes, or less. In some embodiments, a panel-enriched sequencing reaction uses a sequencing panel that enriches for from 25 human genes to 10,000 human genes, from 25 human genes to 5000 human genes, from 25 human genes to 2500 human genes, from 25 human genes to 1000 human genes, from 25 human genes to 500 human genes, or from 25 human genes to 250 human genes. In some embodiments, a panel-enriched sequencing reaction uses a sequencing panel that enriches for from 50 human genes to 10,000 human genes, from 50 human genes to 5000 human genes, from 50 human genes to 2500 human genes, from 50 human genes to 1000 human genes, from 50 human genes to 500 human genes, or from 50 human genes to 250 human genes. In some embodiments, a panel-enriched sequencing reaction uses a sequencing panel that enriches for from 100 human genes to 10,000 human genes, from 100 human genes to 5000 human genes, from 100 human genes to 2500 human genes, from 100 human genes to 1000 human genes, from 100 human genes to 500 human genes, or from 100 human genes to 250 human genes.

In some embodiments, the plurality of probe sequences used to enrich cell-free DNA fragments in the liquid biopsy sample in a first panel-enriched sequencing reaction collectively map to at least 25 different genes in a human reference genome. In some embodiments, the plurality of probe sequences collectively maps to at least 50, at least 100, at least 250, at least 500, or at least 1000 different genes in the human reference genome. In some embodiments, the plurality of probe sequences collectively maps to at least 10 of the genes listed in Table 1. In some embodiments, the plurality of probe sequences collectively maps to at least any 20, 25, 30, 40, 50, 60, 75, 100, or all 105 of the genes listed in Table 1.

In some embodiments, the plurality of probe sequences collectively maps to at least 10 of the genes listed in Table 2. In some embodiments, the plurality of probe sequences collectively maps to at least any 20, 25, 30, 40, 50, 60, 75, 100, or all 105 of the genes listed in Table 2.

For example, in some embodiments, a targeted enrichment panel comprises any of the embodiments described above in the present disclosure. In some embodiments, the targeted enrichment panel includes probes targeting one or more gene loci, *e.g.,* exon or intron loci. In some embodiments, the targeted enrichment panel includes probes targeting one or more loci not encoding a protein, *e.g.,* regulatory loci, miRNA loci, and other non-coding loci, *e.g.,* that have been found to be associated with cancer. In some embodiments, the plurality of loci includes at least any 25, 50, 100, 150, 200, 250, 300, 350, 400, 500, 750, 1000, 2500, 5000, or more human genomic loci.

In some embodiments, the targeted enrichment panel includes probes targeting one or more of the genes listed in Table 1. In some embodiments, the targeted enrichment panel includes probes targeting at least any 5 of the genes listed in Table 1. In some embodiments, the targeted enrichment panel includes probes targeting at least any 10 of the genes listed in Table 1. In some embodiments, the targeted enrichment panel includes probes targeting at least any 25 of the genes listed in Table 1. In some embodiments, the targeted enrichment panel includes probes targeting at least any 50 of the genes listed in Table 1. In some embodiments, the targeted enrichment panel includes probes targeting at least any 75 of the genes listed in Table 1. In some embodiments, the targeted enrichment panel includes probes targeting at least any 100 of the genes listed in Table 1. In some embodiments, the targeted enrichment panel includes probes targeting all of the genes listed in Table 1.

In some embodiments, the targeted enrichment panel includes probes targeting one or more of the genes listed in Table 2. In some embodiments, the targeted enrichment panel includes probes targeting at least any 5 of the genes listed in Table 2. In some embodiments, the targeted enrichment panel includes probes targeting at least any 10 of the genes listed in Table 2. In some embodiments, the targeted enrichment panel includes probes targeting at least any 25 of the genes listed in Table 2. In some embodiments, the targeted enrichment panel includes probes targeting at least any 50 of the genes listed in Table 2. In some embodiments, the targeted enrichment panel includes probes targeting at least any 75 of the genes listed in Table 2. In some embodiments, the targeted enrichment panel includes probes targeting at least any 100 of the genes listed in Table 2. In some embodiments, the targeted enrichment panel includes probes targeting all of the genes listed in Table 2.

In some embodiments, the probe set includes probes targeting one or more of the genes listed in Table 1. In some embodiments, the probe set includes probes targeting at least any 5 of the genes listed in Table 1. In some embodiments, the probe set includes probes targeting at least any 10 of the genes listed in Table 1. In some embodiments, the probe set includes probes targeting at least any 25 of the genes listed in Table 1. In some embodiments, the probe set includes probes targeting at least any 50 of the genes listed in Table 1. In some embodiments, the probe set includes probes targeting at least any 75 of the genes listed in Table 1. In some embodiments, the probe set includes probes targeting at least any 100 of the genes listed in Table 1. In some embodiments, the probe set includes probes targeting all of the genes listed in Table 1.

In some embodiments, the probe set includes probes targeting one or more of the genes listed in Table 2. In some embodiments, the probe set includes probes targeting at least any 5 of the genes listed in Table 2. In some embodiments, the probe set includes probes targeting at least any 10 of the genes listed in Table 2. In some embodiments, the probe set includes probes targeting at least any 25 of the genes listed in Table 2. In some embodiments, the probe set includes probes targeting at least any 50 of the genes listed in Table 2. In some embodiments, the probe set includes probes targeting at least any 75 of the genes listed in Table 2. In some embodiments, the probe set includes probes targeting at least any 100 of the genes listed in Table 2. In some embodiments, the probe set includes probes targeting all of the genes listed in Table 2.

**Table 1. An example panel of 105 genes.**

| | | | | | | |
|---|---|---|---|---|---|---|
| ALK | B2M | ERRFI1 | IDH2 | MSH6 | PIK3R1 | SPOP |
| FGFR2 | BAP1 | ESR1 | JAK1 | MTOR | PMS2 | STK11 |
| FGFR3 | BRCA1 | EZH2 | JAK2 | MYCN | PTCH1 | TERT |
| NTRK1 | BRCA2 | FBXW7 | JAK3 | NF1 | PTEN | TP53 |
| RET | BTK | FGFR1 | KDR | NF2 | PTPN11 | TSC1 |
| ROS1 | CCND1 | FGFR4 | KEAP1 | NFE2L2 | RAD51C | TSC2 |
| BRAF | CCND2 | FLT3 | KIT | NOTCH1 | RAF1 | UGT1A1 |
| AKT1 | CCND3 | FOXL2 | KRAS | NPM1 | RB1 | VHL |
| AKT2 | CDH1 | GATA3 | MAP2K1 | NRAS | RHEB | CCNE1 |
| APC | CDK4 | GNA11 | MAP2K2 | PALB2 | RHOA | CD274 |
| AR | CDK6 | GNAQ | MAPK1 | PBRM1 | RIT1 | EGFR |
| ARAF | CDKN2A | GNAS | MLH1 | PDCD1LG2 | RNF43 | ERBB2 |
| ARID1A | CTNNB1 | HNF1A | MPL | PDGFRA | SDHA | MET |
| ATM | DDR2 | HRAS | MSH2 | PDGFRB | SMAD4 | MYC |
| ATR | DPYD | IDH1 | MSH3 | PIK3CA | SMO | KMT2A |

**Table 2. An example panel of 523 genes.**

| | | | | | |
|---|---|---|---|---|---|
| BCC3 | CIC | FGFR4 | KLF4 | PBRM1 | SIRPA |
| ABL1 | CKS1B | FH | KLHL6 | PDCD1 | SLC34A2 |
| ABL2 | CREBBP | FHIT | KLLN | PDCD1LG2 | SLC9A3R1 |
| ABRAXAS1 | CRKL | FLCN | KMT2A | PDGFRA | SLFN11 |
| ACVR1 | CSF1R | FLT1 | KMT2C | PDGFRB | SLIT2 |
| ACVR1B | CSF3R | FLT3 | KMT2D | PDK1 | SMAD2 |
| AJUBA | CTC1 | FLT4 | KRAS | PHGDH | SMAD3 |
| AKT1 | CTCF | FOLH1 | LATS1 | PHLPP1 | SMAD4 |
| AKT2 | CTLA4 | FOXA1 | LCK | PHLPP2 | SMARCA2 |
| AKT3 | CTNNA1 | FOXL2 | LMO1 | PIAS4 | SMARCA4 |
| ALK | CTNNB1 | FOXO1 | LRP1B | PIK3C2B | SMARCB1 |
| ALOX12B | CUL3 | FOXO3 | LTK | PIK3C2G | SMC1A |
| AMER1 | CUL4A | FOXP1 | LYN | PIK3CA | SMC3 |
| APC | CUX1 | FRS2 | LZTR1 | PIK3CB | SMO |
| APLNR | CXCR4 | FUBP1 | MAF | PIK3CD | SNCAIP |
| AR | CYLD | GABRA6 | MALT1 | PIK3CG | SOCS1 |
| ARAF | CYP17A1 | GALNT12 | MAP2K1 | PIK3R1 | SOS1 |
| ARFRP1 | CYSLTR2 | GATA1 | MAP2K2 | PIK3R2 | SOX2 |
| ARID1A | DAXX | GATA3 | MAP2K4 | PIM1 | SOX9 |
| ARID1B | DDB2 | GATA4 | MAP3K1 | PLCG1 | SPEN |
| ARID2 | DDR1 | GATA6 | MAP3K13 | PLCG2 | SPOP |
| ASNS | DDR2 | GID4 | MAP3K21 | PMS1 | SRC |
| ASXL1 | DDX3X | GLI2 | MAP3K7 | PMS2 | SRSF2 |
| ATM | DDX41 | GNA11 | MAPK1 | POLA1 | STAG2 |
| ATR | DEPTOR | GNA13 | MAPK3 | POLD1 | STAT3 |
| ATRX | DICER1 | GNAQ | MAX | POLE | STAT5B |
| AURKA | DIS3 | GNAS | MC1R | POLQ | STAT6 |
| AURKB | DNMT1 | GPC3 | MCL1 | POT1 | STK11 |
| AURKC | DNMT3A | GPS2 | MDM2 | PPARG | SUFU |
| AXIN1 | DOT1L | GREM1 | MDM4 | PPM1D | SUZ12 |
| AXIN2 | DPYD | GRIN2A | MED12 | PPP2R1A | SYK |
| AXL | EBF1 | GRM3 | MEF2B | PPP2R2A | TBX3 |
| B2M | EED | GSK3B | MEN1 | PPP6C | TCF7L2 |
| BAP1 | EEF2 | GSTP1 | MERTK | PRDM1 | TEK |
| BARD1 | EGFR | H3F3A | MET | PREX2 | TERC |
| BAX | EGLN1 | HAVCR2 | MITF | PRKACA | TERT |
| BCL2 | EIF1AX | HDAC1 | MKNK1 | PRKAR1A | TET2 |
| BCL2L1 | ELF3 | HDAC2 | MLH1 | PRKCI | TFEB |
| BCL2L11 | EMSY | HGF | MLH3 | PRKN | TGFB1 |
| BCL2L2 | EP300 | HIF1A | MPL | PTCH1 | TGFBR1 |
| BCL6 | EPCAM | HIST1H3B | MRE11 | PTEN | TGFBR2 |
| BCLAF1 | EPHA2 | HLA-B | MS4A1 | PTK2 | TIGIT |
| BCOR | EPHA3 | HNF1A | MSH2 | PTPN11 | TIPARP |
| BCORL1 | EPHB1 | HNF1B | MSH3 | PTPN13 | TMEM127 |
| BCR | EPHB4 | HOXB13 | MSH6 | PTPRD | TMPRSS2 |
| BIRC3 | ERBB2 | HRAS | MST1R | PTPRO | TNFAIP3 |
| BLM | ERBB3 | HSD3B1 | MTAP | PTPRT | TNFRSF14 |
| BMPR1A | ERBB4 | HSP90AA1 | MTHFR | QKI | TNFRSF17 |
| BRAF | ERCC2 | HSPH1 | MTOR | RAC1 | TOP1 |
| BRCA1 | ERCC3 | ID3 | MUC16 | RAD21 | TOP2A |
| BRCA2 | ERCC4 | IDH1 | MUTYH | RAD50 | TP53 |
| BRD4 | ERCC6 | IDH2 | MYB | RAD51 | TP53BP1 |
| BRIP1 | ERG | IFNA21 | MYC | RAD51B | TP63 |
| BTG1 | ERRFI1 | IFNAR1 | MYCL | RAD51C | TRAF3 |
| BTG2 | ESR1 | IFNAR2 | MYCN | RAD51D | TRAF7 |
| BTK | ETNK1 | IFNG | MYD88 | RAD52 | TSC1 |
| CALR | ETV1 | IFNGR1 | NBN | RAD54L | TSC2 |
| CARD11 | ETV4 | IFNGR2 | NCOA2 | RAF1 | TSHR |
| CARM1 | ETV5 | IFNW1 | NCOR1 | RARA | TYMS |
| CASP8 | ETV6 | IGF1 | NF1 | RASA1 | TYRO3 |
| CBFB | EWSR1 | IGF1R | NF2 | RB1 | U2AF1 |
| CBL | EZH2 | IKBKE | NFE2L2 | RBM10 | UGT1A1 |
| CCND1 | EZR | IKZF1 | NFKBIA | RECQL4 | VEGFA |
| CCND2 | FAM46C | IL10RA | NKX2-1 | REL | VHL |
| CCND3 | FANCA | IL32 | NOTCH1 | RET | VSIR |
| CCNE1 | FANCC | IL6R | NOTCH2 | RHEB | WEE1 |
| CD22 | FANCD2 | IL7R | NOTCH3 | RHOA | WNK1 |
| CD274 | FANCE | IMPDH1 | NOTCH4 | RICTOR | WRN |
| CD70 | FANCG | ING1 | NPM1 | RIT1 | WT1 |
| CD74 | FANCI | INPP4B | NQO1 | RNF43 | XBP1 |
| CD79A | FANCL | INSR | NRAS | ROS1 | XPA |
| CD79B | FANCM | IRF1 | NRG1 | RPS6KB1 | XPC |
| CDC73 | FAS | IRF2 | NSD1 | RPTOR | XPO1 |
| CDH1 | FAT1 | IRF4 | NSD2 | RRM1 | XRCC1 |
| CDK12 | FBXW7 | IRS2 | NSD3 | RSF1 | XRCC2 |
| CDK4 | FCGR2A | JAK1 | NT5C2 | RSPO2 | YEATS4 |
| CDK6 | FCGR3A | JAK2 | NTRK1 | RUNX1 | ZFHX3 |
| CDK8 | FGF10 | JAK3 | NTRK2 | RXRA | ZMYM3 |
| CDK9 | FGF12 | JUN | NTRK3 | SDC4 | ZNF217 |
| CDKN1A | FGF14 | KAT6A | NUTM1 | SDHA | ZNF703 |
| CDKN1B | FGF19 | KDM5A | P2RY8 | SDHAF2 | ZNF750 |
| CDKN2A | FGF23 | KDM5C | PAK1 | SDHB | ZNRF3 |
| CDKN2B | FGF3 | KDM5D | PALB2 | SDHC | ZRSR2 |
| CDKN2C | FGF4 | KDM6A | PALLD | SDHD | |
| CEBPA | FGF6 | KDR | PARP1 | SETBP1 | |
| CHD4 | FGFR1 | KEAP1 | PARP2 | SETD2 | |
| CHEK1 | FGFR2 | KEL | PARP3 | SF3B1 | |
| CHEK2 | FGFR3 | KIT | PAX5 | SGK1 | |

In some embodiments, a total cfDNA fragment sequencing reaction is performed at a read depth of at least 1X. In some embodiments, a panel-enriched sequencing reaction is performed at a read depth of at least 2X, at least 3X, at least 4X, at least 5X, at least 10X, at least 25X, at least 50X, at least 100X, at least 250X, or greater. In some embodiments, a total cfDNA fragment sequencing reaction is performed at a read depth of no more than 1000X, no more than 500X, no more than 100X, no more than 50X, or less. In some embodiments, a total cfDNA fragment sequencing reaction is performed at a read depth of from 1X to 500X, from 1X to 100X, or from 1X to 50X. In some embodiments, a total cfDNA fragment sequencing reaction is performed at a read depth of from 2.5X to 500X, from 2.5X to 100X, or from 2.5X to 50X. In some embodiments, a total cfDNA fragment sequencing reaction is performed at a read depth of from 5X to 500X, from 5X to 100X, or from 5X to 50X. In some embodiments, a total cfDNA fragment sequencing reaction is performed at a read depth of from 10X to 500X, from 10X to 100X, or from 10X to 50X.

In some embodiments, the probe set includes probes targeting one or more of the genes listed in List 1, provided below. In some embodiments, the probe set includes probes targeting at least any 5 of the genes listed in List 1. In some embodiments, the probe set includes probes targeting at least any 10 of the genes listed in List 1. In some embodiments, the probe set includes probes targeting at least any 25 of the genes listed in List 1. In some embodiments, the probe set includes probes targeting at least any 50 of the genes listed in List 1. In some embodiments, the probe set includes probes targeting at least any 70 of the genes listed in List 1. In some embodiments, the probe set includes probes targeting all of the genes listed in List 1.

In some embodiments, the probe set includes probes targeting one or more of the genes listed in List 2, provided below. In some embodiments, the probe set includes probes targeting at least any 5 of the genes listed in List 2. In some embodiments, the probe set includes probes targeting at least any 10 of the genes listed in List 2. In some embodiments, the probe set includes probes targeting at least any 25 of the genes listed in List 2. In some embodiments, the probe set includes probes targeting at least any 50 of the genes listed in List 2. In some embodiments, the probe set includes probes targeting at least any 75 of the genes listed in List 2. In some embodiments, the probe set includes probes targeting at least any 100 of the genes listed in List 2. In some embodiments, the probe set includes probes targeting all of the genes listed in List 2.

In some embodiments, panels of genes including one or more genes from the following lists are used for analyzing specimens, sequencing, and/or identification. In some embodiments, panels of genes for analyzing specimens, sequencing, and/or identification include one or more genes from List 1 or List 2. In some embodiments, panels of genes for analyzing specimens, sequencing, and/or identification include one or more genes from:

List 1: AKT1 (14q32.33), ALK (2p23.2-23.1), APC (5q22.2), AR (Xq12), ARAF (Xp11.3), ARID1A (1p36.11), ATM (11q22.3), BRAF (7q34), BRCA1 (17q21.31), BRCA2 (13q13.1), CCND1 (11q13.3), CCND2 (12p13.32), CCNE1 (19q12), CDH1 (16q22.1), CDK4 (12q14.1), CDK6 (7q21.2), CDKN2A (9p21.3), CTNNB1 (3p22.1), DDR2 (1q23.3), EGFR (7p11.2), ERBB2 (17q12), ESR1 (6q25.1-25.2), EZH2 (7q36.1), FBXW7 (4q31.3), FGFR1 (8p11.23), FGFR2 (10q26.13), FGFR3 (4p16.3), GATA3 (10p14), GNA11 (19p13.3), GNAQ (9q21.2), GNAS (20q13.32), HNF1A (12q24.31), HRAS (11p15.5), IDH1 (2q34), IDH2 (15q26.1), JAK2 (9p24.1), JAK3 (19p13.11), KIT (4q12), KRAS (12p12.1), MAP2K1 (15q22.31), MAP2K2 (19p13.3), MAPK1 (22q11.22), MAPK3 (16p11.2), MET (7q31.2), MLH1 (3p22.2), MPL (1p34.2), MTOR (1p36.22), MYC (8q24.21), NF1 (17q11.2), NFE2L2 (2q31.2), NOTCH1 (9q34.3), NPM1 (5q35.1), NRAS (1p13.2), NTRK1 (1q23.1), NTRK3 (15q25.3), PDGFRA (4q12), PIK3CA (3q26.32), PTEN (10q23.31), PTPN11 (12q24.13), RAF1 (3p25.2), RB1 (13q14.2), RET (10q11.21), RHEB (7q36.1), RHOA (3p21.31), RIT1 (1q22), ROS1 (6q22.1), SMAD4 (18q21.2), SMO (7q32.1), STK11 (19p13.3), TERT (5p15.33), TP53 (17p13.1), TSC1 (9q34.13), and VHL (3p25.3).

List 2: ABL1, ACVR1B, AKT1, AKT2, AKT3, ALK, ALOX12B, AMER1 (FAM123B), APC, AR, ARAF, ARFRP1, ARID1A, ASXL1, ATM, ATR, ATRX, AURKA, AURKB, AXIN1, AXL, BAP1, BARD1, BCL2, BCL2L1, BCL2L2, BCL6, BCOR, BCORL1, BRAF, BRCA1, BRCA2, BRD4, BRIP1, BTG1, BTG2, BTK, C11orf30 (EMSY), C17orf39 (GID4), CALR, CARD11, CASP8, CBFB, CBL, CCND1, CCND2, CCND3, CCNE1, CD22, CD274 (PD-L1), CD70, CD79A, CD79B, CDC73, CDH1, CDK12, CDK4, CDK6, CDK8, CDKN1A, CDKN1B, CDKN2A, CDKN2B, CDKN2C, CEBPA, CHEK1, CHEK2, CIC, CREBBP, CRKL, CSF1R, CSF3R, CTCF, CTNNA1, CTNNB1, CUL3, CUL4A, CXCR4, CYP17A1, DAXX, DDR1, DDR2, DIS3, DNMT3A, DOT1L, EED, EGFR, EP300, EPHA3, EPHB1, EPHB4, ERBB2, ERBB3, ERBB4, ERCC4, ERG, ERRFI1, ESR1, EZH2, FAM46C, FANCA, FANCC, FANCG, FANCL, FAS, FBXW7, FGF10, FGF12, FGF14, FGF19, FGF23, FGF3, FGF4, FGF6, FGFR1, FGFR2, FGFR3, FGFR4, FH, FLCN, FLT1, FLT3, FOXL2, FUBP1, GABRA6, GATA3, GATA4, GATA6, GNA11, GNA13, GNAQ, GNAS, GRM3, GSK3B, H3F3A, HDAC1, HGF, HNF1A, HRAS, HSD3B1, ID3, IDH1, IDH2, IGF1R, IKBKE, IKZF1, INPP4B, IRF2, IRF4, IRS2, JAK1, JAK2, JAK3, JUN, KDM5A, KDM5C, KDM6A, KDR, KEAP1, KEL, KIT, KLHL6, KMT2A, KMT2D (MLL2), KRAS, LTK, LYN, MAF, MAP2K1 (MEK1), MAP2K2 (MEK2), MAP2K4, MAP3K1, MAP3K13, MAPK1, MCL1, MDM2, MDM4, MED12, MEF2B, MEN1, MERTK, MET, MITF, MKNK1, MLH1, MPL, MRE11A, MSH2, MSH3, MSH6, MST1R, MTAP, MTOR, MUTYH, MYC, MYCL (MYCL1), MYCN, MYD88, NBN, NF1, NF2, NFE2L2, NFKBIA, NKX2-1, NOTCH1, NOTCH2, NOTCH3, NPM1, NRAS, NSD3 (WHSC1L1), NT5C2, NTRK1, NTRK2, NTRK3, P2RY8, PALB2, PARK2, PARP1, PARP2, PARP3, PAX5, PBRM1, PDCD1 (PD-1), PDCD1LG2 (PD-L2), PDGFRA, PDGFRB, PDK1, PIK3C2B, PIK3C2G, PIK3CA, PIK3CB, PIK3R1, PIM1, PMS2, POLD1, POLE, PPARG, PPP2R1A, PPP2R2A, PRDM1, PRKAR1A, PRKCI, PTCH1, PTEN, PTPN11, PTPRO, QKI, RAC1, RAD21, RAD51, RAD51B, RAD51C, RAD51D, RAD52, RAD54L, RAF1, RARA, RB1, RBM10, REL, RET, RICTOR, RNF43, ROS1, RPTOR, SDHA, SDHB, SDHC, SDHD, SETD2, SF3B1, SGK1, SMAD2, SMAD4, SMARCA4, SMARCB1, SMO, SNCAIP, SOCS1, SOX2, SOX9, SPEN, SPOP, SRC, STAG2, STAT3, STK11, SUFU, SYK, TBX3, TEK, TERC, TERT, TET2, ncRNA, TGFBR2, TIPARP, TNFAIP3, TNFRSF14, TP53, TSC1, TSC2, TYRO3, U2AF1, VEGFA, VHL, WHSC1, WT1, XPO1, XRCC2, ZNF217, and ZNF703.

Generally, probes for enrichment of nucleic acids (*e.g.,* cfDNA obtained from a liquid biopsy sample) include DNA, RNA, or a modified nucleic acid structure with a base sequence that is complementary to a locus of interest. For instance, a probe designed to hybridize to a locus in a cfDNA molecule can contain a sequence that is complementary to either strand, because the cfDNA molecules are double stranded. In some embodiments, each probe in the plurality of probes includes a nucleic acid sequence that is identical or complementary to at least 10, at least 11, at least 12, at least 13, at least 14, or at least 15 consecutive bases of a locus of interest. In some embodiments, each probe in the plurality of probes includes a nucleic acid sequence that is identical or complementary to at least 20, 25, 30, 40, 50, 75, 100, 150, 200, or more consecutive bases of a locus of interest.

Targeted panels provide several benefits for nucleic acid sequencing. For example, in some embodiments, algorithms for discriminating between, *e.g.,* a first and second cancer condition can be trained on smaller, more informative data sets (*e.g.,* fewer genes), which leads to more computationally efficient training of classifiers that discriminate between the first and second cancer states. Such improvements in computational efficiency, owing to the reduced size of the discriminating gene set, can advantageously either be used to speed up classifier training or be used to improve the performance of such classifiers (*e.g.,* through more extensive training of the classifier).

In some embodiments, the gene panel is a whole-exome panel that analyzes the exomes of a biological sample. In some embodiments, the gene panel is a whole-genome panel that analyzes the genome of a specimen. In some preferred embodiments, the gene panel is optimized for use with liquid biopsy samples (*e.g.,* to provide clinical decision support for solid tumors). See, for example, Table 1 above.

In some embodiments, the probes include additional nucleic acid sequences that do not share any homology to the loci of interest. For example, in some embodiments, the probes also include nucleic acid sequences containing an identifier sequence, *e.g.,* a unique molecular identifier (UMI), *e.g.,* that is unique to a particular sample or subject. Examples of identifier sequences are described, for example, in Kivioja et al., 2011, Nat. Methods 9(1), pp. 72-74 and Islam et al., 2014, Nat. Methods 11(2), pp. 163-66, which are incorporated by reference herein. Similarly, in some embodiments, the probes also include primer nucleic acid sequences useful for amplifying the nucleic acid molecule of interest, *e.g.,* using PCR. In some embodiments, the probes also include a capture sequence designed to hybridize to an anti-capture sequence for recovering the nucleic acid molecule of interest from the sample.

Likewise, in some embodiments, the probes each include a non-nucleic acid affinity moiety covalently attached to nucleic acid molecule that is complementary to the loci of interest, for recovering the nucleic acid molecule of interest. Non-limited examples of non-nucleic acid affinity moieties include biotin, digoxigenin, and dinitrophenol. In some embodiments, the probe is attached to a solid-state surface or particle, *e.g.,* a dipstick or magnetic bead, for recovering the nucleic acid of interest. In some embodiments, the methods described herein include amplifying the nucleic acids that bound to the probe set prior to further analysis, *e.g.,* sequencing. Methods for amplifying nucleic acids, *e.g.,* by PCR, are well known in the art.

Next-generation sequencing produces millions of short reads (*e.g.,* sequence reads) for each biological sample. Accordingly, in some embodiments, the plurality of sequence reads obtained by next-generation sequencing of cfDNA molecules are DNA sequence reads. In some embodiments, the sequence reads have an average length of at least fifty nucleotides. In other embodiments, the sequence reads have an average length of at least 50, 60, 70, 80, 90, 100, 150, 200, 250, 300, or more nucleotides.

In some embodiments, sequencing is performed after enriching for nucleic acids (*e.g.,* cfDNA, gDNA, and/or RNA) encompassing a plurality of predetermined target sequences, *e.g.,* human genes and/or non-coding sequences associated with cancer. Advantageously, sequencing a nucleic acid sample that has been enriched for target nucleic acids, rather than all nucleic acids isolated from a biological sample, significantly reduces the average time and cost of the sequencing reaction. Accordingly, in some preferred embodiments, the methods described herein include obtaining a plurality of sequence reads of nucleic acids that have been hybridized to a probe set for hybrid-capture enrichment (*e.g.,* of one or more genes listed in Table 1 or of one or more genes listed in Table 2, or one or more genes listed in List 2).

In some embodiments, panel-targeting sequencing is performed to an average on-target depth of at least 500x, at least 750x, at least 1000x, at least 2500x, at least 500x, at least 10,000x, or greater depth. In some embodiments, samples are further assessed for uniformity above a sequencing depth threshold (*e.g.,* 95% of all targeted base pairs at 300x sequencing depth). In some embodiments, the sequencing depth threshold is a minimum depth selected by a user or practitioner.

*Block 516.* Referring to block 516, in some embodiments, the plurality of loci is sequenced at an average sequence depth of at less than 200x or 250x by the first sequencing reaction. In some embodiments the average sequence depth is in accordance with the normal matched buffy coat specimen sequencing at 150x coverage disclosed in Beaubier et al., 2019, "Clinical validation of the tempus xT next-generation targeted oncology sequencing assay," Oncotarget 10(24) pp. 2384-2396, which is hereby incorporated by reference.

In some embodiments, the plurality of loci are sequenced at a read depth of at least at least 50x, at least 60x, at least 70x, at least 80x, at least 90x, as least 100x, at least 110x, at least 120x, at least 130x, at least 140x, at least 150x, or greater. In some embodiments, the panel-enriched sequencing reaction is performed at a read depth, across the plurality of loci, of no more than 1000x, no more than 900x, no more than 800x, no more than 700x, no more than 600x, no more than 600x, no more than 550x, no more than 500x, no more than 400x, no more than 300, or no more than 200d. In some embodiments, the panel-enriched sequencing reaction is performed at a read depth, across the plurality of loci, of from 50x to 2000x, from 60x to 1000x, from 70x to 800x, from 80x to 600x, or from 1002 to 300x.

*Block 518 - 520.* Referring to block 518, in some embodiments, the test subject is afflicted with a cancer condition. *Block 520.* Referring to block 520, in some embodiments, the cancer condition is lung cancer, breast cancer, ovarian cancer, cervical cancer, a uveal melanoma, colorectal cancer, chromophobe renal cell carcinoma, liver cancer, an endocrine tumor, oropharyngeal cancer, retinoblastoma, biliary cancer, adrenal cancer, neural cancer, neuroblastoma, basal cell carcinoma, brain cancer, a non-clear cell renal cell carcinoma, a glioblastoma, a glioma, kidney cancer, gastrointestinal stromal tumor, a medulloblastoma, bladder cancer, gastric cancer, bone cancer, thymoma, prostate cancer, a clear cell renal cell carcinoma, skin cancer, thyroid cancer, a sarcoma, testicular cancer, head and neck cancer, a meningioma, peritoneal cancer, endometrial cancer, pancreatic cancer, mesothelioma, esophageal cancer, small cell lung cancer, HER2 negative breast cancer, ovarian serous carcinoma, HR+ breast cancer, uterine serous carcinoma, a uterine corpus endometrial carcinoma, a gastroesophageal junction adenocarcinoma, gallbladder cancer, chordoma, or a papillary renal cell carcinoma. In some embodiments, the test subject is a patient in a clinical trial.

*Block 522.* Referring to block 522, in some embodiments, the cancer condition is a particular stage of a particular type of cancer.

In some embodiments, the cancer condition is a stage of adrenal cancer, a stage of biliary tract cancer, a stage of bladder cancer, a stage of bone/bone marrow cancer, a stage of brain cancer, a stage of breast cancer, a stage of cervical cancer, a stage of colorectal cancer, a stage of cancer of the esophagus, a stage of gastric cancer, a stage of head/neck cancer, a stage of hepatobiliary cancer, a stage of kidney cancer, a stage of liver cancer, a stage of lung cancer, a stage of ovarian cancer, a stage of pancreatic cancer, a stage of pelvis cancer, a stage of pleura cancer, a stage of prostate cancer, a stage of renal cancer, a stage of skin cancer, a stage of stomach cancer, a stage of testis cancer, a stage of thymus cancer, a stage of thyroid cancer, a stage of uterine cancer, a stage of lymphoma, a stage of melanoma, a stage of multiple myeloma, or a stage of leukemia.

In some embodiments the stage of the cancer is determined using the Tumor, Nodes, Metastasis (TNM) staging system that generally classifies a cancer into one of five stages (0 to IV) based on tumor size, lymph node involvement, and metastasis. In stage 0 (carcinoma in situ), cancer is localized and has not spread beyond the original layer of cells. It is often highly curable with surgical removal. An example of stage 0 cancer is ductal carcinoma in situ (DCIS) in breast cancer. In stage I (early-stage cancer), the tumor is small and has not spread to lymph nodes or distant sites. It is usually treated with surgery, possibly followed by radiation or other therapies. In stage II (localized advanced cancer), the tumor is larger than Stage I, and may have spread to nearby lymph nodes but not distant sites. It requires more aggressive treatment, including surgery, radiation, and/or chemotherapy. In stage III (regional spread), the tumor is larger and more invasive, and the cancer has spread extensively to nearby lymph nodes but not distant organs. Treatment typically includes a combination of surgery, chemotherapy, and radiation therapy. In stage IV (metastatic cancer), the cancer has spread to distant organs (*e.g.,* lungs, liver, bones, brain). Treatment focuses on palliative care, targeted therapy, chemotherapy, immunotherapy, or radiation to manage symptoms and prolong life.

*Block 524.* Referring to block 524, a plurality of tumor sequence reads mapping to the genomic location of the candidate mutation is obtained from a second sequencing reaction using a solid tumor sample of the test subject.

*Block 526.* Referring to block 526, in some embodiments, the second sequencing reaction is a panel-based sequencing reaction of the plurality of loci. In some embodiments, techniques used for the panel-based sequencing are the techniques used for any panel-based sequencing described herein, such as the techniques described in block 514. In some embodiments the plurality of loci sequenced by the second sequencing reaction are any set of genes such as any set of genes described in block 514.

*Block 528.* Referring to block 528, in some embodiments, the plurality of loci sequenced in accordance with block 526 is sequenced at an average sequence depth of greater than 250x or 400x by the second sequencing reaction. In some embodiments the second sequence reaction is low coverage reaction in which tumor specimens are sequenced at 500x. See, Beaubier et al., 2019, "Clinical validation of the tempus xT next-generation targeted oncology sequencing assay," Oncotarget 10(24) pp. 2384-2396, which is hereby incorporated by reference.

In some embodiments the plurality of loci are sequenced at an average cover of between 200x and 1000x. In some embodiments the plurality of loci are sequenced at an average cover of between 300x and 900x. In some embodiments the plurality of loci are sequenced at an average cover of between 400x and 800x. In some embodiments the plurality of loci are sequenced at an average cover of between 350x and 700x. In some embodiments the plurality of loci are sequenced at an average cover of between 400x and 450x.

*Blocks 530-532.* Referring to block 530, in some embodiments, the plurality of loci comprises at least 5 genes, 25 genes, 50 genes, or all the genes in Table 1. Referring to block 532, in some embodiments, the plurality of loci comprises at least 5 genes, 25 genes, 50 genes, or all the genes in Table 2. In some embodiments, the plurality of loci are at least 5 variants, at least 25 variants, at least 50 variants, or all the variants described in Beaubier et al., 2019, "Clinical validation of the tempus xT next-generation targeted oncology sequencing assay," Oncotarget 10(24) pp. 2384-2396, which is hereby incorporated by reference.

*Block 534.* Referring to block 534, in some embodiments, the first sequencing reaction or the second sequencing reaction is a whole genome sequencing. In some such embodiments, any of the whole genome sequencing techniques disclosed in block 502 are used for the first sequencing reaction or the second sequencing reaction.

*Block 536.* Referring to block 536, a tumor base fraction (the percentage of tumor-derived DNA in the tumor sample; second sequencing reaction) of the candidate mutation is determined using the plurality of tumor sequence reads. In this sense, the tumor base fraction is the candidate mutation fraction from the second sequencing reaction (solid tumor sample of block 524). In typical embodiments, each tumor sequence read in the plurality of tumor sequence reads uniquely represents a nucleic fragment in the solid tumor sample. Thus, the tumor base fraction is determined by dividing the number of tumor sequence reads in the plurality of tumor sequence reads that have the candidate mutation by the total number of tumor sequence reads in the plurality of tumor sequence reads that map to the locus of the candidate mutation. The tumor base fraction represents the observed fraction of the candidate mutation at the locus of the candidate mutation in the tumor (second sequencing reaction), reflecting how much of the tumor's DNA contains the candidate mutation.

*Block 538.* Referring to block 538, in some embodiments, a reference base fraction of the candidate mutation is determined using the plurality of reference sequence reads. In this sense, the reference base fraction is the candidate mutation fraction from the first sequencing reaction (reference sample of block 502). In typical embodiments, each reference sequence read in the plurality of reference sequence reads uniquely represents a nucleic fragment in the reference sample. Thus, the reference base fraction is determined by dividing the number of reference sequence reads in the plurality of reference sequence reads that have the candidate mutation by the total number of reference sequence reads in the plurality of reference sequence reads that map to the locus of the candidate mutation. The reference base fraction represents the observed fraction of the candidate mutation at the locus of the candidate mutation in the reference (first sequencing reaction), reflecting how much of the reference sample DNA contains the candidate mutation.

*Block 540.* Referring to block 540, a germline expectation of the candidate mutation is determined using at least: (i) a tumor purity of the solid tumor sample, (ii) a major copy number at the genomic location of the candidate mutation derived from the plurality of tumor sequence reads, and (iii) a minor copy number at the genomic location of the candidate mutation derived from the plurality of tumor sequence reads. Accordingly, block 540 involves evaluating the tumor's genetic characteristics (purity, major copy number, minor copy number) to estimate whether the candidate mutation is more likely to be a germline mutation (inherited) or a somatic mutation (arising in the tumor). This is done by assessing the behavior of the mutation across different cell types (tumor vs. normal) and understanding its prevalence in the tumor cells.

Accordingly, in some embodiments the germline expectation is calculated by combining these three pieces of information from the tumor sample:
In some embodiments, the first model applies the first plurality of parameters to the first information through a plurality of computations and the plurality of computations is at least 10,000 computations. In some embodiments, the first plurality of parameters is at least 50, at least 100, at least 200, at least 1000, at least 10,000, at least 15,000, at least 50,000, at least 100,000, at least 250,000, at least 500,000, or at least 1,000,000 parameters, at least 2,500,000 parameters, at least 5,000,000 parameters, at least 10,000,000 parameters, or more. In some embodiments, the first model applies the first plurality of parameters to the first information through at least 1000 computation, at least 5000 computations, at least 10,000 computations, at least 25,000 computations, at least 50,000 computations, at least 100,000 computations, at least 250,000 computations, at least 500,000 computations, at least 1,000,000 computations, at least 2,500,000 computations, at least 5,000,000 computations, at least 10,000,000 computations, or more.

In some embodiments, the first model is trained against a training dataset including, for each sample in a plurality of samples (i) corresponding information for a corresponding variant and (ii) a corresponding indication of whether the corresponding variant is a true CHIP mutation or a true germline mutation based on evaluation of sequence reads from the sample. Such model training is described in Example 1 below.

*Tumor Purity.* Tumor purity helps to determine how much of the tumor sample is tumor tissue and how much is normal tissue. If the candidate mutation is present in both tumor and normal cells, it increases the likelihood that the mutation is germline (inherited). For this example, consider the case where the tumor purity is 80%. This means that 80% of the cells in the tumor sample are cancerous, and the remaining 20% are normal cells (e.g., stromal, immune, or blood cells).

*Major Copy Number.* Major copy (of the tumor sample) number helps to assess how many copies of the mutation exist in the tumor cells, which provides insight into whether the candidate mutation is likely somatic (from the tumor) or germline (inherited). The major copy number is the number of copies of the mutated allele in the tumor cells. For example, consider the case where, in the tumor cells, the candidate mutation is observed in four copies of the genomic location corresponding to the candidate mutation. This means that the tumor cells have an amplified copy of this region. If the candidate mutation were germline, it would be expected to be present in all cells (both tumor and normal cells), and the major copy number would likely be consistent across both the tumor and normal samples. A major copy number of 4 in only the tumor cells suggests a somatic mutation rather than a germline mutation.

To calculate the major copy number from the second sequencing reaction of block 524, the plurality of tumor sequence reads mapping to the region of interest (the locus of the candidate mutation) is obtained in accordance with block 524. These tumor sequence reads will contain information about the alleles present at the locus. Each tumor sequence read that maps to the candidate mutation locus, is classified as either reference (the allele without the mutation) or mutant (the allele containing the candidate mutation). The total number of tumor sequence reads bearing the reference allele and tumor sequence reads bearing the mutant allele is used to calculate the allelic fraction (the proportion of tumor sequence reads supporting each allele). Note that each of these tumor sequence reads maps to a unique nucleic acid fragment in the tumor sample. The allelic fraction provides the ratio of mutant to reference alleles. The major copy number, in some embodiments, is inferred by looking at the dominant allele based on this ratio. For example, if the allelic fraction of the mutant allele (relative to the reference) is higher, it suggests that the major allele is the mutant allele. Consider the case where 80 tumor sequence reads (representing 80 unique nucleic acid fragments in the tumor sample) align to the reference allele for the locus of the candidate mutation and 20 tumor sequence reads (representing 20 unique nucleic acid fragments in the tumor sample) align to the mutant allele for the locus of the candidate mutation. The allelic fraction is the proportion of mutant reads relative to total reads, or 20/100, or 0.2. In this example, if the reference allele is dominant in the sample, the major copy number may be inferred to be 4 (based on normal diploid inheritance plus the tumor's copy number variation), and the minor copy number may be inferred to be 2 or 1, depending on the tumor's copy number. In some embodiments the major and minor copy number can be refined using additional data, such as the read depth at the locus of the candidate mutation versus the read depth at other loci.

*Minor Copy Number.* Minor copy number (of the solid tumor sample) gives information about subclonal mutations. If the minor copy number is observed in both tumor and normal cells, this can indicate that the candidate mutation is germline. If it is only observed in the tumor, candidate mutation is more likely to be somatic. The minor copy number refers to the number of copies of the non-major allele (alternate allele) at the candidate mutation's genomic location, and in some instances represents a subclonal population of tumor cells. For instance, consider the case where the major copy number is four and the minor copy number is one. This would mean that the candidate mutation is present in a smaller portion of the tumor cells (perhaps a subpopulation of cells that harbor a slightly different variant).

The copy number in the reference sample is assumed to be diploid.

In some embodiments in accordance with block 540 the germline expectation (exp) is calculated as: $exp = \frac{\left(purity x t. copy number x t. m/m ratio\right) - \left(1-purity\right) x n . m / m ratio x 2}{t . copy number}$ where,
purity = the purity of the tumor expressed as a percentage,
t. copy number = the tumor copy number,
t. m/m ratio = the tumor major / minor ratio,
n. m/m ratio = the normal major / minor ratio.

Here, the tumor copy number of the candidate mutation in the tumor cells is the sum of the major and minor copy numbers in the solid tumor sample. The tumor major / minor ratio is the ratio of the major copy number to the minor copy number in the tumor sample. The normal major / minor ratio is the expected ratio of the major and minor alleles in the reference (normal) sample, assuming a diploid genome, meaning there are two copies of each allele (A/A or B/B or A/B for each locus). It is assumed that the copy number of the reference (normal) sample is diploid meaning that the normal major / minor ratio will be 1.

In typical embodiments of block 540, both A/B and B/A values are tested because both configurations of alleles for the candidate mutation are valid configurations. This is because, in diploid organisms, two alleles (denoted A and B here) are inherited at each locus: a maternal allele (A) and a paternal allele (B). The alleles can segregate randomly in different combinations. A/B and B/A refer to the two different possible arrangements of the alleles in a heterozygous individual. For example, if the mutation involves an A allele and a B allele, the copy numbers in the tumor might be expressed in either the A/B or B/A order. Since the alleles are inherited randomly, both A/B and B/A can be valid representations of the heterozygous state and thus, in typical embodiments of block 540, are both tested as potential configurations of the candidate mutation. This means that the germline expectation is calculated, in such embodiments, for both potential allele configurations, and the more probable one is chosen as the germline expectation based on the calculated values. Both A/B and B/A ratios are considered valid because of the random assortment of alleles.

For example, consider the case where tumor purity is 0.8 (80% of the tumor sample contains tumor cells), tumor copy number is 6 (total copies of the mutation in the tumor, combining major and minor copies), the tumor major/minor ratio is 2 (2 major copies for every minor copy), and the normal major minor ratio is 1 (since the reference sample is diploid, the ratio of major/minor alleles would be 1:1). Appling the above formula: $germline expectation = \frac{\left(0.8\times 6\times 2\right) + \left(1-0.8\right) \times 1 \times 2}{6}$ and thus tumor contribution: 0.8 × 6 × 2 = 9.6 while the normal (reference) contribution = (1-0.8) × 1 × 2 = 0.4. Combining these contributions gives: $germline expectation = \frac{9.6 + 0.4}{6} = \frac{10}{6} \approx 1.67$

In this case, the germline expectation is approximately 1.67, which suggests that the mutation has a relatively high likelihood of being germline (as opposed to somatic), since it's closer to 2, the expected value for a germline mutation (in the diploid reference sample). If the value was closer to 0, it would indicate a somatic mutation (arising in the tumor).

When calculating the germline expectation in two different configurations the A allele is considered the major allele while the B allele is considered the minor allele in configuration one whereas the A allele is considered the minor allele while the B allele is considered the major allele in configuration two.

*Block 541.* Referring to block 541, first information is inputted into a first model comprising a first plurality of parameters thereby obtaining, as output from the first model, through application of the first plurality of parameters to the first information, an indication of whether the candidate mutation is (a) a clonal hematopoiesis of indeterminate potential (CHIP) mutation or (b) a germline mutation. The first information comprises (i) the tumor base fraction for the candidate mutation and (ii) the germline expectation of the candidate mutation.

CHIP describes an expansion of hematopoietic stem cells that harbor somatic mutations without an underlying malignancy. CHIP has been identified through genomic profiling of peripheral blood from healthy individuals. See, Busque et al., 2012, "Recurrent somatic TET2 mutations in normal elderly individuals with clonal hematopoiesis," Nat Genet. 44(11), pp. 1179-1181, which is hereby incorporated by reference. Its incidence increases with age and has been detected in peripheral blood of patients with solid tumors. *See,* Xie et al.., 2014, "Age-related mutations associated with clonal hematopoietic expansion and malignancies," Nat Med. 20(12), pp, 1472-1478, which is hereby incorporated by reference. Hematopoietic cells permeate all tissues and are present in solid tumor specimens. See Severson et al., 2018, "Detection of clonal hematopoiesis of indeterminate potential in clinical sequencing of solid tumor specimens," Blood 131(22), pp. 2501-2502, which is hereby incorporated by reference.

The tumor base fraction represents the observed fraction of the candidate mutation at the locus of the candidate mutation in the tumor, reflecting how much of the tumor's DNA contains the candidate mutation. Germline mutations are inherited from one's parents and are present in all cells in the body, including both tumor and normal cells. The tumor base fraction for a germline mutation would likely be high and consistent in both the tumor and reference samples. If the germline expectation is close to 2 (since a normal diploid sample would have two copies of each gene) and the tumor base fraction is close to 1.0 (*e.g.,* the mutation is present in both alleles in tumor cells), then it suggests that the mutation is germline. CHIP mutations are somatic mutations that occur in the hematopoietic stem cells but are not always associated with malignancy. These mutations may not be present in all cells of the body (they are not germline), and typically only a subset of cells will carry the mutation. The tumor base fraction for a CHIP mutation would typically be lower than expected for a germline mutation, because the mutation is not present in all the tumor cells - just a subset of them. The tumor cells with the CHIP mutation may reflect the mutation's clonal expansion in the hematopoietic lineage, so the mutation would not be uniformly represented across all tumor cells. If the tumor base fraction is less than the expected fraction for a germline mutation, it suggests a somatic (*e.g.,* CHIP) origin. In particular, a lower tumor base fraction, especially in a heterogeneous tumor sample, can be a strong indicator of CHIP.

If the tumor base fraction is significantly lower than the germline expectation (*e.g.,* the mutation is present in only a subset of tumor cells), this suggests that the mutation is somatic and might be a CHIP mutation. This would indicate that the mutation is confined to a subset of hematopoietic cells and is not inherited, but rather arose during the development of the individual's blood system.

If the tumor base fraction is similar to or higher than the germline expectation, this would suggest that the mutation is likely germline, since the mutation is present in all cells (normal and tumor alike) at similar proportions.

The present disclosure provides improved insight on whether a given candidate mutation is CHIP versus germline by training the first model on the germline expectation and tumor base fraction values of a number of different CHIP and germline variants as discussed in the Example section below. In some embodiments the first model uses additional input such as the input discussed in blocks 542, 544, and/or 546 below.

*Block 542.* Referring to block 542, in some embodiments, the first information further comprises an absolute value of a difference between the reference base fraction for the candidate mutation and the germline expectation of the candidate mutation. The germline expectation gives an estimate of how the candidate mutation would behave if it were inherited (germline). In sequencing data, the reference base fraction is the frequency at which a particular allele (mutation or variant) appears at a specific genomic position. For example: in the buffy coat (germline / reference) sample, the allele frequency of the candidate mutation could be close to 0.5 for a heterozygous variant (*e.g.,* 50% of the alleles at that position are mutated, and 50% are wild-type). In the tumor sample, the allele frequency could be 0.0, 0.5, or 1.0, depending on whether the mutation is homozygous, heterozygous, or absent. The germline expectation for a mutation would be the expected allele fraction in the germline, based on whether the mutation is inherited in a heterozygous or homozygous state. For example, if the test subject is heterozygous in the germline (*e.g.,* A/B at the mutation site), the expected reference base fraction should be close to 0.5 (50% of the alleles are the reference, and 50% are the variant). If the individual is homozygous (*e.g.,* A/A or B/B), the expected reference base fraction will be closer to 1.0 or 0.0 (depending on the reference allele). In the case of heterozygosity in the germline, the germline expectation would be 0.5 for a heterozygous variant. Once the reference base fraction from the tumor or buffy coat has been determined in accordance with block 538, the difference between this value and the germline expectation of block 540 can be calculated. For example, consider the case in which the germline expectation is 0.5 (for a heterozygous site, A/B), the observed base fraction in the buffy coat (germline) is 0.5 (as expected, since it's heterozygous), and the observed base fraction in the tumor is 1.0 (homozygous for one allele, *e.g.,* A/A). The absolute difference: $Δ = \left|Observed tumor base fraction-Germline expectation\right|$ for the tumor sample is calculated as: $Δ = \left|1.0-0.5\right| = 0.5$ A difference of 0.0 indicates no change in heterozygosity, meaning the tumor retained the same heterozygous state as the germline (or remained homozygous if the germline was homozygous).

A difference of 0.5 suggests loss of heterozygosity (LOH), indicating that the tumor became homozygous at that locus (from heterozygous in the germline).

A difference of 0.5 can also indicate a gain in heterozygosity if the tumor was initially homozygous in the germline but became heterozygous due to a mutation (though this scenario is rarer in the context of cancer).

The absolute value of the difference ensures that the magnitude of the change in heterozygosity is captured, regardless of whether the change was due to a loss or gain.

*Block 544.* Referring to block 544, in some embodiments, the first information further comprises the reference base fraction of the candidate mutation. In some embodiments, the reference base fraction for the candidate mutation is determined by analyzing the normal (*e.g.,* buffy coat) sample. This is done by evaluating the base composition at the corresponding locus in the normal sample of block 502 to establish the expected fraction of the allele for the candidate mutation at that position. Specifically, the reference base fraction is determined by (i) counting how often the reference mutation is observed across the nucleic acid sequences of the normal sample (buffy coat) (*e.g.,* the plurality of reference sequence reads of the first sequencing reaction) and (ii) comparing this to the total number of reference sequence reads covering the same locus in the normal sample. This provides a baseline reference for the allele frequency of the candidate mutation in the normal (*e.g.,* buffy coat) sample. In such calculations, the reference sequence reads are deduplicated so that each reference sequence read uniquely represents a distinct nucleic acid fragment in the reference sample.

Thus, the reference base fraction represents the proportion of reference sequence reads at a given locus in the normal sample that correspond to the allele of the candidate mutation. For instance, the reference base fraction can be determined by dividing the number of times the allele of the candidate mutation is observed in the normal sample by the total number of reference sequence reads mapped to the locus of the candidate mutation in the normal sample, establishing the baseline reference allele fraction for that locus.

In some embodiments, this process involves the analysis of aligned reference sequence reads (*e.g.,* in SAM or BAM format) from the first sequencing reaction. A variant allele fraction module 151 may tally instances where each allele (including the allele of the candidate mutation) is represented in unique sequence reads encompassing the locus of the candidate mutation. These counts can then be used to compute the ratio of the allele of the candidate mutation to wildtype.

*Block 546.* Referring to block 546, in some embodiments, the first information further comprises the tumor purity of the solid tumor sample.

Tumor purity refers to the proportion of tumor cells within a sample, relative to the total number of cells in that sample, that may include normal cells, stromal cells, immune cells, and other non-tumor cells. There are many different methods used to determine tumor purity.

In some embodiments tumor purity is found in a histopathological assessment associated with the tumor sample of block 524. For a histopathological assessment (manual review), pathologists visually examine tissue samples under a microscope. They estimate the proportion of tumor cells relative to the normal cells in the sample. This method can be subjective and prone to error but gives an initial estimate based on the tissue morphology.

In some embodiments tumor purity is determined by the second sequencing reaction. Genomic sequencing can be used to analyze the DNA of a tumor sample. Tumor purity is estimated by looking at the allelic frequency of mutations in the sample. Tumor purity is inferred from the ratio of the mutant allele (cancer-related mutation) to the wild-type allele (non-mutated) across different loci (*e.g.,* the genes of Table 1 and/or 2). If the tumor has high purity, the mutant allele will be more abundant. Conversely, in low-purity tumors, normal (non-cancerous) cells may dilute the signal of the mutant alleles, leading to a lower frequency of mutations. In some embodiments tumor purity is determined by tools such as VarScan, MuTect, or FACETS based on the second sequencing reaction.

In some embodiments tumor purity is determined by microdissection of the solid tumor sample of the test subject of block 524. Laser capture microdissection (LCM) is a technique where specific areas of the tissue sample that contain tumor cells are isolated under a microscope. This minimizes contamination from non-tumor cells. After microdissection, genomic analysis can be done on just the tumor cells, allowing for a more accurate estimate of tumor purity.

In some embodiments tumor purity is determined by copy number variation (CNV) analysis using the second sequencing reaction of block 524. Tumors often exhibit specific patterns of chromosomal copy number variations (gains or losses of whole chromosomes or parts of chromosomes). By analyzing these CNVs across the genome, purity can be inferred. For instance, regions with significant losses or gains in tumor cells compared to normal cells can indicate the proportion of tumor versus normal cells.

In some embodiments tumor purity is determined by digital PCR and quantitative PCR (qPCR). These methods can target specific mutations or genes and measure their presence in the tumor sample. Tumor purity can be estimated by comparing the concentration of tumor-specific mutations to a reference gene or normal tissue.

In some embodiments tumor purity is determined by any of several bioinformatics tools that estimate tumor purity based on large-scale genomic data from the second sequencing reaction, using algorithms that account for different types of genetic variations and mutation patterns. Such tools can process the second sequencing reaction data and generate estimates of tumor purity by analyzing the depth of sequencing coverage, allele frequencies, and copy number alterations across the genome.

*Block 548.* Referring to block 548, in some embodiments, a determination is made as to whether the reference base fraction for the candidate mutation satisfies a first threshold. When the reference base fraction for the candidate mutation satisfies the first threshold, the candidate mutation is deemed to be (a) the CHIP mutation or (b) the germline mutation in accordance with the first model. When the reference base fraction for the candidate mutation fails to satisfy the first threshold, the candidate mutation is deemed to be (a) the CHIP mutation or (b) an artifact in accordance with a second model.

Thus, in some such embodiments, the first model classifies candidate mutations that satisfy the first threshold (*e.g.,* above 20% VAF) in the reference (*e.g.* buffy coat, saliva) sample of block 502 as CHIP versus germline. Use of an example first model to classify candidate mutations satisfying the first threshold as CHIP versus germline is illustrated in Example 1. Moreover, in such embodiments, a second model evaluates candidate mutations that fail to satisfy the first threshold (*e.g.,* have a VAF of between 2% and 20% in the reference sample) to classify the candidate mutation as CHIP versus artifact. Use of an example second model to classify candidate mutations failing to satisfy the first threshold as CHIP versus artifact is illustrated in Example 2.

*Block 550.* Referring to block 550, in some embodiments, the reference base fraction for the candidate mutation satisfies the first threshold when the reference base fraction exceeds twenty percent. In some embodiments, the reference base fraction for the candidate mutation satisfies the first threshold when the reference base fraction exceeds 15 percent, 16 percent, 17, percent, 18 percent, 19 percent, 20 percent, 21 percent, 22 percent, 23 percent, 24 percent, 25 percent, 30 percent, 35 percent, or 40 percent. In some embodiments, the reference base fraction for the candidate mutation satisfies the first threshold when the reference base fraction is between 15 percent and 100 percent, between 18 percent and 99 percent, between 20 percent and 100 percent, between 20 percent and 99 percent, between 20 percent and 80 percent, or between 20 percent and 70 percent.

*Block 552.* Referring to block 552, in some embodiments, second information is inputted into a second model comprising a second plurality of parameters thereby obtaining as output from the second model, through application of the second plurality of parameters to the second information, an indication of whether the candidate mutation is (a) the CHIP mutation or (b) the artifact. An example of this is illustrated in Example 2. The second information comprises at least two of (i) a total number of reference sequence reads in the plurality of reference sequence reads mapping to the locus of the candidate mutation, (ii) a sequencing depth of the first sequencing reaction at the locus of the candidate mutation, and (iii) an indication of whether the candidate mutation satisfied a minimum variant fraction or a minimum insertion or deletion test. The minimum variant fraction refers to the lowest allele frequency at which a variant (SNV or indel) is reported by a variant caller such as VarDict. See, for example, VarDict documentation, available on the internet at github.com/AstraZeneca-NGS/VarDictJava. In some the candidate mutation satisfies the minimum variant fraction or a minimum insertion or deletion test when the candidate mutation has a variant allele frequency of at least 0.25% in the reference sample. In some the candidate mutation satisfies the minimum variant fraction or a minimum insertion or deletion test when the candidate mutation has a variant allele frequency of at least 0.35% in the reference sample. In some the candidate mutation satisfies the minimum variant fraction or a minimum insertion or deletion test when the candidate mutation has a variant allele frequency of at least 0.1%, 0.15%, 0.20%, 0.25%, 0.30%, 0.35%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, or 2.5% in the reference sample.

In some embodiments, the second model applies the second plurality of parameters to the second information through a plurality of computations and the plurality of computations is at least 10,000 computations. In some embodiments, the second plurality of parameters is at least 50, at least 100, at least 200, at least 1000, at least 10,000, at least 15,000, at least 50,000, at least 100,000, at least 250,000, at least 500,000, or at least 1,000,000 parameters, at least 2,500,000 parameters, at least 5,000,000 parameters, at least 10,000,000 parameters, or more. In some embodiments, the second model applies the second plurality of parameters to the second information through at least 1000 computation, at least 5000 computations, at least 10,000 computations, at least 25,000 computations, at least 50,000 computations, at least 100,000 computations, at least 250,000 computations, at least 500,000 computations, at least 1,000,000 computations, at least 2,500,000 computations, at least 5,000,000 computations, at least 10,000,000 computations, or more.

In some embodiments, the second model is trained against a training dataset including, for each respective sample in a plurality of samples, (i) corresponding information for a corresponding variant and (ii) a corresponding indication of whether the corresponding variant is a true CHIP or an artifact based on evaluation of sequence reads mapping to the corresponding variant in the sample. Such model training is described in the Example 2 below.

*Block 554.* Referring to block 554, in some embodiments, the second information further comprises the reference base fraction of the candidate mutation. The reference base fraction is discussed, for example, in block 538 above.

*Block 556.* Referring to block 556, in some embodiments, the first or second information further comprises an indication as to whether or not the candidate mutation is in DNMT3A, STAG2, TET2, ASXL1, JAK2, SF3B1, PPM1d, EGFR, or KMT2C.

In some embodiments, the first or second information further comprises an indication as to whether or not the candidate mutation is in a gene listed in Table 3. In some embodiments the first or second information comprises an indication for each of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, or all of the respective genes listed in Table 3 as to whether or not the mutation is in the respective gene.

**Table 3. Example genes known to have variants in clinical examples of clonal hematopoiesis of indeterminate potential (CHIP).**

| **Table 3: CHIP Genes** | | | | |
|---|---|---|---|---|
| CSF3R | IDH2 | CALR | U2AF1 | JAK2 |
| MPL | CREBBP | CEBPA | MYD88 | NOTCH1 |
| NRAS | TP53 | DNMT3A | RHOA | PIGA |
| WT1 | STAT5B | CXCR4 | KIT | ZRSR2 |
| ATM | STAT3 | SF3B1 | TET2 | BCOR |
| CBL | PPM1D | IDH1 | TERT | STAG2 |
| PTPN11 | SRSF2 | ASXL1 | NPM1 | BCORL1 |
| FLT3 | SETBP1 | RUNX1 | EZH2 | SF3B1 |
| PPM1D | EGFR | KMT2C | | |

*Block 558.* Referring to block 558, in some embodiments, the first or second information further comprises a length of the candidate mutation.

In some embodiments, the length of the candidate mutation is represented by a fragment length metric that is a measure of the distribution of the lengths of all sequenced cfDNA fragments encompassing the candidate mutation, *e.g.,* a measure of central tendency, a characterization of the shape of the distribution of the lengths of all sequenced cfDNA fragments encompassing the candidate mutation.

In some embodiments, the length of the candidate mutation is represented by a fragment length metric that is a measure of a difference between the distribution of the lengths of all sequenced cfDNA fragments encompassing the candidate mutation and a distribution of lengths of sequenced cfDNA fragments that map to the locus of the candidate mutation but do not contain the candidate mutation.

In some embodiments, the length of the candidate mutation is represented by a fragment length metric that is a measure of central tendency of the set of sequenced cfDNA fragments that contain the candidate mutation. In some embodiments, the length of the candidate mutation is represented by a fragment length metric that is a mean of the lengths of all sequenced cfDNA fragments containing the candidate mutation.

In some embodiments, the length of the candidate mutation is represented by a fragment length metric that is a kurtosis of the set of sequenced cfDNA fragments that contain the candidate mutation. Kurtosis is a measure of the "tailedness" of the probability distribution of a real-valued random variable. A description of determining kurtosis is found, for example, in Joanes and Gill, 1998, "Comparing measures of sample skewness and kurtosis," Journal of the Royal Statistical Society, Series D, 47 (1): 1831-89, the content of which is incorporated herein by reference in its entirety for all purposes.

In some embodiments, the length of the candidate mutation is represented by a fragment length metric that is a skew of the set of sequenced cfDNA fragments that contain the candidate mutation. Skewness is a measure of the asymmetry of the probability distribution of a real-valued random variable about its mean. A description of determining skew is found, for example, in Joanes and Gill, 1998, "Comparing measures of sample skewness and kurtosis," Journal of the Royal Statistical Society, Series D, 47 (1): 183-189, the content of which is incorporated herein by reference in its entirety for all purposes.

In some embodiments, the length of the candidate mutation is represented by a fragment length metric that is a p-value determined by application of a Kolmogorov-Smirnov test to a difference in (i) a distribution of fragment lengths of the set of sequenced cfDNA fragments that contain the candidate mutation versus (ii) a distribution of fragment lengths of a second set of sequenced cfDNA fragments in the plurality of sequenced cfDNA fragments that do not contain the candidate mutation In some such embodiments, the second set of sequenced cfDNA fragments is the sequenced cfDNA fragments in the plurality of sequenced cfDNA fragments from the biological sample, other than the set of cfDNA fragments, that include the locus of the candidate mutation.

*Block 560.* Referring to block 560, in some embodiments, the first or second information further comprises a length of a wildtype allele corresponding to the candidate mutation.

In some embodiments, the length of the wildtype allele corresponding to the candidate mutation is represented by a fragment length metric that is a measure of the distribution of the lengths of all sequenced cfDNA fragments encompassing mapping to the locus of the candidate mutation that are wild type at the locus of the candidate mutation, *e.g.,* a measure of central tendency, a characterization of the shape of the distribution of the lengths of all sequenced cfDNA fragments that are wild type at the locus of the candidate mutation.

In some embodiments, the length of the wildtype allele corresponding to the candidate mutation is represented by a fragment length metric that is a measure of a difference between (i) the distribution of the lengths of all sequenced cfDNA fragments that map to the locus of the candidate mutation but do not contain the candidate mutation and (ii) the distribution of the lengths of all sequenced cfDNA fragments encompassing the candidate mutation.

In some embodiments, the length of the wildtype allele corresponding to the candidate mutation is represented by a fragment length metric that is a measure of central tendency of the set of sequenced cfDNA fragments that map to the locus of the candidate mutation but are wildtype at the locus of the candidate mutation. In some embodiments, the length of the wildtype allele is represented by a fragment length metric that is a mean of the lengths of all sequenced cfDNA fragments that map to the locus of the candidate mutation but that are wildtype at the locus of the candidate mutation.

In some embodiments, the length of the wildtype allele corresponding to the candidate mutation is represented by a fragment length metric that is a kurtosis of the set of sequenced cfDNA fragments that map to the locus of the candidate mutation but are wildtype at the locus of the candidate mutation. Kurtosis is a measure of the "tailedness" of the probability distribution of a real-valued random variable. A description of determining kurtosis is found, for example, in Joanes and Gill, 1998, "Comparing measures of sample skewness and kurtosis," Journal of the Royal Statistical Society, Series D, 47 (1): 1831-89, the content of which is incorporated herein by reference in its entirety for all purposes.

In some embodiments, the length of the wildtype allele corresponding to the candidate mutation is represented by a fragment length metric that is a skew of the set of sequenced cfDNA fragments that map to the locus of the candidate mutation but are wildtype at the locus of the candidate mutation. Skewness is a measure of the asymmetry of the probability distribution of a real-valued random variable about its mean. A description of determining skew is found, for example, in Joanes and Gill, 1998, "Comparing measures of sample skewness and kurtosis," Journal of the Royal Statistical Society, Series D, 47 (1): 183-189, the content of which is incorporated herein by reference in its entirety for all purposes.

In some embodiments, the length of the wildtype allele corresponding to the candidate mutation is represented by a fragment length metric that is a p-value determined by application of a Kolmogorov-Smirnov test to a difference in (i) a distribution of fragment lengths of the set of sequenced cfDNA fragments that map to the locus of the candidate mutation but are wildtype at the locus of the candidate mutation versus (ii) a distribution of fragment lengths of a second set of sequenced cfDNA fragments in the plurality of sequenced cfDNA fragments obtained from the biological sample that contain the candidate mutation.

*Block 562.* Referring to block 562, in some embodiments, the first or second information further comprises a binary indication of whether or not the candidate mutation is in a curated list of known CHIP mutations. For instance, in some embodiments, the curated list of known CHIP mutations include mutations in 1, 2, 3, 4, 5, or all the following genes: TET2, DNMT3A, ASXL1, SF3B1, CBL, U2AF1, IDH2,2,3, MYD88,13, EP300, CDKN2C, and HNF1A when the subject is 70 years of age or older. Such genes are known CHIP genes in subjects of this age. *See,* Severson, 2018, "Detection of clonal hematopoiesis of indeterminate potential in clinical sequencing of solid tumor specimens," Blood 131(22), pp. 2501-2505, which is hereby incorporated by reference.

In some embodiments, the curated list of known CHIP mutations include mutations in 1, 2, 3, 4, 5, or all the following genes: TET2, DNMT3A, ASXL1, SF3B1, CBL, U2AF1, IDH2,2,3, MYD88,13, EP300, CDKN2C, and HNF1A.

In some embodiments, the curated list of known CHIP mutations include mutations in 1, 2, 3, or all the following genes: TET2, DNMT3A, ASXL1, and SF3B1.

In some embodiments, the curated list of known CHIP mutations include mutations in 1, 2, 3, 4, 5, 6, 7, 8, 9, 10 or all the following genes: ASXL1, BCOR, BCORL1, CBL, CREBBP, CUX1, DNMT3A, GNB1, JAK2, PPM1D, PRPF8, SETDB1, SF3B1, SRSF2, TET2, and U2AF. In some embodiments, mutations in this set of genes, or any subset thereof, is used for the curated list of known CHIP mutations when the cancer condition of the test subject is a BRCA-associated cancer. See Marshall et al., "Germline mutations and the presence of CHIP in 20,963 patients with BRCA-associated cancers.," DOI: 10.1200/JCO.2023.41.16_suppl.10522 Journal of Clinical Oncology 41, no. 16_suppl (June 01, 2023) 10522-10522, which is hereby incorporated by reference.

*Block 564.* Referring to block 564, in some embodiments, the first or second information further comprises a binary indication as to whether or not the candidate mutation is in a CHIP driver gene.. In some embodiments the CHIP driver genes are DNMT3A, TET2, ASXL1, JAK2, SF3B1, and PPM1D. In some embodiments the CHIP driver genes are DNMT3A, TET2, ASXL1, JAK2, SF3B1, PPM1D, CBL, and KMT2C. In some embodiments the CHIP driver genes are DNMT3A and TET2. In some embodiments the CHIP driver genes are DNMT3A, TET2, and ASXL1.

*Block 566.* Referring to block 566, in some embodiments, the first or second information further comprises a COSMIC solid tumor frequency of the candidate mutation. The "COSMIC solid tumor frequency" of a candidate mutation is the variant allele frequency of the candidate mutation in those subjects in the Catalog of Somatic Mutations in Cancer (COSMIC) database that have solid tumors. See Tate et al, 2019, "COSMIC: The catalogue of somatic mutations in cancer," Nucleic Acids Res 47, D941-D947, which is hereby incorporated by reference. Thus, in such embodiments that make use of the feature "COSMIC solid tumor frequency," the information inputted into the first model or second model includes the variant allele frequency of the candidate mutation in subjects having solid tumors that are in the COSMIC database. In some embodiments, the frequency of the candidate mutation in solid tumors is calculated by summing all instances of the candidate mutation found in non-other and nonhematopoietic and lymphoid tissues in the COSMIC database and dividing by the total number of instances of the candidate mutation found in non-other tissues.

*Blocks 568 - 570.* Referring to block 568, in some embodiments, the first or second information further comprises a score for the candidate mutation using any one of single-base substitution signatures SBS1 - SBS60. Referring to block 570, in some embodiments, the first or second information further comprises a score for the candidate mutation using any one of single-base substitution signatures in the group consisting of SBS55, SBS2, SBS35, SBS7a, SBS54, SBS8, SBS6, SBS44, SBS10a, SBS59, SBS25, SBS21, SBS19, SBS30, SBS10b, SBS7b, SBS11, SBS32, SBS17b, SBS26, SBS4, and SBS33. SBS1 through SBS60, including SBS55, SBS2, SBS35, SBS7a, SBS54, SBS8, SBS6, SBS44, SBS10a, SBS59, SBS25, SBS21, SBS19, SBS30, SBS10b, SBS7b, SBS11, SBS32, SBS17b, SBS26, SBS4, and SBS33, each represent a specific pattern of single-base substitutions that can be linked to different biological processes, mutational causes, or environmental factors (*e.g.,* UV light, smoking, or DNA repair deficiencies). While there are 96 possible SBS mutation types based on the nucleotide change and the two surrounding bases for a candidate somatic variation that is a single nucleotide polymorphism, they are grouped into 60 distinct mutational patterns for analysis purposes. *See* Alexandrov et al., 2020, "The repertoire of mutational signatures in human cancer," Nature 578, 94-101, which is hereby incorporated by reference. If one of these features is used, the score for the candidate mutation for this feature is included in the information inputted into the first model. For instance, if the "SBS55" feature is used, the score for the candidate mutation against the SBS55 single-base substitution signature is inputted into the first model or second model. See Fairchild et al., 2023, "Clonal hematopoiesis detection in patients with cancer using cell-free DNA sequencing," Science Translational Medicine 15, eabm8729 for methods for calculating such a score.

*Block 572.* Referring to block 572, in some embodiments, the first or second information further comprises an ExAC frequency of the candidate mutation. In some embodiments, the "ExAC frequency" feature of a candidate mutation is calculated as the variant allele frequency of the candidate mutation in the Exome Aggregation Consortium Database (ExAC). *See* Karczewski et al, 2017, "The ExAC browser: Displaying reference data information form over 60,000 exomes," Nucleic Acids Res 45, D840-D845, which is hereby incorporated by reference. Thus, in such embodiments that make use of the feature "ExAC frequency," the information inputted into the first or second model includes the variant allele frequency of the candidate mutation in the ExAC database.

In some embodiments, any 1, 2, 3, 4, 5, 6, 7, 8, 9, 10, 11, 12, 13, 14, 15, 16, 17, 18, 19, 20, 21, 22, 23, 24, 25 or more features from Table 6 of United States Patent Application No. 18/969,159, entitled "Systems and Methods for Detecting Somatic Variants Derived from Circulating Tumor Nucleic Acids," filed on December 4, 2024, which is hereby incorporated by reference, is used in addition to any combination of features disclosed herein as information about a candidate mutation that is inputted into the first or second model. In some embodiments, any feature of Table 6 is used in place of an existing feature in any combination of features disclosed herein, *e.g.,* it is swapped in to replace a different feature in the information about a candidate mutation that is inputted into the first or second model. In some embodiments, any combination of features of Table 6 is used in combination with any other feature or features described herein in the information about a candidate mutation that is inputted into the first or second model.

In some embodiments, the first or second information further comprises one or more clonal hematopoiesis prevalence metrics. In some embodiments, a clonal hematopoiesis prevalence metric (*e.g.,* CHIP likelihood of Example 3 of United States Patent Application No. 18/969,159, entitled "Systems and Methods for Detecting Somatic Variants Derived from Circulating Tumor Nucleic Acids," filed on December 4, 2024) is determined for the candidate mutation based on sequencing results for a cohort of solid tumor samples with matched liquid biopsy samples. The metric is calculated as the total number of occurrences of the candidate mutation in the solid tumor samples that were classified as somatic divided by the total number of occurrences of the candidate mutation in the solid tumor samples that were classified as either somatic or non-somatic (*e.g.,* either of germline or CHIP lineage).

In some embodiments, a clonal hematopoiesis prevalence metric in the one or more clonal hematopoiesis prevalence metrics is a comparison of (i) instances of the candidate mutation, in a cohort of solid tumors, that are of hematopoietic or germline lineage and (ii) total instances of the candidate mutation in the cohort of solid tumors, or a comparison of (i) instances of the candidate mutation, in the cohort of solid tumors, that are of somatic lineage and (ii) total instances of the candidate mutation in the cohort of solid tumors.

In some embodiments, the first or second information uses any 1, 2, 3, 4, 5, 6, 7, 8, 9, or 10 features associated with a candidate mutation that is being evaluated (to validate whether it is (a) a CHIP mutation or (b) germ line mutation (or alternatively in the case of the second information whether it is a CHIP mutation or artifact) from the group consisting of gene frequency, alternate allele fragment length median, CHIP likelihood, Kolmogorov-Smirnov test metric for fragment length, p-value for Kolmogorov-Smirnov test metric for fragment length, alternate allele fragment length kurtosis, estimated circulating tumor fraction, ensemble variant allele fraction residual, alternate allele fragment length skew, and variant allele fraction, where such features are further described in Examples 3 and 4 of United States Patent Application No. 18/969,159, entitled "Systems and Methods for Detecting Somatic Variants Derived from Circulating Tumor Nucleic Acids," filed on December 4, 2024, which is hereby incorporated by reference.

In some embodiments the first or second information includes any of the following features of a candidate mutation to classify the candidate mutation: VAF, polymorphism length, reference and alternate allele fragment length distribution statistics, and historical variant chip likelihood.

In some embodiments the first or second information inputted includes a fragment length feature representing the frequency that cfDNA fragments encompassing the candidate mutation have fragment lengths more frequently associated with non-somatic sequence variants than somatic sequence variants. For example, Marass et al., 2020, Clin Chem., 66(4):616-18, the disclosure of which is incorporated herein by reference, reports that cfDNA fragments containing a CHIP variant are more likely to have a length between 173-191 bp or between 346-361 bp than other lengths, while cfDNA fragments containing a somatic variant are more likely to have a length between 127-141 bp or between 272-292 bp than other lengths. In some embodiments, this feature is used in addition to any combination of features disclosed herein in the first or second information. In some embodiments, this feature is used in place of an existing feature in any combination of features disclosed herein for the first or second information, *e.g.,* it is swapped in to replace a different feature in the first or second information. In some embodiments, this feature is used in combination with any other feature or features described herein in the first or second information (inputted into the first mode or second model).

Accordingly, in some embodiments, such a feature is a comparison of (i) the number of cfDNA fragments encompassing a candidate mutation with fragment lengths falling within a range of fragment lengths enriched for CHIP variants and (ii) the total number of cfDNA fragments encompassing the respective candidate mutation. However, the comparison can also be determined against other related measurements. For example, in some embodiments, such a feature is a comparison of (i) the number of cfDNA fragments encompassing a respective candidate mutation with fragment lengths falling within a range of fragments lengths enriched for CHIP variants and (ii) the total number of cfDNA fragments encompassing the respective candidate mutation having fragment lengths that either (a) fall within a range of fragment lengths enriched for CHIP variants or (b) fall within a range of fragment lengths enriched for somatic (*e.g.,* germline) variants. In yet other embodiments, such a feature is a comparison of (i) the number of cfDNA fragments encompassing the candidate mutation with fragment lengths falling within a range of fragment lengths enriched for CHIP variants and (ii) the number of cfDNA fragments encompassing a candidate mutation with fragment lengths falling within a range of fragment lengths enriched for somatic variants. In some embodiments, any of the comparisons described above can be made with respect to a number of cfDNA fragments encompassing a candidate mutation with fragment lengths falling within a range of fragment lengths enriched for non-somatic variants, rather than just for CHIP variants.

In some embodiments, the first or second information includes a historical prevalence feature representing the comparison of the frequency at which the candidate mutation is identified in cfDNA of cancer patients relative to the frequency at which the candidate mutation is identified in solid tumor samples. This feature is based on the observation that some CHIP variants are identified more frequently in cfDNA than in DNA from solid tumors. Without intending to be limited to any particular theory, this observation may be based on the rationale that cfDNA should contain a higher frequency of nucleic acids originating from cells of hematopoietic lineage than DNA extracted from solid tumors. Accordingly, a candidate mutation that is more frequently identified in cfDNA than in DNA from solid tumors should have a higher likelihood of having a hematopoietic lineage than a candidate mutation that is not more frequently identified in cfDNA than in DNA from solid tumors. In some embodiments, this feature is used in addition to any combination of features disclosed herein for the first or second information (that is inputted into the first or second model). In some embodiments, this feature is used in place of an existing feature in any combination of features disclosed herein, *e.g.,* it is swapped in to replace a different feature in the first or second information (that is inputted into the first model or second model). In some embodiments, this feature is used in combination with any other feature or features described herein in the first or information (that is inputted into the first or second model).

In some embodiments, the first or second information uses a historical prevalence feature representing how uniformly a candidate mutation is detected across a range of cancer types. Such a feature is based on the observation that some sequence variants occur more frequently in one or more cancer types than in other cancer types and thus, without being limited to any particular theory, may be drivers of those specific cancer types and, therefore, more likely to be a somatic variant. In contrast, variants that occur at a consistent frequency across many types of cancers are more likely to be passenger mutations without a somatic lineage. In some embodiments, this feature is used in addition to any combination of features disclosed herein in the first or second information (that is inputted into the first model or second model). In some embodiments, this feature is used in place of an existing feature in any combination of features disclosed herein, *e.g.,* it is swapped in to replace a different feature in the first or second information (that is inputted into the first model or second model). In some embodiments, this feature is used in combination with any other feature or features described herein in the first or second information (that is inputted into the first or second model).

In some embodiments, the first or second information includes a feature representing whether a frequency of identifying the candidate mutation in samples, *e.g.,* liquid biopsy samples, from a subject is stable over time. For example, in some such embodiments, the feature is a representation of the amount of change in the variant allele frequency of the candidate mutation in biological samples from the test subject over time. In some embodiments, the change in frequency is normalized against the measure of the amount of tumor DNA in the sample over time, *e.g.,* against a circulating tumor fraction estimate for the sample(s). In some embodiments, the change in frequency is alternatively, or additionally, normalized against relative changes in the frequency of other sequence variants over time. For example, it has been observed that the prevalence of some somatic variants in cfDNA should increase over time when the circulating tumor fraction is increasing and that the prevalence of non-somatic variants should decrease over time when the circulating tumor fraction is decreasing over time. In some embodiments, this feature is used in addition to any combination of features disclosed herein in the first or second information (that is inputted into the first model). In some embodiments, this feature is used in place of an existing feature in any combination of features disclosed herein, *e.g.,* it is swapped in to replace a different feature in the first or second information (that is inputted into the first model or second model). In some embodiments, this feature is used in combination with any other feature or features described herein in the first or second information (that is inputted into the first or second model).

*Blocks 574 - 576.* Referring to block 574, in some embodiments, the first model or the second model is selected from the group consisting of a regression model, a neural network, a support vector machine, a Naive Bayes model, a nearest neighbor model, a boosted trees model, a random forest model, a decision tree, a gradient boosted tree, an elastic net, a logistic regression model, and a clustering model. Referring to block 576, in some embodiments, the first model or the second model is a random forest model. Referring to block 578, in some embodiments, the first model or the second model is an XGboost model.

As used herein, the term "model" refers to a machine learning model, algorithm, or task. In some embodiments, a model is an unsupervised learning algorithm. One example of an unsupervised learning algorithm is cluster analysis. In some embodiments, a model is supervised machine learning. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, GradientBoosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network (*e.g.,* a deep-and-wide sample-level classifier). In some embodiments, a model is utilized to normalize a value or data set, such as by transforming the value or a set of values to a common frame of reference for comparison purposes.

In some embodiments, an untrained model (*e.g.,* "untrained classifier" and/or "untrained neural network") includes a machine learning model or algorithm, such as a classifier or a neural network, that has not been trained on a target dataset. In some embodiments, training a model (*e.g.,* training a neural network) refers to the process of training an untrained or partially trained model (*e.g.,* an untrained or partially trained neural network). For instance, consider the case of a plurality of training samples comprising a corresponding plurality of medical images (*e.g.,* of a medical dataset). The plurality of medical images is applied as collective input to an untrained or partially trained model, in conjunction with a corresponding measured indication of one or more features for each respective medical image (hereinafter training dataset) to train the untrained or partially trained model on indications that identify features related to morphological classes, thereby obtaining a trained model. Moreover, it will be appreciated that the term "untrained model" does not exclude the possibility that transfer learning techniques are used in such training of the untrained or partially trained model. For instance, Fernandes et al., 2017, "Transfer Learning with Partial Observability Applied to Cervical Cancer Screening," Pattern Recognition and Image Analysis: 8th Iberian Conference Proceedings, 243-250, which is hereby incorporated by reference in its entirety for all purposes, provides non-limiting examples of such transfer learning. In instances where transfer learning is used, the untrained model described above is provided with additional data over and beyond that of the primary training dataset. That is, in non-limiting examples of transfer learning embodiments, the untrained model receives (i) the plurality of images and the measured indications for each respective image ("primary training dataset") and (ii) additional data. In some embodiments, this additional data is in the form of parameters (*e.g.,* coefficients, weights, and/or hyperparameters) that were from another, auxiliary training dataset. Moreover, while a description of a single auxiliary training dataset has been disclosed, it will be appreciated that there is no limit on the number of auxiliary training datasets that may be used to complement the primary training dataset in training the untrained model in the present disclosure. For instance, in some embodiments, two or more auxiliary training datasets, three or more auxiliary training datasets, four or more auxiliary training datasets or five or more auxiliary training datasets are used to complement the primary training dataset through transfer learning, where each such auxiliary dataset is different than the primary training dataset. Any manner of transfer learning may be used in such embodiments. For instance, consider the case where there is a first auxiliary training dataset and a second auxiliary training dataset in addition to the primary training dataset. The parameters learned from the first auxiliary training dataset (by application of a first model to the first auxiliary training dataset) may be applied to the second auxiliary training dataset using transfer learning techniques (*e.g.,* a second model that is the same or different from the first model), which in turn may result in a trained intermediate model whose parameters are then applied to the primary training dataset and this, in conjunction with the primary training dataset itself, is applied to the untrained model. Alternatively, a first set of parameters learned from the first auxiliary training dataset (by application of a first model to the first auxiliary training dataset) and a second set of parameters learned from the second auxiliary training dataset (by application of a second model that is the same or different from the first model to the second auxiliary training dataset) may each individually be applied to a separate instance of the primary training dataset (*e.g.,* by separate independent matrix multiplications) and both such applications of the parameters to separate instances of the primary training dataset in conjunction with the primary training dataset itself (or some reduced form of the primary training dataset such as principal components or regression coefficients learned from the primary training set) may then be applied to the untrained model in order to train the untrained model. In some instances, additionally or alternatively, knowledge regarding objects related to morphological classes derived from an auxiliary training dataset is used, in conjunction with the object and/or class-labeled images in the primary training dataset, to train the untrained model.

*Support vector machines.* In some embodiments, the first and/or second model is a support vector machine (SVM). SVM algorithms suitable for use as models are described in, for example, Cristianini and Shawe-Taylor, 2000, An Introduction to Support Vector Machines, Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety for all purposes. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space can correspond to a non-linear decision boundary in the input space. In some embodiments, the plurality of parameters (*e.g.,* weights) associated with the SVM define the hyper-plane. In some embodiments, the hyper-plane is defined by at least 10, at least 20, at least 50, or at least 100 parameters and the SVM model requires a computer to calculate because it cannot be mentally solved.

*Naïve Bayes algorithms.* In some embodiments, the first and/or second model is a Naive Bayes algorithm. Naïve Bayes classifiers suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference in its entirety for all purposes. A Naive Bayes classifier is any classifier in a family of "probabilistic classifiers" based on applying Bayes' theorem with strong (naïve) independence assumptions between the features. In some embodiments, they are coupled with Kernel density estimation. *See,* for example, Hastie et al., 2001, The elements of statistical learning : data mining, inference, and prediction, eds. Tibshirani and Friedman, Springer, New York, which is hereby incorporated by reference in its entirety for all purposes.

*Nearest neighbor algorithms.* In some embodiments, the first and/or second model is a nearest neighbor algorithm. Nearest neighbor models can be memory-based and include no model to be fit. For nearest neighbors, given a query point x₀ (a first image), the k training points x_{(*r*)}, r, . . ., k (here the training images) closest in distance to x₀ are identified and then the point x₀ is classified using the k nearest neighbors. In some embodiments, the distance to these neighbors is a function of the values of a discriminating set. In some embodiments, Euclidean distance in feature space is used to determine distance as *d*_{(*i*)} = ∥*x*_{(*i*)} - x_{(*O*)}∥. In some embodiments, when the nearest neighbor algorithm is used, the value data used to compute the linear discriminant is standardized to have mean zero and variance 1. The nearest neighbor rule can be refined to address issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, New York, each of which is hereby incorporated by reference in its entirety for all purposes.

A k-nearest neighbor model is a non-parametric machine learning method in which the input consists of the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. *See,* Duda et al., 2001, Pattern Classification, Second Edition, John Wiley & Sons, which is hereby incorporated by reference in its entirety for all purposes. In some embodiments, the number of distance calculations needed to solve the k-nearest neighbor model is such that a computer is used to solve the model for a given input because it cannot be mentally performed.

*Random forest, decision tree, and boosted tree algorithms.* In some embodiments, the first and/or second model is a decision tree. Decision trees suitable for use as models are described generally by Duda, 2001, Pattern Classification, John Wiley & Sons, Inc., New York, pp. 395-396, which is hereby incorporated by reference in its entirety for all purposes. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference in its entirety for all purposes. CART, MART, and C4.5 are described in Hastie et al., 2001, The Elements of Statistical Learning, Springer-Verlag, New York, Chapter 9, which is hereby incorporated by reference in its entirety for all purposes. Random Forests are described in Breiman, 1999, "Random Forests--Random Features," Technical Report 567, Statistics Department, U.C. Berkeley, September 1999, which is hereby incorporated by reference in its entirety for all purposes. In some embodiments, the decision tree model includes at least 10, at least 20, at least 50, or at least 100 parameters (*e.g.,* weights and/or decisions) and requires a computer to calculate because it cannot be mentally solved.

*Linear discriminant analysis algorithms.* In some embodiments the first and/or second model is linear discriminant analysis (LDA), normal discriminant analysis (NDA), or discriminant function analysis that finds a linear combination of features that characterizes or separates two or more classes of objects or events. The resulting combination can be used as the model (*e.g.,* a linear classifier) in some embodiments of the present disclosure.

*Mixture model and Hidden Markov model.* In some embodiments, the first and/or second model is a mixture model, such as that described in McLachlan et al., Bioinformatics 18(3):413-422, 2002. In some embodiments, in particular, those embodiments including a temporal component, the model is a hidden Markov model such as described by Schliep et al., 2003, Bioinformatics 19(1):i255-i263.

*Clustering.* In some embodiments, the first and/or second model is an unsupervised clustering model. In some embodiments, the model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter, "Duda 1973") which is hereby incorporated by reference in its entirety for all purposes. The clustering problem can be described as one of finding natural groupings in a dataset. To identify natural groupings, two issues can be addressed. First, a way to measure similarity (or dissimilarity) between two samples can be determined. This metric (*e.g.,* similarity measure) can be used to ensure that the samples in one cluster are more like one another than they are to samples in other clusters. Second, a mechanism for partitioning the data into clusters using the similarity measure can be determined. One way to begin a clustering investigation can be to define a distance function and to compute the matrix of distances between all pairs of samples in a training dataset. If distance is a good measure of similarity, then the distance between reference entities in the same cluster can be significantly less than the distance between the reference entities in different clusters. However, clustering may not use a distance metric. For example, a nonmetric similarity function s(x, x') can be used to compare two vectors x and x'. s(x, x') can be a symmetric function whose value is large when x and x' are somehow "similar." Once a method for measuring "similarity" or "dissimilarity" between points in a dataset has been selected, clustering can use a criterion function that measures the clustering quality of any partition of the data. Partitions of the data set that extremize the criterion function can be used to cluster the data. Particular exemplary clustering techniques that can be used in the present disclosure can include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering includes unsupervised clustering (*e.g.,* with no preconceived number of clusters and/or no predetermination of cluster assignments).

*Ensembles of models and boosting.* In some embodiments, an ensemble (two or more) of models is used for the first and/or second model. In some embodiments, a boosting technique such as AdaBoost is used in conjunction with many other types of learning algorithms to improve the performance of the first and/or second model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, *etc.* In some embodiments, the plurality of outputs is combined using a voting method. In some embodiments, a respective model in the ensemble of models is weighted or unweighted.

One of skill in the art will readily appreciate other models that are applicable to the systems and methods of the present disclosure. In some embodiments, the systems, methods, and devices of the present disclosure utilize more than one model to provide an evaluation (*e.g.,* arrive at an evaluation given one or more inputs) with an increased accuracy. For instance, in some embodiments, each respective model arrives at a corresponding evaluation when provided a respective data set. Accordingly, each respective model can independently arrive at a result and then the result of each respective model is collectively verified through a comparison or amalgamation of the models. From this, a cumulative result is provided by the models. However, the present disclosure is not limited thereto.

*Block 580.* Referring to block 580, in some embodiments, when the first model indicates the candidate mutation is a CHIP variant, a report for the test subject includes the identity of the candidate mutation.

In some embodiments, the methods described herein include generating a clinical report 139-3 (*e.g.,* a patient report), providing clinical support for personalized cancer therapy, and/or using the information curated from sequencing biopsy samples, as described above. In some embodiments, the report is provided to a patient, physician, medical personnel, or researcher in a digital copy (for example, a JSON object, a pdf file, or an image on a website or portal), a hard copy (for example, printed on paper or another tangible medium). A report object, such as a JSON object, can be used for further processing and/or display. For example, information from the report object can be used to prepare a clinical laboratory report for return to an ordering physician. In some embodiments, the report is presented as text, as audio (for example, recorded or streaming), as images, or in another format and/or any combination thereof.

In some embodiments, the report includes information related to the specific characteristics of the patient's cancer, *e.g.,* detected genetic variants, epigenetic abnormalities, associated oncogenic pathogenic infections, and/or pathology abnormalities. In some embodiments, other characteristics of a patient's sample and/or clinical records are also included in the report. For example, in some embodiments, the clinical report includes information on clinical variants, *e.g.,* one or more of copy number variants (*e.g.,* for actionable genes CCNE1, CD274(PD-L1), EGFR, ERBB2(HER2), MET, MYC, BRCA1, and/or BRCA2), fusions, translocations, and/or rearrangements (*e.g.,* in actionable genes ALK, ROS1, RET, NTRK1, FGFR2, FGFR3, NTRK2 and/or NTRK3), pathogenic single nucleotide polymorphisms, insertion-deletions (*e.g.,* somatic/tumor and/or germline/normal), therapy biomarkers, microsatellite instability status, and/or tumor mutational burden.

*Block 582.* Referring to block 582, in some embodiments, the report further comprises a therapeutic recommendation for the test subject based on the identity of the candidate mutation. Start here

*Block 584.* Referring to block 584, in some embodiments, the report further comprises an identification of the candidate mutation as being associated with a secondary hematologic malignancy. A secondary hematologic malignancy is a blood cancer that develops as a result of prior exposure to chemotherapy, radiation therapy, environmental toxins, or genetic predisposition. These malignancies often occur as a late complication in patients who have been treated for a different primary cancer (such as breast cancer, lymphoma, or leukemia) or have underlying bone marrow disorders like aplastic anemia or myelodysplastic syndromes (MDS). Common types of SHMs include: therapy-related acute myeloid leukemia (t-AML), therapy-related myelodysplastic syndromes (t-MDS), secondary acute lymphoblastic leukemia (s-ALL), secondary myeloproliferative neoplasms (MPNs)

Mutations associated with secondary hematologic malignancies include mutations in genes involved in DNA repair, epigenetic regulation, and tumor suppression are commonly seen in SHMs. Some mutations that are associated with a secondary hematologic malignancy include mutations in TP53 (frequently mutated in therapy-related myeloid neoplasms; associated with poor prognosis), RUNX1 (affects hematopoietic differentiation and is commonly seen in therapy-related MDS/AML), ASXL1 (involved in epigenetic regulation, associated with poor outcomes in secondary MDS), DNMT3A (alters DNA methylation patterns, leading to increased leukemogenic potential), TET2 (plays a role in DNA demethylation; frequently mutated in secondary AML/MDS), FLT3-ITD (common in secondary AML; associated with aggressive disease), and NRAS/KRAS (involved in cell signaling pathways; frequently mutated in therapy-related leukemia.

The treatment approach for secondary hematologic malignancies depends on the specific malignancy, patient factors, and genetic mutations but generally includes chemotherapy (t-AML and t-MDS: standard AML induction therapy with cytarabine and anthracycline, such as under a 7+3 regimen, or alternative hypomethylating agents like azacitidine or decitabine for MDS; s-ALL: intensive chemotherapy, such as Hyper-CVAD or BFM protocols, MPNs: targeted therapies like ruxolitinib for myelofibrosis or hydroxyurea for polycythemia vera), targeted therapy (FLT3 inhibitors, such as midostaurin or gilteritinib, for FLT3-mutated AML, IDH1/2 inhibitors, such as ivosidenib or enasidenib, for IDH-mutated AML, BCL-2 inhibitors, such as venetoclax, in combination with azacitidine for high-risk MDS/AML), allogeneic hematopoietic stem cell transplantation (HSCT) (considered for fit patients with high-risk disease or poor prognostic mutations like TP53 or RUNX1), supportive care (transfusions, such as red blood cells, platelets, for cytopenias, growth factors, such as G-CSF, for neutropenia, infection prophylaxis using antibiotics, antifungals), and experimental and clinical trials that evaluate novel therapies like immune checkpoint inhibitors, CAR-T therapy, and epigenetic drugs. Secondary hematologic malignancies typically have a worse prognosis than de novo cases due to higher rates of chemotherapy resistance, presence of high-risk mutations (*e.g.,* TP53), and prior chemotherapy/radiation exposure leading to weakened bone marrow function.

*Block 586.* Referring to block 586, in some embodiments, the secondary hematologic malignancy is a therapy-related neoplasm, a secondary acute lymphoblastic leukemia, a secondary myelodysplastic syndrome, a secondary myeloproliferative neoplasm, a secondary non-Hodgkin lymphoma, a secondary plasma cell dyscrasia, or Richter's Transformation.

In some embodiments, the candidate mutation, now classified by the first or second model is used for further downstream analysis and biomarker detection. In some embodiments, the classified candidate mutation is used as a metric for disease detection, diagnosis, and/or treatment. In some embodiments, the classified candidate mutation is included in a clinical report made available to the patient or a clinician. In some embodiments, the classification of the candidate mutation, by the first or second model is used as a basis to select appropriate therapies and/or clinical trials.

As described herein, in some embodiments, one or more classified candidate mutations are used to match the subject with a targeted therapy and/or a clinical trial. In some embodiments, as described in further detail herein, one or more classified candidate mutations, one or more matched therapies 139-1-2, and/or one or more matched clinical trials are used to generate a patient report 139-1-3. In some embodiments, the patient report is transmitted to a medical professional treating the subject. In some embodiments, the patient is then administered a personalized course of therapy, *e.g.,* based on a matched therapy and/or clinical trial.

In some embodiments, the methods of classifying the candidate mutation fall within the context of a larger variant detection method such as illustrated in Figure 15 of United States Patent Application No. 18/969,159, entitled "Systems and Methods for Detecting Somatic Variants Derived from Circulating Tumor Nucleic Acids," filed on December 4, 2024, which is hereby incorporated by reference. For example, in some embodiments, the method includes obtaining sequence reads, as described herein, and aligning those reads to a reference construct (*e.g.,* a reference genome or mapped representation of several reference genomes), to generate aligned sequences 124 (*e.g.,* a plurality of unique sequence reads). In some embodiments, putative candidate mutations are identified and classified. In some embodiments, one or more candidate mutations are further validated by applying one or more filters. For instance, in some embodiments as described in U.S. Patent No. 11,475,981, a dynamic variant count threshold is used to apply a dynamic probabilistic variant count filter to sequencing data for the candidate mutations. In some embodiments, the method also includes applying a variant loci coverage filter. In some embodiments, the method also includes applying a variant allele fraction filter. In some embodiments, the method also includes applying a variant support mapping filter. In some embodiments, the method also includes applying a variant support sequencing quality filter. In some embodiments, the method also includes applying a low complexity region filter. In some embodiments, one or more candidate mutations are then further classified using the methods and systems described herein. When all candidate mutations have been classified according to these filters, the process proceeds with a reporting function.

Similarly, in some embodiments, for one or more target loci falling within a gene exon, only candidate mutations that would result in an amino acid change in the amino acid sequence encoded by the gene are evaluated. In some embodiments, any candidate mutations resulting in an amino acid change are evaluated. In some embodiments, candidate mutations resulting in a defined amino acid change, e.g., an amino acid change associated with cancer etiology and/or a particular actionable cancer therapy, are evaluated.

In some embodiments, the subject has breast cancer and candidate mutations associated with at least one of the following genes and/or genetic loci are evaluated by the first or second model: ERBB2 (or a genetic locus including a chromosomal position of 17:37880220 and/or 17:37881064), EGFR (or a genetic locus including a chromosomal position of 7:55227926, 7:55242511, and/or 7:55249022), ESR1 (or a genetic locus including a chromosomal position of 6:152419922, 6:152419923 and/or 6:152419926), KRAS (or a genetic locus including a chromosomal position of 12:25380275, 12:25380276, 12:25380277, and/or 12:25380279), MAP2K1 (or a genetic locus including a chromosomal position of 15:66729162 and/or 15:66729163), MET (or a genetic locus including a chromosomal position of 7:116422117 and/or 7:116423413); MTOR (or a genetic locus including a chromosomal position of 1:11187094, 1:11187096, and/or 1:11187796), NTRK1 (or a genetic locus including a chromosomal position of 1:156846342, 1:156849044 and/or 1:156849144), and PIK3CA (or a genetic locus including a chromosomal position of 3:178936082, 3:178936091, 3:178936092, 3:178936093, 3:178952084, and/or 3:178952085). In some embodiments, the subject has breast cancer and candidate mutations associated with at least 2, at least 3, at least 4, at least 5, at least 6, at least 7, or at least 8 of the genes listed above (or loci including the enumerated corresponding chromosomal positions) are evaluated. In some embodiments, the subject has breast cancer and candidate mutations associated with any of the genes listed above (or loci including the enumerated corresponding chromosomal positions) are evaluated by the first or second model.

Similarly, in some embodiments, the subject has non-small cell lung cancer and candidate mutations associated with at least one of the following genes and/or genetic loci are evaluated by the first or second model: ALK (or a genetic locus including a chromosomal position of 2:29443613, 2:29443631, 2:29443695, 2:29443697, 2:29445213, and/or 2:29445258), B2M (or a genetic locus including a chromosomal position of 15:45003745), BRAF (or a genetic locus including a chromosomal position of 7:140453135, 7:140453136, and/or 7:140453137), EGFR (or a genetic locus including a chromosomal position of 7:55227926, 7:55241704, 7:55241705, 7:55241706, 7:55242469, 7:55242511, 7:55249022, 7:55249071, 7:55249091, 7:55249092, 7:55249093, 7:55249094, and/or 7:55259515), ERBB2 (or a genetic locus including a chromosomal position of 17:37880220), KRAS (or a genetic locus including a chromosomal position of 12:25378562, 12:25378643, 12:25380275, 12:25380276, 12:25380277, 12:25380279, 12:25398255, 12:25398280, 12:25398281, 12:25398282, 12:25398283, 12:25398284, and/or 12:25398285), MAP2K1 (or a genetic locus including a chromosomal position of 15:66729162 and/or 15:66729163), MET (or a genetic locus including a chromosomal position of 7:116422117 and/or 7:116423413), NTRK1 (or a genetic locus including a chromosomal position of 1:156846342, 1:156849044, and/or 1:156849144), PIK3CA (or a genetic locus including a chromosomal position of 3:178936091, 3:178936092, 3:178936093, 3:178952072, 3:178952084, and/or 3:178952085), and STK11 (or a genetic locus including a chromosomal position of 19:1218483, 19:1220370, 19:1220487, 19:1220629, and/or 19:1220649). In some embodiments, the subject has non-small cell lung cancer and candidate mutations associated with at least 2, at least 3, at least 4, at least 5, at least 6, at least 7, at least 8, at least 9, or at least 10 of the genes listed above (or loci including the enumerated corresponding chromosomal positions) are evaluated. In some embodiments, the subject has non-small cell lung cancer and candidate mutations associated with any of the genes listed above (or loci including the enumerated corresponding chromosomal positions) are evaluated by the first and/or second model.

Similarly, in some embodiments, the subject has prostate cancer and candidate mutations associated with at least one of the following genes and/or genetic loci are evaluated by the first and/oor second model: AR (or a genetic locus including a chromosomal position of X:66766292, X:66931463, X:66931504, X:66937370, X:66937371, X:66937372, X:66943543, X:66943549, and/or X:66943552), EGFR (or a genetic locus including a chromosomal position of 7:55227926, 7:55242511, and/or 7:55249022), ERBB2 (or a genetic locus including a chromosomal position of 17:37880220), KRAS (or a genetic locus including a chromosomal position of 12:25380275, 12:25380276, and/or 12:25380277), MAP2K1 (or a genetic locus including a chromosomal position of 15:66729162 and/or 15:66729163), MET (or a genetic locus including a chromosomal position of 7:116422117 and/or 7:116423413), NTRK1 (or a genetic locus including a chromosomal position of 1:156846342, 1:156849044, and/or 1:156849144), and PIK3CA (or a genetic locus including a chromosomal position of 3:178952084 and/or 3:178952085). In some embodiments, the subject has prostate cancer and candidate mutations associated with at least 2, at least 3, at least 4, at least 5, at least 6, or at least 7 of the genes listed above (or loci including the enumerated corresponding chromosomal positions) are evaluated. In some embodiments, the subject has prostate cancer and candidate mutations associated with any of the genes listed above (or loci including the enumerated corresponding chromosomal positions) are evaluated.

In one example, the cancer condition is any type of cancer (for example, pan-cancer) and the candidate mutations evaluate by the method of the present disclosure include candidate mutations associated with any of the following genes: EGFR (or a genetic locus including a chromosomal position of 7:55227926, 7:55242511, and/or 7:55249022), ERBB2 (or a genetic locus including a chromosomal position of 17:37880220), KRAS (or a genetic locus including a chromosomal position of 12:25380275, 12:25380276, and/or 12:25380277), MAP2K1 (or a genetic locus including a chromosomal position of 15:66729162 and/or 15:66729163), MET (or a genetic locus including a chromosomal position of 7:116422117 and/or 7:116423413), NTRK1 (or a genetic locus including a chromosomal position of 1:156846342, 1:156849044, and/or 1:156849144), PIK3CA (or a genetic locus including a chromosomal position of 3:178952084 and/or 3:178952085), and TP53. In some embodiments, the subject has any cancer (*e.g.,* pan cancer) and candidate mutations associated with at least 2, at least 3, at least 4, at least 5, at least 6, or at least 7 of the genes listed above (or loci including the enumerated corresponding chromosomal positions) are evaluated. In some embodiments, the subject has any cancer (*e.g.,* pan cancer) and candidate mutations associated with any of the genes listed above (or loci including the enumerated corresponding chromosomal positions) are evaluated.

Similarly, in some embodiments, the subject has a tumor of unknown origin or a cancer of unknown primary and candidate mutations associated with at least one of the following genes and/or genetic loci are evaluated by the first or second model: EGFR (or a genetic locus including a chromosomal position of 7:55227926, 7:55242511, and/or 7:55249022), ERBB2 (or a genetic locus including a chromosomal position of 17:37880220), KRAS (or a genetic locus including a chromosomal position of 12:25380275, 12:25380276, 12:25380277, and/or 12:25398255), MAP2K1 (or a genetic locus including a chromosomal position of 15:66729162 and/or 15:66729163), MET (or a genetic locus including a chromosomal position of 7:116422117 and/or 7:116423413), NRAS (or a genetic locus including a chromosomal position of 1:115258748), NTRK1 (or a genetic locus including a chromosomal position of 1:156846342, 1:156849044, and/or 1:156849144), PIK3CA (or a genetic locus including a chromosomal position of 3:178927980, 3:178952084 and/or 3:178952085), and TP53. In some embodiments, the subject has any cancer (e.g., pan cancer) and candidate mutations associated with at least 2, at least 3, at least 4, at least 5, at least 6, at least 7, or at least 8 of the genes listed above (or loci including the enumerated corresponding chromosomal positions) are evaluated. In some embodiments, the subject has any cancer (*e.g.,* pan cancer) and candidate mutations associated with any of the genes listed above (or loci including the enumerated corresponding chromosomal positions) are evaluated by the first or second model.

### Candidate mutation classification

In some embodiments, a predicted functional effect and/or clinical interpretation for one or more classified candidate mutations is curated by using information from variant databases. In some embodiments, a weighted-heuristic model is used to characterize each variant.

In some embodiments, classified candidate mutations are labeled as "potentially actionable", "biologically relevant", "variants of unknown significance (VUSs)", or "benign". Potentially actionable alterations are protein-altering variants with an associated therapy based on evidence from the medical literature. Biologically relevant alterations are protein-altering variants that may have functional significance or have been observed in the medical literature but are not associated with a specific therapy. Classified candidate mutations of unknown significance (VUSs) are protein-altering variants exhibiting an unclear effect on function and/or without sufficient evidence to determine their pathogenicity. In some embodiments, benign variants are not reported. In some embodiments, classified candidate mutations are identified through aligning the patient's DNA sequence to the human genome reference sequence version hg19 (GRCh37). In some embodiments, actionable and biologically relevant somatic variants are provided in a clinical summary during report generation.

For instance, in some embodiments, candidate mutation classification and reporting is performed, where detected candidate mutations are investigated following criteria from known evolutionary models, functional data, clinical data, literature, and other research endeavors, including tumor organoid experiments. In some embodiments, candidate mutations are prioritized and classified based on known gene-disease relationships, hotspot regions within genes, internal and external somatic databases, primary literature, and other features of somatic drivers. Mutations can be added to a patient (or sample, for example, organoid sample) report based on recommendations from the AMP/ASCO/CAP guidelines. Additional guidelines may be followed. Briefly, pathogenic mutations with therapeutic, diagnostic, or prognostic significance may be prioritized in the report. Non-actionable pathogenic mutations may be included as biologically relevant, followed by mutations of uncertain significance. Translocations may be reported based on features of known gene fusions, relevant breakpoints, and biological relevance. Evidence may be curated from public and private databases or research and presented as 1) consensus guidelines 2) clinical research, or 3) case studies, with a link to the supporting literature. Germline alterations may be reported as secondary findings in a subset of genes for consenting patients. These may include genes recommended by the ACMG and additional genes associated with cancer predisposition or drug resistance.

In some embodiments, a clinical report 139-3 includes information about clinical trials for which the patient is eligible, therapies that are specific to the patient's cancer, and/or possible therapeutic adverse effects associated with the specific characteristics of the patient's cancer, *e.g.,* the patient's genetic variations, epigenetic abnormalities, associated oncogenic pathogenic infections, and/or pathology abnormalities, or other characteristics of the patient's sample and/or clinical records. For example, in some embodiments, the clinical report includes such patient information and analysis metrics, including cancer type and/or diagnosis, variant allele fraction, patient demographic and/or institution, matched therapies (e.g., FDA approved and/or investigational), matched clinical trials, variants of unknown significance (VUS), genes with low coverage, panel information, specimen information, details on reported variants, patient clinical history, status and/or availability of previous test results, and/or version of bioinformatics pipeline.

In some embodiments, the results included in the report, and/or any additional results (for example, from the bioinformatics pipeline), are used to query a database of clinical data, for example, to determine whether there is a trend showing that a particular therapy was effective or ineffective in treating (*e.g.,* slowing or halting cancer progression), and/or adverse effects of such treatments in other patients having the same or similar characteristics.

In some embodiments, the results are used to design cell-based studies of the patient's biology, *e.g.,* tumor organoid experiments. For example, an organoid may be genetically engineered to have the same characteristics as the specimen and may be observed after exposure to a therapy to determine whether the therapy can reduce the growth rate of the organoid, and thus may be likely to reduce the growth rate of cancer in the patient associated with the specimen. Similarly, in some embodiments, the results are used to direct studies on tumor organoids derived directly from the patient. An example of such experimentation is described in U.S. Patent No. 11,415,571, the content of which is hereby incorporated by reference, in its entirety, for all purposes.

As illustrated in Figure 2A, in some embodiments, a clinical report is checked for final validation, review, and sign-off by a medical practitioner (*e.g.,* a pathologist). The clinical report is then sent for action (*e.g.,* for precision oncology applications).

### Specific Embodiments of the Disclosure.

In some aspects, the systems and methods disclosed herein may be used to support clinical decisions for personalized treatment of cancer. For example, in some embodiments, the methods described herein identify actionable genomic variants and/or genomic states with associated recommended cancer therapies. In some embodiments, the recommended treatment is dependent upon whether or not the subject has a particular actionable variant and/or genomic status. Recommended treatment modalities can be therapeutic drugs and/or assignment to one or more clinical trials. Generally, current treatment guidelines for various cancers are maintained by various organizations, including the National Cancer Institute and Merck & Co., in the Merck Manual.

In some embodiments, the methods described herein further includes assigning therapy and/or administering therapy to the subject based on the identification of an actionable genomic variant and/or genomic state, e.g., based on whether or not the subject's cancer will be responsive to a particular personalized cancer therapy regimen. For example, in some embodiments, when the subject's cancer is classified as having a first actionable variant and/or genomic state, the subject is assigned or administered a first personalized cancer therapy that is associated with the first actionable variant and/or genomic state, and when the subject's cancer is classified as having a second actionable variant and/or genomic state, the subject is assigned or administered a second personalized cancer therapy that is associated with the second actionable variant. Assignment or administration of a therapy or a clinical trial to a subject is thus tailored for treatment of the actionable variants and/or genomic states of the cancer patient.

### Examples

In these examples novel methods that use the expected VAF for a heterozygous germline variant in a tumor sample, considering factors such as copy number, A/B allele balance, and tumor content, were developed in accordance with the disclosure of Figure 5 above. A machine learning model was trained on common germline variants and known clonal hematopoiesis (CH) hotspot mutations. The accuracy of a low coverage tissue based CH variant calling pipeline in which tumor specimens were sequenced at 500x with normal matched buffy coat specimens sequenced at 150x coverage, hereafter referenced as "xT" (See, Beaubier *et al.,* 2019, "Clinical validation of the tempus xT next-generation targeted oncology sequencing assay," Oncotarget 10(24) pp. 2384-2396, which is hereby incorporated by reference) was compared to CH variant calling that used higher depth sequencing from liquid biopsies, hereafter referenced as "xF+" (Dreissen *et al.,* 2024, "A Novel Combination of Tissue-Informed, Comprehensive Genomic Profiling (CGP) And Non-Bespoke Blood-Based Profiling for Quantifying Circulating Tumor DNA (ctDNA)," Cancer Research 84(6) 3498, which is hereby incorporated by reference) across 3,000 unique samples using the CHIP calling models of the present disclosure. That is, both the xT and xF+ pipeline was run on samples from such subjects. The xT test used a buffy coat matched normal (500x coverage) and a tumor tissue sample (150x coverage) from the subject while the xF+ test used 5000x coverage on a plasma sample from the same subject.

The disclosed models, in accordance with Figure 5, substantially improves the separation between CH and germline variants using low coverage data. In a representative distribution of variants with VAFs over 20% held out for testing, sensitivity for CH variants improved from 77% to 91%, precision from 80% to 97%, and specificity from 99.5% to 99.9% (ROC-AUC of 0.995) compared to a heuristic logic that does not incorporate copy number.

Canonical CHIP variants were detected using xF+ for SNVs, INDELs, copy number gains, and gene rearrangements with a VAF above 5%. Of these canonical CHIP variants, 92% were detected in the low coverage xT pipeline.

Variants identified as CH in xT using the models of the present disclosure, with a VAF above 2%, were validated as true CH variants by xF+ with 90% precision. CH variants detected in both plasma (xF+) and buffy coat (xF) have highly correlated VAFs (R² = 0.79, cor = 0.89, m = 0.94), and 3.6% of the samples assessed contain a high VAF (>=20%) CH variant.

This demonstrates that a platform that makes use of low coverage data (500x depth coverage for tumor specimens and 150x coverage for matched normal specimens) can distinguish between CH, germline, and artifactual variants with high sensitivity and a low false positive rate. Although CH at high VAF is present in only a minority of samples (3.6%), it is regularly observed in high throughput clinical sequencing. These variants are likely to be clinically significant and may represent secondary hematologic malignancies, which are more common in cancer patients than in healthy populations. Therefore, accuracy-driven approaches for identifying CH in buffy coat matched data (*e.g.,* xF) are important for improving clinical outcomes and understanding the underlying biology of these variants.

The two following examples, 1 and 2, provide specific methods in accordance with the present disclosure for distinguishing CHIP variants from germline variants.

### Example 1 - CHIP versus germline method

*True Chip.* True CHIP variants were identified as variants on a curated list of 126 unique mutations with strong literature support associated with CHIP or heme malignancies. These CHIP variants were queried using xT buffy coat samples (normal). There were 4,430 occurrences of variants on this list of 126 unique mutation in the buffy coat (normal) samples, and 1,288 of them occurred at VAFs of 20% or higher in the buffy coat.

*True Germline.* To find true germline variants were queried in xT buffy coat (normal tissue). A total of 5 million random variants from normal tissue samples were queried. These were filtered by gnomad (See Karczewski et al, 2020, "The mutational constraint spectrum quantified from variation in 141,456 humans," Nature 581, pp. 434-443, which is hereby incorporated by reference), to those with gnomad frequency of 10% or higher. Any variants with typical 'artifact' filters (exclusion list, grey list, *etc*.) were excluded. A total of 80,000 variants meeting these criteria were randomly selected.

*Balance* of *the training*/*testing data.* True CHIP and true germline variants for training and testing were restricted to those with VAF of 20% or greater in buffy coat samples of xT, as there was not much need for CHIP/germline discrimination below that level. This left 1,287 true CHIP variants, and 79,230 true germline variants. The germline variants were randomly under-sampled to bring the relative proportions of CHIP to germline variants to a 1:50 ratio. The small number of variants that were filter passing in the xT pipeline were excluded.

This led to a final dataset of 1,297 CHIP variants and 64,275 germline variants. This was divided 50/50 into model-testing and model-training.

*Germline expectation.* Germline expectation (expected VAF of germline variant in the tumor isolate) was calculated from the tumor purity, the major copy number, and the minor copy number in accordance with block 540 and Figure 5 above. This incorporation of CNV information allows much cleaner separation of CHIP and germline under loss of heterozygosity conditions.

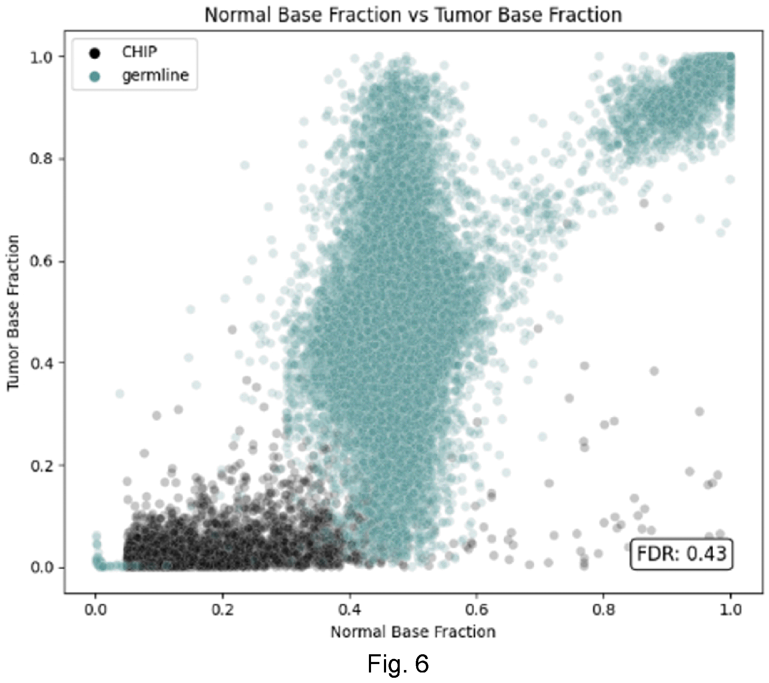

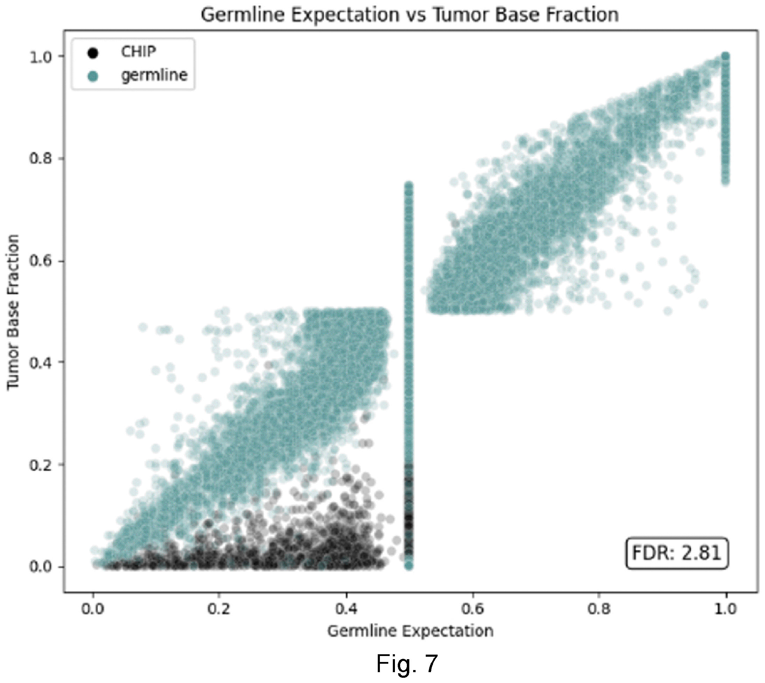

As illustrated in Figure 6, comparison of CHIP versus germline based on VAF in normal (normal base fraction) ~ VAF in tumor (tumor base fraction) does have visual separation, but with significant overlap due to loss of heterozygosity (LOH; Fisher's Discriminant Ratio of 0.43). However, as illustrated in Figure 7, this comparison of CHIP versus germline based on germline expectation in tumor ~ VAF in tumor (tumor base fraction), has clear separation under all conditions (Fisher's Discriminant Ratio of 2.81).

*Model training.* A random forest model, which outperformed sever other machine learning modes including support vector machines and logistic regression, was selected for this example. Random forest was also chosen for flexibility/interpretability. Features used are shown in Figure 7. This model only used tumor purity and VAF information - it was naive to mutation effect, gene identity, etc.. This model was directed to determining whether the VAF in tumor relative to normal is more consistent with CHIP or germline.

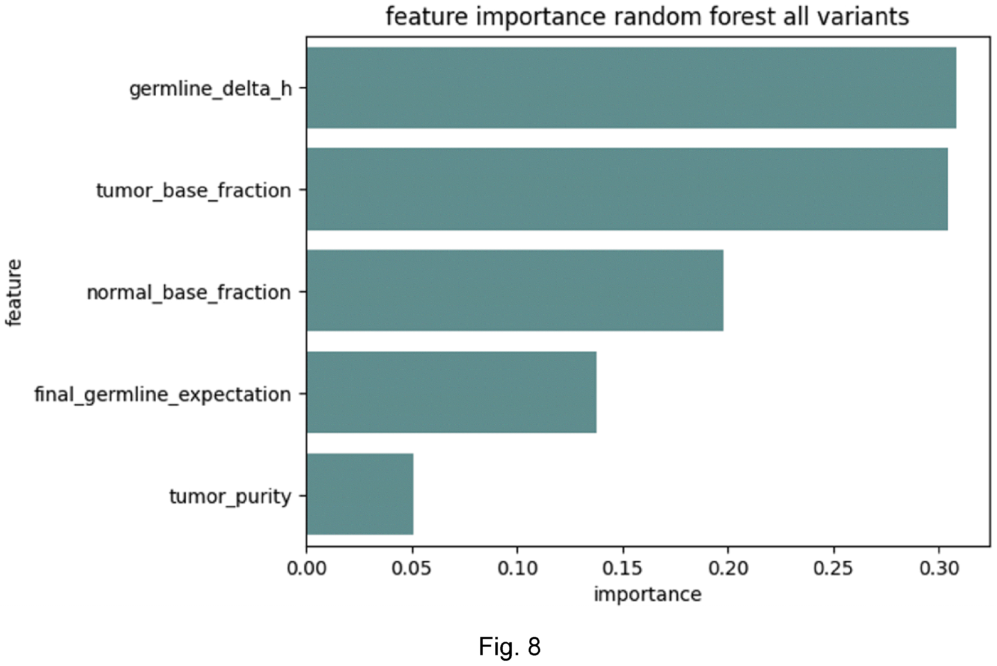

Figure 8 shows the relative feature importance of each of the features used in the model.

In Figure 8, the feature "germline_delta_h" refers to the change in heterozygosity of a candidate mutation as discussed in block 542 (an absolute value of a difference between the reference base fraction for the candidate mutation and the germline expectation of the candidate mutation) in conjunction with Figure 5.

The feature "tumor_base_fraction" is the tumor base fraction for a given mutation, as discussed in block 536 in conjunction with Figure 5.

The feature "normal_base_fraction" is the normal (reference) base fraction for a given mutation, as discussed in block 538 in conjunction with Figure 5.

The feature "final_germline_expectation" is the germline expectation for a given mutation, as discussed in block 540 in conjunction with Figure 5.

The feature "tumor_purity" is the tumor purity of the tumor sample for a given mutation, which is typically provided in annotation data associated with a given tumor.

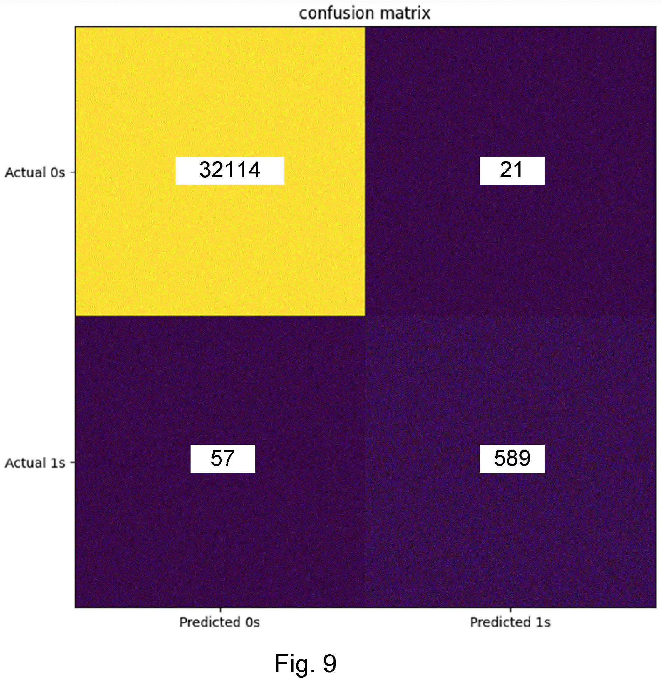

*Model performance.* The F1 for the model with 10-fold cross-validation was 0.936 (0.022). The F1 score is the harmonic mean of precision and recall, providing a balance between the two. It is a measure of a model's accuracy when dealing with imbalanced classes (*e.g.,* when the classes are not equally represented in the dataset). The F1 score formula is: $F 1 score = \frac{2 \times \left(Precision\times Recall\right)}{Precision + Recall}$ An F1 score of 0.936 means the model had a high balance between precision and recall. The value 0.022 is the standard deviation, indicating that across the 10 folds, the F1 score varied slightly by 2.2%. This suggests the model's F1 score was stable. Figure 9 illustrates the overall confusion matrix for the model across the ten folds from which the F1 score was calculated.

The accuracy for the model with 10-fold cross-validation was 0.998 (0.001). Accuracy is the percentage of correctly classified instances (CHIP versus germline) over the total instances, and it measures the overall performance of the model. An accuracy of 0.998 means that the model correctly classified 99.8% of all mutation on average across the 10 folds. The value 0.001 was the standard deviation, indicating that the accuracy was highly consistent across the folds with minimal variation (just 0.1% difference on average). Figure 9 illustrates the overall confusion matrix for the model across the ten folds from which accuracy was calculated.

The precision for the model with 10-fold cross-validation was 0.973 (0.020). Precision measures the proportion of true positives among all instances classified as positive. Precision informs on how many of the positive predictions (CHIP predictions) were actually correct. A value of 0.973 means that 97.3% of the instances predicted as positive (CHIP) were correctly identified as CHIP. The given value 0.020 was the standard deviation, indicating a slight variation of 2% across the 10 folds for precision. Figure 9 illustrates the overall confusion matrix for the model across the ten folds from which the precision was calculated.

The recall for the model with 10-fold cross-validation was 0.903 (0.037). Recall measures the proportion of true positives (CHIP) among all actual positives. It informs how many of the actual positive instances (true CHIP) were correctly identified. A value of 0.903 means the model correctly identified 90.3% of the actual CHIP mutations on average. The given value 0.037 was the standard deviation, indicating a 3.7% variation in recall across the 10 folds. Figure 9 illustrates the overall confusion matrix for the model across the ten folds from which the recall was calculated.

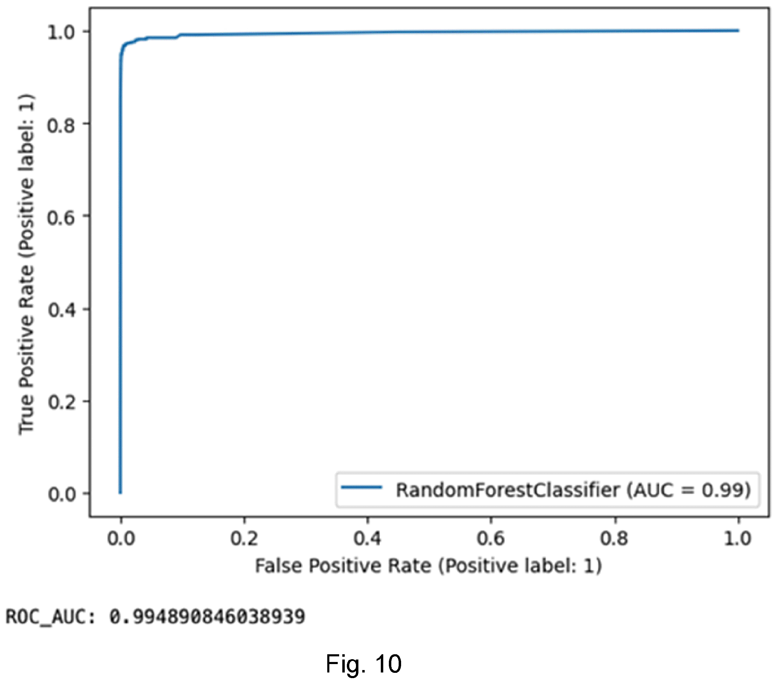

As illustrated in Figure 10, the Receiver Operating Characteristic - Area Under the Curve (ROC-AUC) for the model with 10-fold cross-validation was 0.995 (0.004). ROC-AUC measures of the model's ability to distinguish between positive (CHIP) and negative (germline) classes, where a score of 1 represents perfect classification and a score of 0.5 represents a model with no discriminatory power (akin to random guessing). An ROC-AUC value of 0.995 means the model had a very high ability to discriminate between CHIP and germline mutations, with 99.5% of the time being able to correctly distinguish between the two. The given value of 0.004 was the standard deviation, indicating that the model's discrimination ability was stable with a minimal 0.4% variation across the folds.

With regard to performance on CHIP vs germline discrimination, despite highly unbalanced data, both sensitivity and specificity were very high. This model outperformed random forest models without the germline expectation, and substantially outperformed the existing production models.

### Example 2 - CHIP vs artifact description

While the CHIP versus germline model of example 1 was trained and tested on variants above 20% VAF, a second model was created to evaluate variants between 2% VAF and 20% VAF, to discriminate CHIP and artifacts in accordance with block 552 and Figure 5. The assumption was that variants that were detected using the xT normal buffy coat samples, but that were somatic and filter passing in the xF+ for the same patient, are CHIP, whereas variants that were not somatic and filter passing in xF+ for the same patient were artifacts. Training/calling variants down to 2% was done for good performance metrics at 5%.

The variants for this example were sourced from 3,311 paired xt/XF+ patients. CHIP variants were all occurrences of the curated hotspot variants in these samples below 20% VAF. Artifacts were sourced from low VAF variants seen in the buffy coat xT normals, that are candidates for CHIP (rare in gnomAD, no artifact filters applied, high impact mutation effect), and are not somatic filter passing in xF+. This leads to a final balance of 1155 CHIP variants and 3351 artifacts. This may be slightly more unbalanced than the true ratios, as a large percentage of CHIP was not on the curated hotspot list.

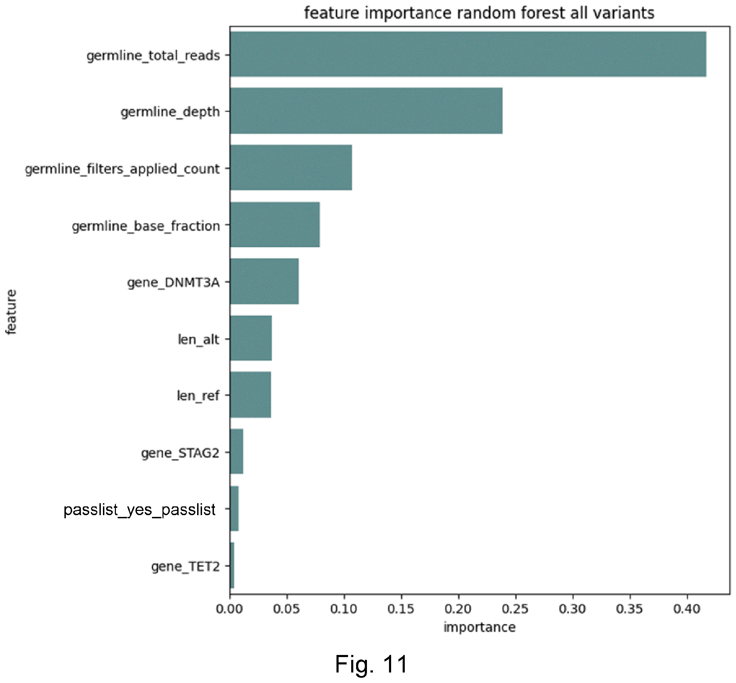

Figure 11 lists the features used in this second model and their relative importance. Most features were related to sequencing depth, and total support. DNMT3A, STAG2 and TET2 are one-hot encoded (mutations in DNMT3A and TET2 tend to be real, mutations in STAG2 tends to have artifacts). Total number of filters applied (germline_filters_applied_count) was a valuable feature (if a variant is only filtered by var_frac_1, it tended to be real - although of note, variants filtered by exclusion lists were already excluded from testing/training/CHIP consideration). The pass list (passlist_yes_passlist) was presence on a list of known CHIP variants. It was a proxy for 'pathogenic, canonical CHIP', and includes many TP53 mutations.

Two probability cutoffs were tuned for the random forest, one for variants between 5%-20% VAF in the matched normal buffy coat of xT, and a more stringent cutoff for variants between 2%-5%. This was intended to balance sensitivity and specificity. Excluding variants below 5%, and using that as a hard floor, substantially reduced performance metrics for variants above 5%, as many variants that were detected in xF+ at 5% were detected in xT at 4%, just under the floor.

Performance at the chosen cutoff for variants between 2%-5% were: 79.7% positive predictive agreement (PPA) and 85.8% positive predictive value (PPV) against xF+ filter passing somatic mutations, 94% accuracy, with 82.6% F1. The formula for PPA was: $PPA = \frac{True Positives \left(TP\right)}{True Positives \left(TP\right) + False Negatives \left(FN\right)}$ where TP means correctly identified mutations and FN means mutations missed by the model. Thus, PPA measures the sensitivity or recall of the model, e.g., how many of the actual positive instances (mutations) were correctly detected.

The formula for PPV was: $PPV = \frac{True Positives \left(TP\right)}{True Positives \left(TP\right) + False Positives \left(FP\right)}$ where TP means correctly identified mutations and FP means non-mutated regions incorrectly identified as mutations by the model. Thus, PPV measures the reliability of positive predictions of the model.

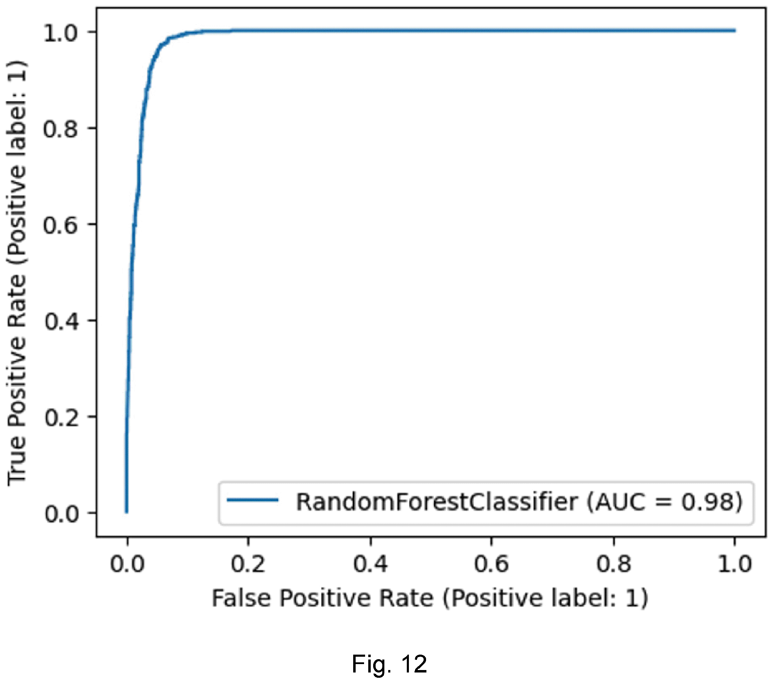

By contrast, performance at the chosen cutoff for variants between 5% and 20% was: 98.3% PPA (Figure 12) and 95.6% PPV against xF+ filter passing, 94.7% accuracy, 96.9% F1, and 098 ROC-AUC (Figure 12).

### Additional Embodiments

Another aspect of the present disclosure provides a computer system comprising one or more processors and a non-transitory computer-readable medium including computer-executable instructions that, when executed by the one or more processors, cause the processors to perform any of the methods and/or embodiments disclosed herein.

Yet another aspect of the present disclosure provides a non-transitory computer-readable storage medium having stored thereon program code instructions that, when executed by a processor, cause the processor to perform any of the methods and/or embodiments disclosed herein.

Although inventions have been particularly shown and described with reference to a preferred embodiment and various alternate embodiments, it will be understood by persons skilled in the relevant art that various changes in form and details can be made therein without departing from the spirit and scope of the invention.

### Equivalents and Incorporation by Reference

All references cited herein are incorporated by reference to the same extent as if each individual publication, database entry (*e.g.,* Genbank sequences or GenelD entries), patent application, or patent, was specifically and individually indicated to be incorporated by reference in its entirety, for all purposes. This statement of incorporation by reference is intended by Applicants, pursuant to 37 C.F.R. §1.57(b)(1), to relate to each and every individual publication, database entry (*e.g.,* Genbank sequences or GenelD entries), patent application, or patent, each of which is clearly identified in compliance with 37 C.F.R. §1.57(b)(2), even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

### Additional Considerations

The foregoing description of the embodiments has been presented for the purpose of illustration; it is not intended to be exhaustive or to limit the patent rights to the precise forms disclosed. Persons skilled in the relevant art will appreciate that many modifications and variations are possible in light of the above disclosure.

Any feature mentioned in one item category, *e.g.,* method, can be claimed in another item category, e.g., computer program product, system, storage medium, as well. The dependencies or references back in the attached items are chosen for formal reasons only. However, any subject matter resulting from a deliberate reference back to any previous item (in particular multiple dependencies) can be claimed as well, so that any combination of items and the features thereof is disclosed and can be claimed regardless of the dependencies chosen in the attached items. The subject matter, in some embodiments, includes not only the combinations of features as set out in the disclosed embodiments but also any other combination of features from different embodiments. Various features mentioned in the different embodiments can be combined with explicit mentioning of such combination or arrangement in an example embodiment or without any explicit mentioning. Furthermore, any of the embodiments and features described or depicted herein, in some embodiments, are claimed in a separate item and/or in any combination with any embodiment or feature described or depicted herein or with any of the features.

Some portions of this description describe the embodiments in terms of algorithms and symbolic representations of operations on information. These operations and algorithmic descriptions, while described functionally, computationally, or logically, are understood to be implemented by computer programs or equivalent electrical circuits, microcode, or the like. Furthermore, it has also proven convenient at times, to refer to these arrangements of operations as engines, without loss of generality. The described operations and their associated engines are, in some embodiments, embodied in software, firmware, hardware, or any combinations thereof.

Any of the steps, operations, or processes described herein, in some embodiments, are performed or implemented with one or more hardware or software engines, alone or in combination with other devices. In one embodiment, a software engine is implemented with a computer program product comprising a computer-readable medium containing computer program code, which can be executed by a computer processor for performing any or all of the steps, operations, or processes described. The term "steps" does not mandate or imply a particular order. For example, while this disclosure describes, in some embodiments, a process that includes multiple steps sequentially with arrows present in a flowchart, the steps in the process do not need to be performed by the specific order claimed or described in the disclosure. In some implementations, some steps are performed before others even though the other steps are claimed or described first in this disclosure. Likewise, any use of (i), (ii), (iii), etc., or (a), (b), (c), etc. in the specification or in the items, unless specified, is used to better enumerate items or steps and also does not mandate a particular order.

**The following items constitute embodiments of the invention:**
1. A method of characterizing a candidate mutation, the method comprising:
   A) obtaining a plurality of reference sequence reads mapping to a genomic location of the candidate mutation from a first sequencing reaction using a reference sample of a test subject;
   B) obtaining a plurality of tumor sequence reads mapping to the genomic location of the candidate mutation from a second sequencing reaction using a solid tumor sample of the test subject;
   C) determining a reference base fraction of the candidate mutation using the plurality of reference sequence reads;
   D) determining a germline expectation of the candidate mutation using at least:
      a tumor purity of the solid tumor sample,
      a major copy number at the genomic location of the candidate mutation derived from the plurality of tumor sequence reads, and
      a minor copy number at the genomic location of the candidate mutation derived from the plurality of tumor sequence reads;
   E) inputting first information into a first model comprising a first plurality of parameters thereby obtaining, as output from the first model, through application of the first plurality of parameters to the first information, an indication of whether the candidate mutation is (a) a clonal hematopoiesis of indeterminate potential (CHIP) mutation or (b) a germline mutation,
   wherein the first information comprises (i) the reference base fraction for the candidate mutation and (ii) the germline expectation of the candidate mutation.
2. The method of item 1, wherein the first information further comprises an absolute value of a difference between the reference base fraction for the candidate mutation and the germline expectation of the candidate mutation.
3. The method of item 2 or 3, wherein
   The method further comprises determining the tumor base fraction of the candidate mutation using the plurality of tumor sequence reads, and
   the first information further comprises the tumor base fraction of the candidate mutation.
4. The method of any one of items 1-3, wherein the first information further comprises the tumor purity of the solid tumor sample.
5. The method of any one of items 1-4, the method further comprising:
   determining whether the reference base fraction for the candidate mutation satisfies a first threshold, wherein
   when the reference base fraction for the candidate mutation satisfies the first threshold, the candidate mutation is deemed to be (a) the CHIP mutation or (b) the germline mutation in accordance with the first model, and
   when the reference base fraction for the candidate mutation fails to satisfy the first threshold, the candidate mutation is deemed to be (a) the CHIP mutation or (b) an artifact in accordance with a second model.
6. The method of items 5, wherein the reference base fraction for the candidate mutation satisfies the first threshold when the reference base fraction exceeds twenty percent.
7. The method of item5, the method further comprising:
   inputting second information into the second model, wherein the second model comprises a second plurality of parameters, thereby obtaining as output from the second model, through application of the second plurality of parameters to the second information, an indication of whether the candidate mutation is (a) the CHIP mutation or (b) the artifact,
   wherein the second information comprises at least two of (i) a total number of sequence reads in the plurality of reference sequence reads mapping to the locus of the candidate mutation, (ii) a sequencing depth of the first sequencing reaction at the locus of the candidate mutation, and (iii) an indication of whether the candidate mutation satisfied a minimum variant fraction or a minimum insertion or deletion test.
8. The method of item7, wherein
   the first or second information further comprises the reference base fraction of the candidate mutation.
9. The method of item7 or 8, wherein
   the first or second information further comprises an indication as to whether or not the candidate mutation is in DNMT3A.
10. The method of any one of items 7-9, wherein
   the first or second information further comprises an indication as to whether or not the candidate mutation is in STAG2.
11. The method of any one of items 7-10, wherein
   the first or second information further comprises an indication as to whether or not the candidate mutation is in TET2.
12. The method of any one of items 7-11, wherein
   the second information further comprises a length of the candidate mutation.
13. The method of any one of items 7-12, wherein
   the second information further comprises a length of a wildtype allele corresponding to the candidate mutation.
14. The method of any one of items 7-13, wherein
   the second information further comprises a binary indication of whether or not the candidate mutation is in a curated list of known CHIP mutations.
15. The method of any one of items 1-14, wherein the first model or the second model is selected from the group consisting of a regression model, a neural network, a support vector machine, a Naive Bayes model, a nearest neighbor model, a boosted trees model, a random forest model, a decision tree, a gradient boosted tree, an elastic net, a logistic regression model, and a clustering model.
16. The method of any one of items 1-14, wherein the first model or the second model is a random forest model.
17. The method of any one of items 1-14, wherein the first model or the second model is an XGboost model.
18. The method of any one of items 1-17, wherein the reference sample is blood or saliva.
19. The method of any one of items 1-17, wherein the reference sample comprises blood, whole blood, peripheral blood, plasma, serum, or lymph.
20. The method of any one of items 1-17, wherein the reference sample consists of blood, whole blood, peripheral blood, plasma, serum, or lymph.
21. The method of any one of items 1-17, wherein the reference sample consists of buffy coat.
22. The method of any one of items 1-17, wherein the reference sample comprises buffy coat.
23. The method of any one of items 1-22, wherein the candidate mutation is a single nucleotide variant (SNV).
24. The method of any one of items 1-22, wherein the candidate mutation is an indel.
25. The method of any one of items 1-22, wherein the candidate mutation is a copy number variant or a translocation.
26. The method of any one of items 1-25, wherein the first sequencing reaction is a panel-based sequencing reaction of a plurality of loci.
27. The method of item 26, wherein the plurality of loci is sequenced at an average sequence depth of at less than 250x by the first sequencing reaction.
28. The method of item 26, wherein the plurality of loci is sequenced at an average sequence depth of at less than 200x by the first sequencing reaction.
29. The method of any one of items 26-28, wherein the second sequencing reaction is a panel-based sequencing reaction of the plurality of loci.
30. The method of item 29, wherein the plurality of loci is sequenced at an average sequence depth of less than 1000x by the second sequencing reaction.
31. The method of item 29, wherein the plurality of loci is sequenced at an average sequence depth of less than 600x by the second sequencing reaction.
32. The method of any one of items 26-31, wherein the plurality of loci comprises at least 5 genes in Table 1.
33. The method of any one of items 26-31, wherein the plurality of loci comprises at least 25 genes in Table 1.
34. The method of any one of items 26-31, wherein the plurality of loci comprises at least 50 genes in Table 1.
35. The method of any one of items 26-31, wherein the plurality of loci comprises at least 5 genes in Table 2.
36. The method of any one of items 26-31, wherein the plurality of loci comprises at least 25 genes in Table 2.
37. The method of any one of items 26-31, wherein the plurality of loci comprises at least 50 genes in Table 2.
38. The method of any one of items 1-25, wherein the first sequencing reaction or the second sequencing reaction is a whole genome sequencing.
39. The method of any one of items 1-38, wherein the test subject is afflicted with a cancer condition.
40. The method of item 39, wherein the cancer condition is lung cancer, breast cancer, ovarian cancer, cervical cancer, a uveal melanoma, colorectal cancer, chromophobe renal cell carcinoma, liver cancer, an endocrine tumor, oropharyngeal cancer, retinoblastoma, biliary cancer, adrenal cancer, neural cancer, neuroblastoma, basal cell carcinoma, brain cancer, a non-clear cell renal cell carcinoma, a glioblastoma, a glioma, kidney cancer, gastrointestinal stromal tumor, a medulloblastoma, bladder cancer, gastric cancer, bone cancer, thymoma, prostate cancer, a clear cell renal cell carcinoma, skin cancer, thyroid cancer, a sarcoma, testicular cancer, head and neck cancer, a meningioma, peritoneal cancer, endometrial cancer, pancreatic cancer, mesothelioma, esophageal cancer, small cell lung cancer, HER2 negative breast cancer, ovarian serous carcinoma, HR+ breast cancer, uterine serous carcinoma, a uterine corpus endometrial carcinoma, a gastroesophageal junction adenocarcinoma, gallbladder cancer, chordoma, or a papillary renal cell carcinoma.
41. The method of item 39, wherein the cancer condition is a particular stage of a particular type of cancer.
42. The method of any one of item 1-41, wherein the first model indicates the candidate variant is a CHIP variant and a report is generated for the test subject comprising the identity of the candidate variant.
43. The method of item 42, wherein the report further comprises a therapeutic recommendation for the test subject based on the identity of the candidate variant.
44. The method of item 42 or 43, wherein the report further comprises an identification of the candidate variant as being associated with a secondary hematologic malignancy.
45. The method of item 44, wherein the secondary hematologic malignancy is a therapy-related neoplasm, a secondary acute lymphoblastic leukemia, a secondary myelodysplastic syndrome, a secondary myeloproliferative neoplasm, a secondary non-Hodgkin lymphoma, a secondary plasma cell dyscrasia, or Richter's Transformation.
46. The method of any one of items 1-45, wherein the first or second information further comprises a binary indication as to whether or not the candidate mutation is in the ASXL1 gene.
47. The method of any one of items 1-46, wherein the first or second information further comprises a binary indication as to whether or not the candidate mutation is in the JAK2 gene.
48. The method of any one of items 1-47, wherein the first or second information further comprises a binary indication as to whether or not the candidate mutation is in the SF3B1 gene.
49. The method of any one of items 1-48, wherein the first or second information further comprises a binary indication as to whether or not the candidate mutation is in the PPM1d gene.
50. The method of any one of items 1-49, wherein the first or second information further comprises a binary indication as to whether or not the candidate mutation is in the EGFR gene.
51. The method of any one of items 1-50, wherein the first or second information further comprises a binary indication as to whether or not the candidate mutation is in the KMT2C gene.
52. The method of any one of items 1-51, wherein the first or second information further comprises a binary indication as to whether or not the candidate mutation is in a CHIP driver gene.
53. The method of any one of items 1-52, wherein the first or second information further comprises a COSMIC solid tumor frequency of the candidate mutation.
54. The method of any one of items 1-53, wherein the first or second information further comprises a GnomAD frequency of the candidate mutation.
55. The method of any one of items 1-54, wherein the first or second information further comprises a score for the candidate mutation using any one of single-base substitution signatures SBS1 - SBS60.
56. The method of any one of items 1-55, wherein the first or second information further comprises a score for the candidate mutation using any one of single-base substitution signatures in the group consisting of SBS55, SBS2, SBS35, SBS7a, SBS54, SBS8, SBS6, SBS44, SBS10a, SBS59, SBS25, SBS21, SBS19, SBS30, SBS10b, SBS7b, SBS11, SBS32, SBS17b, SBS26, SBS4, and SBS33.
57. The method of any one of items 1-56, wherein the first or second information further comprises an ExAC frequency of the candidate mutation.
58. The method of any one of items 1-57, wherein
   the first sequencing reaction is at a depth of less than 200x, and
   the second sequencing reaction is at a depth of less than 600x.
59. The method of item 58, wherein
   the first sequencing reaction is at a depth of at least 50x, and
   the second sequencing reaction is at a depth of at least 100x.
60. A computer system comprising:
   one or more processors; and
   a non-transitory computer-readable medium including computer-executable instructions that, when executed by the one or more processors, cause the processors to perform a method according to any one of items 1-59.
61. A non-transitory computer-readable storage medium having stored thereon program code instructions that, when executed by a processor, cause the processor to perform the method according to any one of items 1-59.

## Claims

1. A method of characterizing a candidate mutation, the method comprising:
A) obtaining a plurality of reference sequence reads mapping to a genomic location of the candidate mutation from a first sequencing reaction using a reference sample of a test subject;
B) obtaining a plurality of tumor sequence reads mapping to the genomic location of the candidate mutation from a second sequencing reaction using a solid tumor sample of the test subject;
C) determining a reference base fraction of the candidate mutation using the plurality of reference sequence reads;
D) determining a germline expectation of the candidate mutation using at least:
a tumor purity of the solid tumor sample,
a major copy number at the genomic location of the candidate mutation derived from the plurality of tumor sequence reads, and
a minor copy number at the genomic location of the candidate mutation derived from the plurality of tumor sequence reads;
E) inputting first information into a first model comprising a first plurality of parameters thereby obtaining, as output from the first model, through application of the first plurality of parameters to the first information, an indication of whether the candidate mutation is (a) a clonal hematopoiesis of indeterminate potential (CHIP) mutation or (b) a germline mutation,
wherein the first information comprises (i) the reference base fraction for the candidate mutation and (ii) the germline expectation of the candidate mutation,
optionally wherein the first information further comprises an absolute value of a difference between the reference base fraction for the candidate mutation and the germline expectation of the candidate mutation,
optionally wherein the first information further comprises the tumor purity of the solid tumor sample.

2. The method of claim 1, wherein
the method further comprises determining the tumor base fraction of the candidate mutation using the plurality of tumor sequence reads, and
the first information further comprises the tumor base fraction of the candidate mutation; and/or
the method further comprises:
determining whether the reference base fraction for the candidate mutation satisfies a first threshold, wherein
when the reference base fraction for the candidate mutation satisfies the first threshold, the candidate mutation is deemed to be (a) the CHIP mutation or (b) the germline mutation in accordance with the first model, and
when the reference base fraction for the candidate mutation fails to satisfy the first threshold, the candidate mutation is deemed to be (a) the CHIP mutation or (b) an artifact in accordance with a second model, optionally, wherein the reference base fraction for the candidate mutation satisfies the first threshold when the reference base fraction exceeds twenty percent; and
inputting second information into the second model, wherein the second model comprises a second plurality of parameters, thereby obtaining as output from the second model, through application of the second plurality of parameters to the second information, an indication of whether the candidate mutation is (a) the CHIP mutation or (b) the artifact,
wherein the second information comprises at least two of (i) a total number of sequence reads in the plurality of reference sequence reads mapping to the locus of the candidate mutation, (ii) a sequencing depth of the first sequencing reaction at the locus of the candidate mutation, and (iii) an indication of whether the candidate mutation satisfied a minimum variant fraction or a minimum insertion or deletion test,
optionally wherein the second information further comprises a length of the candidate mutation,
optionally wherein the second information further comprises a length of a wildtype allele corresponding to the candidate mutation,
optionally wherein the second information further comprises a binary indication of
whether or not the candidate mutation is in a curated list of known CHIP mutations.

3. The method of claims 1 or 2, wherein the first or second information further comprises:
the reference base fraction of the candidate mutation,
an indication as to whether or not the candidate mutation is in DNMT3A,
an indication as to whether or not the candidate mutation is in STAG2,
an indication as to whether or not the candidate mutation is in TET2,
a binary indication as to whether or not the candidate mutation is in the ASXL1 gene,
a binary indication as to whether or not the candidate mutation is in the JAK2 gene,
a binary indication as to whether or not the candidate mutation is in the SF3B1 gene,
a binary indication as to whether or not the candidate mutation is in the PPM1d gene,
a binary indication as to whether or not the candidate mutation is in the EGFR gene,
a binary indication as to whether or not the candidate mutation is in the KMT2C gene,
a binary indication as to whether or not the candidate mutation is in a CHIP driver gene,
a COSMIC solid tumor frequency of the candidate mutation,
a GnomAD frequency of the candidate mutation,
a score for the candidate mutation using any one of single-base substitution signatures SBS1-SBS60,
a score for the candidate mutation using any one of single-base substitution signatures in the group consisting of SBS55, SBS2, SBS35, SBS7a, SBS54, SBS8, SBS6, SBS44, SBS10a, SBS59, SBS25, SBS21, SBS19, SBS30, SBS10b, SBS7b, SBS11, SBS32, SBS17b, SBS26, SBS4, and SBS33,
an ExAC frequency of the candidate mutation, or
any combination thereof.

4. The method of any one of claims 1-3, wherein the first model or the second model is selected from the group consisting of a regression model, a neural network, a support vector machine, a Naive Bayes model, a nearest neighbor model, a boosted trees model, a random forest model, a decision tree, a gradient boosted tree, an elastic net, a logistic regression model, and a clustering model, or is an XGboost model.

5. The method of any one of claims 1-4, wherein the reference sample:
is blood or saliva;
comprises blood, whole blood, peripheral blood, plasma, serum or lymph;
consists of blood, whole blood, peripheral blood, plasma, serum, or lymph;
consists of buffy coat; or
comprises buffy coat.

6. The method of any one of claims 1-5, wherein the candidate mutation is a single nucleotide variant (SNV), an indel, a copy number variant, or a translocation.

7. The method of any one of claims 1-6, wherein the first sequencing reaction is a panel-based sequencing reaction of a plurality of loci, optionally, wherein the plurality of loci is sequenced at an average sequence depth of less than 250x by the first sequencing reaction or less than 200x by the first sequencing reaction.

8. The method of claim 7, wherein the second sequencing reaction is a panel-based sequencing reaction of the plurality of loci, optionally wherein the plurality of loci is sequenced at an average sequence depth of less than 1000x by the second sequencing reaction or less than 600x by the second sequencing reaction.

9. The method of any one of claims 7 or 8, wherein the plurality of loci comprises at least 5 genes, 25 genes, or 50 genes in Table 1 or at 5 genes, 25 genes, or 50 genes in Table 2.

10. The method of any one of claims 1-9, wherein the first sequencing reaction or the second sequencing reaction is a whole genome sequencing.

11. The method of any one of claims 1-10, wherein the test subject is afflicted with a cancer condition; optionally, wherein the cancer condition is lung cancer, breast cancer, ovarian cancer, cervical cancer, a uveal melanoma, colorectal cancer, chromophobe renal cell carcinoma, liver cancer, an endocrine tumor, oropharyngeal cancer, retinoblastoma, biliary cancer, adrenal cancer, neural cancer, neuroblastoma, basal cell carcinoma, brain cancer, a non-clear cell renal cell carcinoma, a glioblastoma, a glioma, kidney cancer, gastrointestinal stromal tumor, a medulloblastoma, bladder cancer, gastric cancer, bone cancer, thymoma, prostate cancer, a clear cell renal cell carcinoma, skin cancer, thyroid cancer, a sarcoma, testicular cancer, head and neck cancer, a meningioma, peritoneal cancer, endometrial cancer, pancreatic cancer, mesothelioma, esophageal cancer, small cell lung cancer, HER2 negative breast cancer, ovarian serous carcinoma, HR+ breast cancer, uterine serous carcinoma, a uterine corpus endometrial carcinoma, a gastroesophageal junction adenocarcinoma, gallbladder cancer, chordoma, or a papillary renal cell carcinoma, or is a particular stage of a particular type of cancer.

12. The method of any one of claims 1-11, wherein the first model indicates the candidate variant is a CHIP variant and a report is generated for the test subject comprising the identity of the candidate variant; optionally wherein the report further comprises a therapeutic recommendation for the test subject based on the identity of the candidate variant and/or the report further comprises an identification of the candidate variant as being associated with a secondary hematologic malignancy, wherein optionally, the secondary hematologic malignancy is a therapy-related neoplasm, a secondary acute lymphoblastic leukemia, a secondary myelodysplastic syndrome, a secondary myeloproliferative neoplasm, a secondary non-Hodgkin lymphoma, a secondary plasma cell dyscrasia, or Richter's Transformation.

13. The method of any one of claims 1-12, wherein
the first sequencing reaction is at a depth of less than 200x, and
the second sequencing reaction is at a depth of less than 600x; or
the first sequencing reaction is at a depth of at least 50x, and
the second sequencing reaction is at a depth of at least 100x.

14. A computer system comprising:
one or more processors; and
a non-transitory computer-readable medium including computer-executable instructions that, when executed by the one or more processors, cause the processors to perform a method according to any one of claims 1-13.

15. A non-transitory computer-readable storage medium having stored thereon program code instructions that, when executed by a processor, cause the processor to perform the method according to any one of claims 1-13.
